# EUROPEAN PATENT APPLICATION

(11) **EP 4 647 430 A1**
(43) Date of publication of application: **12.11.2025**
(21) Application number: 24738563.6
(22) Date of filing: 05.01.2024
(51) Int. Cl.: C07D 409/12, A61K 31/4436, A61P 29/00

(54) **TETRAHYDROTHIOPHENE DERIVATIVE AND USE THEREOF IN MEDICINE**

(30) Priority: 06.01.2023 CN 202310014893; 28.03.2023 CN 202310310653; 03.04.2023 CN 202310346142; 09.05.2023 CN 202310516277; 25.05.2023 CN 202310599616; 16.06.2023 CN 202310717133; 14.08.2023 CN 202311019252
(71) Applicant: Haisco Pharmaceutical Group Co., Ltd., Shannan, Tibet 856000 (CN)
(72) Inventor: ZHANG, Chen, Lhoka, Tibet 856099 (CN); LEI, Ming, Lhoka, Tibet 856099 (CN); WENG, Guanglin, Lhoka, Tibet 856099 (CN); MOU, Tao, Lhoka, Tibet 856099 (CN); JIAO, Ziyu, Lhoka, Tibet 856099 (CN); GOU, Xiaoli, Lhoka, Tibet 856099 (CN); YU, Yan, Lhoka, Tibet 856099 (CN); LI, Yao, Lhoka, Tibet 856099 (CN); YAN, Pangke, Lhoka, Tibet 856099 (CN)
(74) Representative: AWA Denmark A/S
(86) International application number: PCT/CN2024/070802
(87) International publication number: WO 2024/146632

(57) **Abstract**

A compound as represented by general formula (I) or a stereoisomer, deuterated compound, solvate, prodrug, metabolite, pharmaceutically acceptable salt or a cocrystal thereof, and an intermediate thereof, a preparation method therefor, and the use thereof in the preparation of a drug for treating pain.

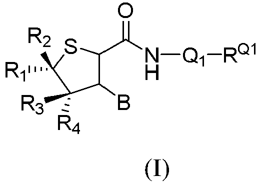

## Description

### Technical Field

The present invention relates to a compound as represented by general formula (I) or a stereoisomer, deuterated compound, solvate, prodrug, metabolite, pharmaceutically acceptable salt or cocrystal thereof, and an intermediate thereof, a preparation method therefor, and the use thereof in the preparation of a drug for treating or relieving pain.

### Background Art

Pain originates from nociceptors in the peripheral nervous system. The nociceptors can convert the thermal, mechanical or chemical stimuli they sense into nerve impulses (action potentials), which are transmitted via afferent nerve fibres to their cell bodies located in the dorsal root ganglia (DRG), and ultimately to higher nerve centres, evoking the perception of pain. The generation and propagation of action potentials in neurons rely on voltage-gated sodium channels (VGSCs) in the cell membrane. When the cell membrane depolarizes, sodium ion channels are activated and open, causing sodium ion influx, which further depolarizes the cell membrane, leading to the generation of action potentials.

VGSCs are composed of a pore-forming α-subunit (approximately 260 kDa) and an associated β-subunit(s) of smaller size (30-40 kDa). The related α-subunit family consists of 10 members, 9 of which (Nav1.1-1.9) are voltage-gated. Nav1.8, encoded by the gene SCN10A and predominantly expressed in peripheral sensory neurons, has been demonstrated to shape action potentials in these neurons. Nav1.8 transcripts and protein have been identified in dorsal root ganglia (DRG) neurons. Nav1.8 is undetectable in non-neuronal tissues (e.g., heart and skeletal muscle) or the central nervous system (including brain and spinal cord).

The critical role of Nav1.8 in pain signal transduction is supported by multiple lines of evidence. Based on a series of animal experiments and human genetic evidence, selective inhibition of Nav1.8 has the potential to become a new type of analgesic therapy. Currently, there are drugs for this target entering clinical research.

### Summary of the Invention

The objective of the present invention is to provide a class of tetrahydrothiophene derivatives with inhibitory activity on Nav1.8. Such compounds selectively inhibit Nav1.8 and can effectively reduce side effects, while exhibiting good analgesic activity and oral bioavailability, and excellent inhibitory activity and selectivity toward hNav1.8. The compounds demonstrate superior oral absorption performance in animals (such as mice, rats, dogs, and monkeys), weak inhibition of CYP enzymes, and good permeability.

The present invention provides a compound as represented by general formula (I) or a stereoisomer, deuterated compound, solvate, prodrug, metabolite, pharmaceutically acceptable salt or cocrystal thereof:

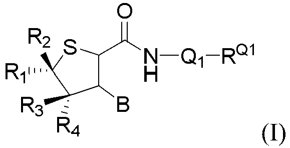

In some embodiments, 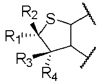 is selected from 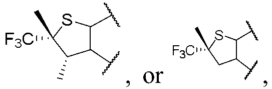 and B is in trans configuration with the adjacent amide group;
in some embodiments, 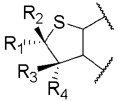 is selected from 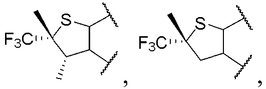 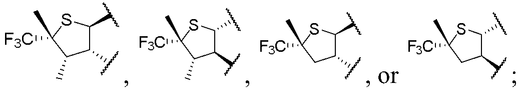
in some embodiments, 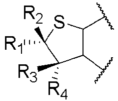 is selected from 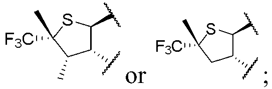
in some embodiments, the compound as represented by general formula (I) is selected from a compound as represented by general formula (Ia), (Ib), (Ic), (Id), (Ie), or (If): 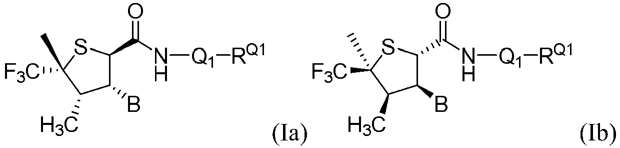 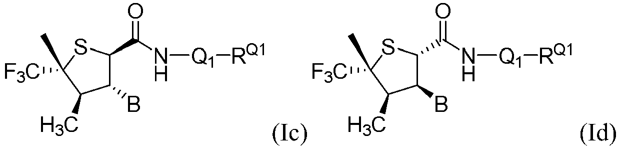 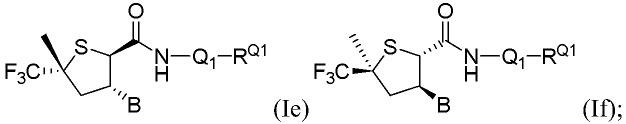
in some embodiments, Q₁ is selected from C₆₋₁₀ aryl, 5- to 10-membered heteroaryl, C₅₋₁₀ carbocyclyl, 5- to 10-membered heterocyclyl or 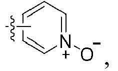 and the aryl, heteroaryl, carbocyclyl or heterocyclyl is optionally substituted with 1 to 5 R^{q};
in some embodiments, Q₁ is selected from phenyl, benzo C₄₋₆ carbocyclyl, benzo 4- to 6-membered heterocyclyl, 5- to 6-membered heteroaryl, 8- to 10-membered fused heteroaryl or 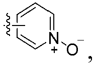 preferably, Q₁ is selected from phenyl, pyridyl or 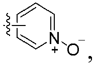 and the Q₁ is optionally substituted with 1 to 4 R^{q};
in some embodiments, Q₁ is selected from 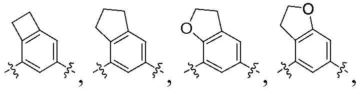 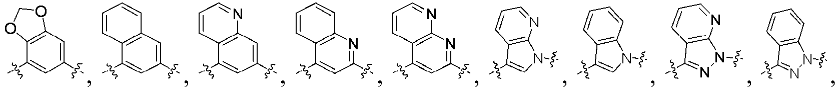 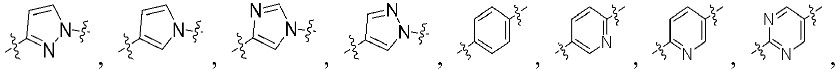 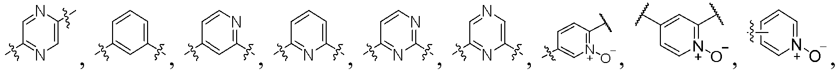 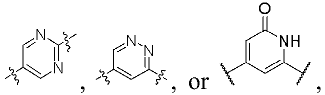 preferably, Q₁ is selected from 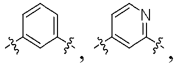 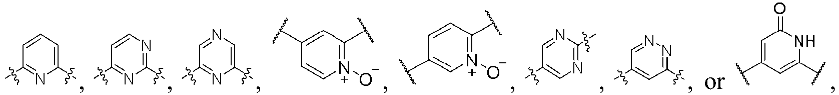 and the Q₁ is optionally substituted with 1 to 5 R^{q};
in some embodiments, R^{Q1} is selected from H, COOH, NR^{q1}R^{q2}, - C(=O)NR^{q1}R^{q2}, -S(=O)₂NR^{q1}R^{q2}, OH, =O, -OR^{q1}, -C(=O)R^{q1}, -S(=O)₂R^{q1}, - S(=O)(=NR^{q1})R^{q2} or -P(=O)R^{q1}R^{q2};
in some embodiments, R^{Q1} is selected from H, COOH, NH₂, -C(=O)NH₂, - S(=O)₂NH₂, OH, =O, -S(=O)₂CH₃, -S(=O)₂-cyclopropyl, -S(=O)(=NH)CH₃, - P(=O)(CH₃)₂, -P(=O)(CH₂CH₃)₂, or -P(=O)(CH₃)(cyclopropyl);
in some embodiments, 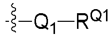 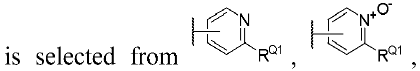 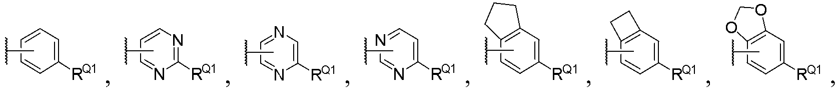 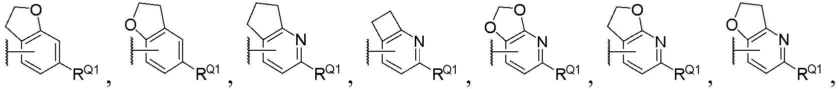 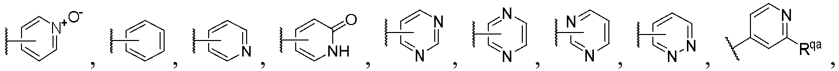 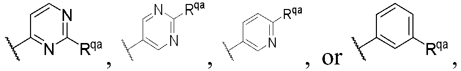 and the Q₁ is optionally substituted with 1 to 3 R^{q};
in some embodiments, 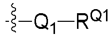 is selected from 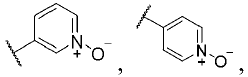 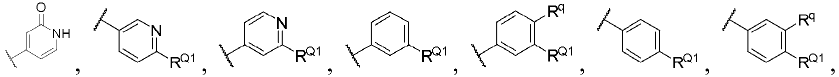 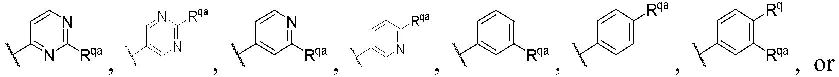 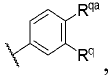 R^{Q1} is selected from COOH, -C(=O)NH₂, -S(=O)₂NH₂, -S(=O)₂CH₃, - S(=O)(=NH)CH₃, or -P(=O)(CH₃)₂, R^{qa} is selected from 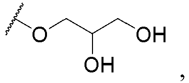 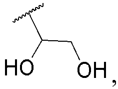 or -CH₂OH, and R^{q} is selected from F, Cl or methyl;
in some embodiments, when R^{Q1} is selected from H, the Q₁ is substituted with 1 to 5 R^{q}, and at least one R^{q} is R^{qa}, wherein R^{qa} is selected from 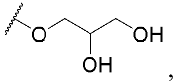 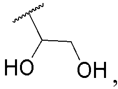 -CH₂OH, -CF₂CH₂OH, NH₂, -P(=O)(CH₃)₂, -P(=O)(CH₂CH₃)₂, or - P(=O)(CH₃)(cyclopropyl);
in some embodiments, R^{qa} is selected from 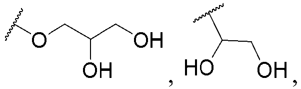 or - CH₂OH;
in some embodiments, 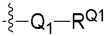 is selected from 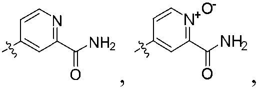 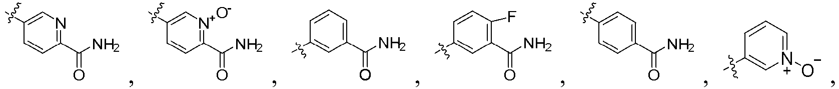 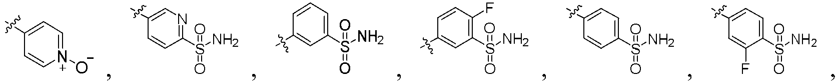 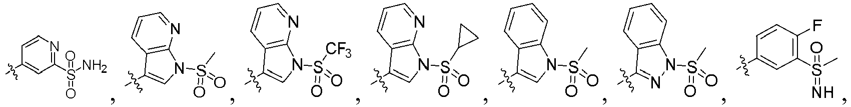 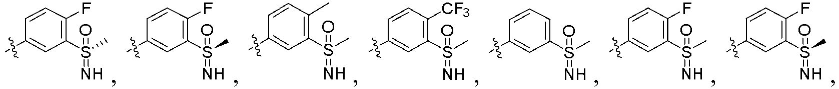 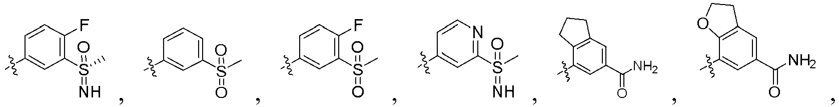 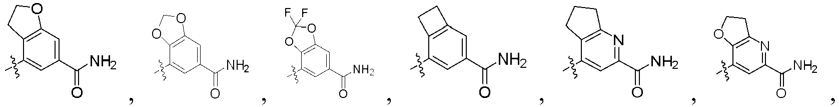 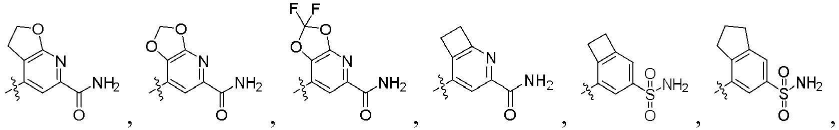 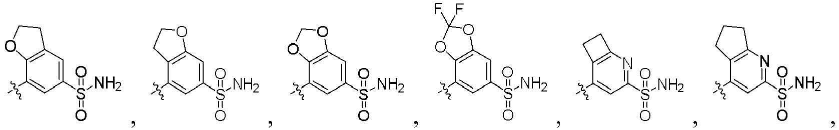 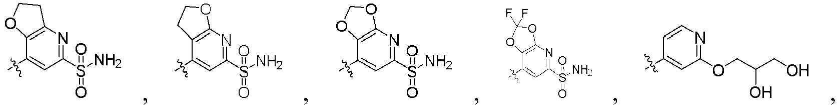 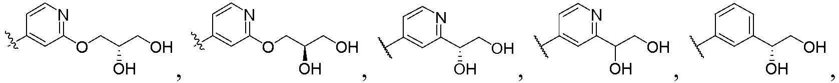 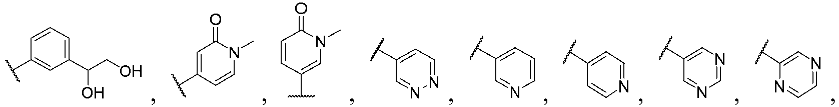 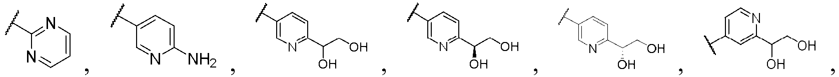 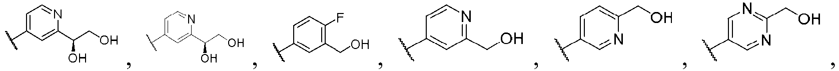 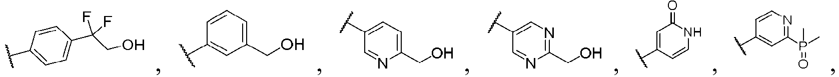 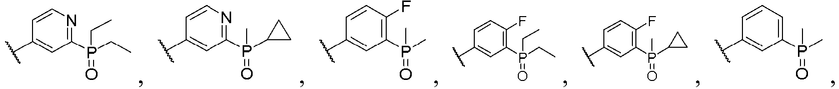 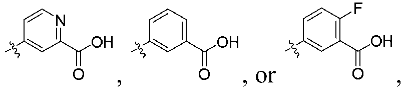 and the Q₁ is optionally substituted with 1 to 3 R^{q};
in some embodiments, R^{q1} and R^{q2} are each independently selected from H, C₁₋₆ alkyl, C₃₋₆ carbocyclyl, or 4- to 7-membered heterocyclyl, and the alkyl, carbocyclyl or heterocyclyl is optionally substituted with 1 to 4 R^{k};
alternatively, R^{q1} and R^{q2} are directly linked to form 4- to 7-membered heterocyclyl, and the heterocyclyl is optionally substituted with 1 to 4 R^{k};
in some embodiments, R^{q1} and R^{q2} are each independently selected from H, C₁₋₄ alkyl, C₃₋₆ carbocyclyl, or 4- to 7-membered heterocyclyl, and the alkyl, carbocyclyl or heterocyclyl is optionally substituted with 1 to 4 R^{k};
alternatively, R^{q1} and R^{q2} are directly linked to form 4- to 7-membered heterocyclyl, and the heterocyclyl is optionally substituted with 1 to 4 R^{k};
in some embodiments, R^{q1} and R^{q2} are each independently selected from H, methyl, ethyl, propyl, isopropyl, cyclopropyl, cyclobutyl or cyclopentyl, and the methyl, ethyl, propyl, isopropyl, cyclopropyl, cyclobutyl or cyclopentyl is optionally substituted with 1 to 4 R^{k};
in some embodiments, B is selected from C₆₋₁₀ aryl, 5- to 10-membered heteroaryl, C₅₋₁₀ carbocyclyl, or 5- to 10-membered heterocyclyl, and the aryl, heteroaryl, carbocyclyl or heterocyclyl is optionally substituted with 1 to 4 R^{B};
in some embodiments, B is selected from phenyl, benzo C₄₋₆ carbocyclyl, benzo 4- to 6-membered heterocyclyl, 5- to 6-membered heteroaryl, or 8- to 10-membered fused heteroaryl, and the B is optionally substituted with 1 to 4 R^{B};
in some embodiments, B is selected from phenyl, 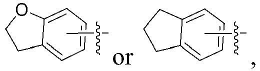 and the B is optionally substituted with 1 to 4 R^{B};
in some embodiments, B is selected from 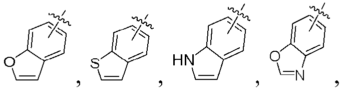 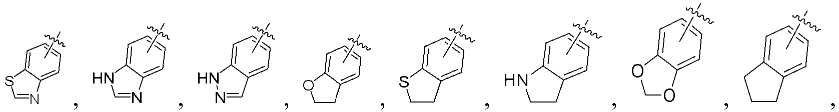 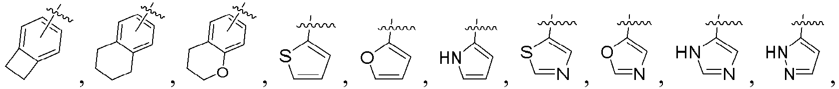 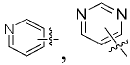 or phenyl, and the B is optionally substituted with 1 to 4 R^{B};
in some embodiments, B is selected from 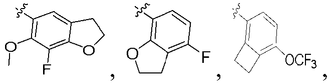 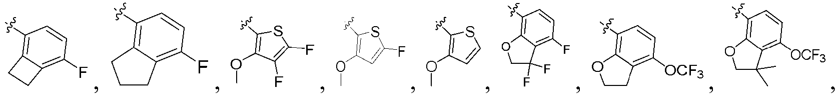 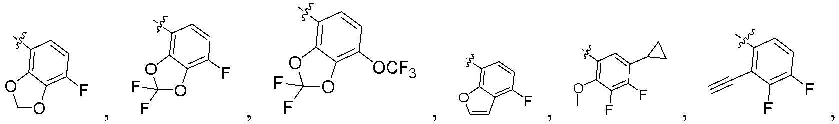 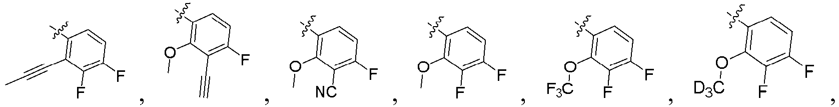 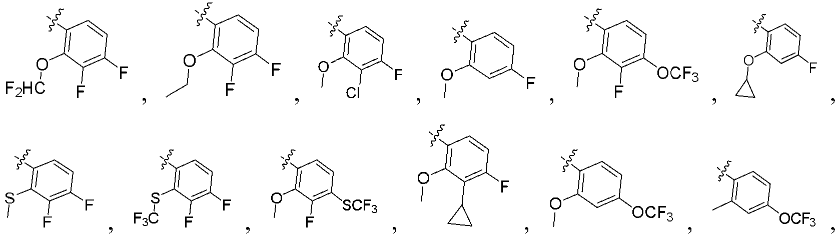 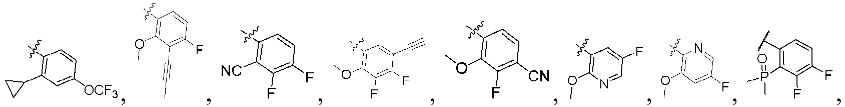 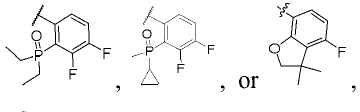 preferably 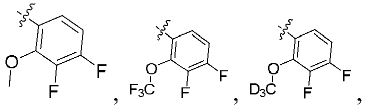 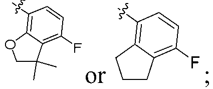
in some embodiments, R¹ and R² are each independently selected from H, halogen, CN, OH, C₁₋₆ alkyl, C₂₋₆ alkenyl, C₂₋₆ alkynyl, -OC₁₋₆ alkyl, -SC₁₋₆ alkyl, C₃₋₆ carbocyclyl, or 3- to 7-membered heterocyclyl, and the alkyl, alkenyl, alkynyl, carbocyclyl or heterocyclyl is optionally substituted with 1 to 4 R^{k}, provided that R¹ and R² are not both H;
in some embodiments, R¹ and R² are each independently selected from H, halogen, CN, OH, C₁₋₄ alkyl, C₂₋₄ alkenyl, C₂₋₄ alkynyl, -OC₁₋₄ alkyl, -SC₁₋₄ alkyl, C₃₋₆ carbocyclyl, or 3- to 7-membered heterocyclyl, and the alkyl, alkenyl, alkynyl, carbocyclyl or heterocyclyl is optionally substituted with 1 to 4 R^{k}, provided that R¹ and R² are not both H;
in some embodiments, R¹ and R² are each independently selected from H, F, Cl, Br, cyano, methyl, ethyl, methoxy, ethoxy, methylthio, cyclopropyl, cyclobutyl, ethenyl, or ethynyl, and the methyl, ethyl, methoxy, ethoxy, methylthio, cyclopropyl, cyclobutyl, ethenyl, or ethynyl is optionally substituted with 1 to 4 R^{k}, provided that R¹ and R² are not both H;
in some embodiments, R¹ and R² are each independently selected from H, methyl, ethyl, CH₂F, CHF₂, or CF₃, provided that R¹ and R² are not both H;
in some embodiments, R³, R⁴, R^{q}, and R^{B} are each independently selected from H, deuterium, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, C₃₋₇ carbocyclyl, -OC₃₋₇ carbocyclyl, 3- to 7-membered heterocyclyl or -P(=O)R^{q1}R^{q2}, and the alkyl, alkenyl, alkynyl, carbocyclyl or heterocyclyl is optionally substituted with 1 to 4 R^{k};
in some embodiments, R³, R⁴, R^{q}, and R^{B} are each independently selected from H, deuterium, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, C₃₋₇ carbocyclyl, -OC₃₋₇ carbocyclyl, 3- to 7-membered heterocyclyl or -P(=O)R^{q1}R^{q2}, and the alkyl, alkenyl, alkynyl, carbocyclyl or heterocyclyl is optionally substituted with 1 to 4 R^{k};
in some embodiments, R³, R⁴, R^{q}, and R^{B} are each independently selected from H, deuterium, F, Cl, Br, cyano, methyl, ethyl, isopropyl, methoxy, ethoxy, isopropyloxy, methylthio, cyclopropyl, cyclopropyloxy, cyclobutyl, ethenyl, ethynyl, -P(=O)(CH₃)₂, -P(=O)(CH₂CH₃)₂, or -P(=O)(CH₃)(cyclopropyl), and the methyl, ethyl, isopropyl, methoxy, ethoxy, isopropyloxy, methylthio, cyclopropyl, cyclobutyl, ethenyl, or ethynyl is optionally substituted with 1 to 4 R^{k};
in some embodiments, R³ and R⁴ are each independently selected from H, methyl, ethyl, CH₂F, CHF₂, or CF₃;
in some embodiments, R^{q} and R^{B} are each independently selected from H, F, Cl, Br, cyano, CH₂F, CHF₂, CF₃, -OCH₂F, -OCHF₂, -OCF₃, -OCD₃, methyl, -S-methyl, -S-CF₃, ethyl, isopropyl, ethynyl, methoxy, ethoxy, isopropyloxy, propyloxy, cyclopropyl, -O-cyclopropyl, -P(=O)(CH₃)₂, -P(=O)(CH₂CH₃)₂, or - P(=O)(CH₃)(cyclopropyl), and the methyl, ethyl, isopropyl, ethynyl, methoxy, ethoxy, isopropyloxy, propyloxy, or cyclopropyl is optionally substituted with 1 to 4 R^{k};
in some embodiments, each R^{B} is independently selected from F, Cl, Br, cyano, CH₂F, CHF₂, CF₃, -OCH₂F, -OCHF₂, -OCF₃, -OCD₃, methyl, ethyl, isopropyl, methoxy, ethoxy, isopropyloxy, propyloxy, cyclopropyl, or -O-cyclopropyl;
in some embodiments, each R^{q} is independently selected from H, F, Cl, Br, cyano, CH₂F, CHF₂, CF₃, -OCH₂F, -OCHF₂, -OCF₃, -OCD₃, methyl, -S-methyl, - S-CF₃, ethyl, isopropyl, ethynyl, methoxy, ethoxy, isopropyloxy, propyloxy, cyclopropyl, -O-cyclopropyl, 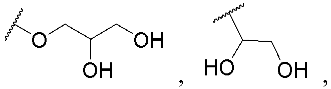 -CH₂OH, or - CF₂CH₂OH;
in some embodiments, each R^{q} is independently selected from F, Cl, Br, cyano, CH₂F, CHF₂, or CF₃;
in some embodiments, any one of R^{q} is R^{qa};
in some embodiments, R^{k} is selected from deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3- to 7-membered heterocyclyl, -C₁₋₄ alkylene-C₃₋₆ carbocyclyl, -C₁₋₄ alkylene-3- to 7-membered heterocyclyl, C₃₋₆ carbocyclyl, or 3-to 7-membered heterocyclyl, and the alkyl, alkylene, alkenyl, alkynyl, carbocyclyl or heterocyclyl is optionally substituted with 1 to 4 substituents selected from deuterium, halogen, =O, CN, OH, NH₂, C₁₋₆ alkyl, or C₁₋₆ alkoxy;
in some embodiments, R^{k} is selected from deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocyclyl, or -O-3- to 7-membered heterocyclyl, and the alkyl, alkylene, alkenyl, alkynyl, carbocyclyl or heterocyclyl is optionally substituted with 1 to 4 substituents selected from deuterium, halogen, CN, OH, NH₂, C₁₋₄ alkyl, or C₁₋₄ alkoxy;
in some embodiments, R^{k} is selected from deuterium, =O, F, Cl, Br, I, CN, OH, NH₂, NH(CH₃), NH(CH₂CH₃), N(CH₃)₂, N(CH₂CH₃)₂, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl, and the methyl, ethyl, ethenyl, ethynyl, methoxy, ethoxy, methylthio, cyclopropyl, cyclobutyl, cyclopentyl, or cyclohexyl is optionally substituted with 1 to 4 substituents selected from deuterium, halogen, CN, OH, NH₂, C₁₋₄ alkyl, or C₁₋₄ alkoxy;
in some embodiments, R^{k} is selected from deuterium, F, Cl, Br, I, CN, OH, - CH₂OH, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl.

A first embodiment of the present invention provides the above-described compound as represented by general formula (I) or the stereoisomer, deuterated compound, solvate, prodrug, metabolite, pharmaceutically acceptable salt or cocrystal thereof, wherein
Q₁ is selected from C₆₋₁₀ aryl, 5- to 10-membered heteroaryl, C₅₋₁₀ carbocyclyl, 5- to 10-membered heterocyclyl or 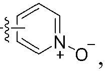 and the aryl, heteroaryl, carbocyclyl or heterocyclyl is optionally substituted with 1 to 5 R^{q};
R^{Q1} is selected from H, COOH, NR^{q1}R^{q2}, -C(=O)NR^{q1}R^{q2}, -S(=O)₂NR^{q1}R^{q2}, OH, =O, -OR^{q1}, -C(=O)R^{q1}, -S(=O)₂R^{q1}, -S(=O)(=NR^{q1})R^{q2} or -P(=O)R^{q1}R^{q2};
R^{q1} and R^{q2} are each independently selected from H, C₁₋₆ alkyl, C₃₋₆ carbocyclyl, or 4- to 7-membered heterocyclyl, and the alkyl, carbocyclyl or heterocyclyl is optionally substituted with 1 to 4 R^{k};
alternatively, R^{q1} and R^{q2} are directly linked to form 4- to 7-membered heterocyclyl, and the heterocyclyl is optionally substituted with 1 to 4 R^{k};
B is selected from C₆₋₁₀ aryl, 5- to 10-membered heteroaryl, C₅₋₁₀ carbocyclyl, or 5- to 10-membered heterocyclyl, and the aryl, heteroaryl, carbocyclyl or heterocyclyl is optionally substituted with 1 to 4 R^{B};
R¹ and R² are each independently selected from H, halogen, CN, OH, C₁₋₆ alkyl, C₂₋₆ alkenyl, C₂₋₆ alkynyl, -OC₁₋₆ alkyl, -SC₁₋₆ alkyl, C₃₋₆ carbocyclyl, or 3-to 7-membered heterocyclyl, and the alkyl, alkenyl, alkynyl, carbocyclyl or heterocyclyl is optionally substituted with 1 to 4 R^{k}, provided that R¹ and R² are not both H;
R³, R⁴, R^{q}, and R^{B} are each independently selected from H, deuterium, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, C₃₋₇ carbocyclyl, -OC₃₋₇ carbocyclyl, 3- to 7-membered heterocyclyl or -P(=O)R^{q1}R^{q2}, and the alkyl, alkenyl, alkynyl, carbocyclyl or heterocyclyl is optionally substituted with 1 to 4 R^{k};
R^{k} is selected from deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3-to 7-membered heterocyclyl, -C₁₋₄ alkylene -C₃₋₆ carbocyclyl, -C₁₋₄ alkylene -3- to 7-membered heterocyclyl, C₃₋₆ carbocyclyl, or 3- to 7-membered heterocyclyl, and the alkyl, alkylene, alkenyl, alkynyl, carbocyclyl or heterocyclyl is optionally substituted with 1 to 4 substituents selected from deuterium, halogen, =O, CN, OH, NH₂, C₁₋₆ alkyl, or C₁₋₆ alkoxy.

A second embodiment of the present invention provides the above-described compound as represented by general formula (I) or the stereoisomer, deuterated compound, solvate, prodrug, metabolite, pharmaceutically acceptable salt or cocrystal thereof, wherein
R^{q1} and R^{q2} are each independently selected from H, C₁₋₄ alkyl, C₃₋₆ carbocyclyl, or 4- to 7-membered heterocyclyl, and the alkyl, carbocyclyl or heterocyclyl is optionally substituted with 1 to 4 R^{k};
alternatively, R^{q1} and R^{q2} are directly linked to form 4- to 7-membered heterocyclyl, and the heterocyclyl is optionally substituted with 1 to 4 R^{k};
Q₁ is selected from phenyl, benzo C₄₋₆ carbocyclyl, benzo 4- to 6-membered heterocyclyl, 5- to 6-membered heteroaryl, 8- to 10-membered fused heteroaryl or 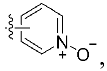 preferably, Q₁ is selected from phenyl, pyridyl or 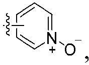 and the Q₁ is optionally substituted with 1 to 4 R^{q};
B is selected from phenyl, benzo C₄₋₆ carbocyclyl, benzo 4- to 6-membered heterocyclyl, 5- to 6-membered heteroaryl, or 8- to 10-membered fused heteroaryl, and the B is optionally substituted with 1 to 4 R^{B};
R¹ and R² are each independently selected from H, halogen, CN, OH, C₁₋₄ alkyl, C₂₋₄ alkenyl, C₂₋₄ alkynyl, -OC₁₋₄ alkyl, -SC₁₋₄ alkyl, C₃₋₆ carbocyclyl, or 3-to 7-membered heterocyclyl, and the alkyl, alkenyl, alkynyl, carbocyclyl or heterocyclyl is optionally substituted with 1 to 4 R^{k}, provided that R¹ and R² are not both H;
R³, R⁴, R^{q}, and R^{B} are each independently selected from H, deuterium, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, C₃₋₇ carbocyclyl, -OC₃₋₇ carbocyclyl, 3- to 7-membered heterocyclyl or -P(=O)R^{q1}R^{q2}, and the alkyl, alkenyl, alkynyl, carbocyclyl or heterocyclyl is optionally substituted with 1 to 4 R^{k};
R^{k} is selected from deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocyclyl, or -O-3- to 7-membered heterocyclyl, and the alkyl, alkylene, alkenyl, alkynyl, carbocyclyl or heterocyclyl is optionally substituted with 1 to 4 substituents selected from deuterium, halogen, CN, OH, NH₂, C₁₋₄ alkyl, or C₁₋₄ alkoxy;
the definitions of other groups are the same as those in the first embodiment of the present invention.
A third embodiment of the present invention provides the above-described compound as represented by general formula (I) or the stereoisomer, deuterated compound, solvate, prodrug, metabolite, pharmaceutically acceptable salt or cocrystal thereof, wherein
R^{q1} and R^{q2} are each independently selected from H, methyl, ethyl, propyl, isopropyl, cyclopropyl, cyclobutyl or cyclopentyl, and the methyl, ethyl, propyl, isopropyl, cyclopropyl, cyclobutyl or cyclopentyl is optionally substituted with 1 to 4 R^{k};
R¹ and R² are each independently selected from H, F, Cl, Br, cyano, methyl, ethyl, methoxy, ethoxy, methylthio, cyclopropyl, cyclobutyl, ethenyl, or ethynyl, and the methyl, ethyl, methoxy, ethoxy, methylthio, cyclopropyl, cyclobutyl, ethenyl, or ethynyl is optionally substituted with 1 to 4 R^{k}, provided that R¹ and R² are not both H;
B is selected from 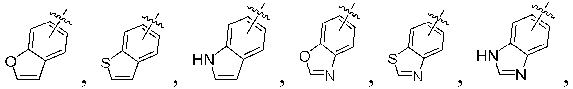 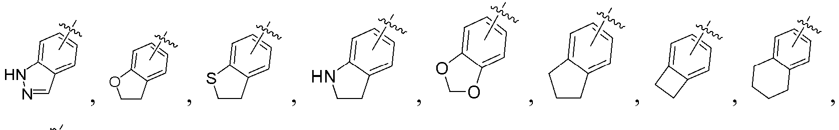 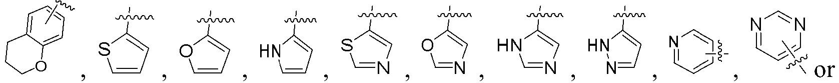 phenyl, preferably phenyl, 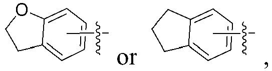 and the B is optionally substituted with 1 to 4 R^{B};
R³, R⁴, R^{q}, and R^{B} are each independently selected from H, deuterium, F, Cl, Br, cyano, methyl, ethyl, isopropyl, methoxy, ethoxy, isopropyloxy, methylthio, cyclopropyl, cyclopropyloxy, cyclobutyl, ethenyl, ethynyl, -P(=O)(CH₃)₂, - P(=O)(CH₂CH₃)₂, or -P(=O)(CH₃)(cyclopropyl), and the methyl, ethyl, isopropyl, methoxy, ethoxy, isopropyloxy, methylthio, cyclopropyl, cyclobutyl, ethenyl, or ethynyl is optionally substituted with 1 to 4 R^{k}; R^{k} is selected from deuterium, =O, F, Cl, Br, I, CN, OH, NH₂, NH(CH₃), NH(CH₂CH₃), N(CH₃)₂, N(CH₂CH₃)₂, methyl, ethyl, ethenyl, ethynyl, methoxy, ethoxy, methylthio, -O-cyclopropyl, - NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl, and the methyl, ethyl, ethenyl, ethynyl, methoxy, ethoxy, methylthio, cyclopropyl, cyclobutyl, cyclopentyl, or cyclohexyl is optionally substituted with 1 to 4 substituents selected from deuterium, halogen, CN, OH, NH₂, C₁₋₄ alkyl, or C₁₋₄ alkoxy;
the definitions of other groups are the same as those in the first or second embodiment of the present invention.

A fourth embodiment of the present invention provides the above-described compound as represented by general formula (I) or the stereoisomer, deuterated compound, solvate, prodrug, metabolite, pharmaceutically acceptable salt or cocrystal thereof, wherein
Q₁ is selected from 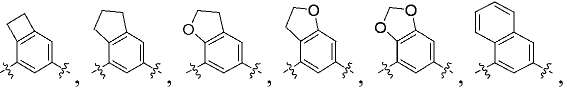 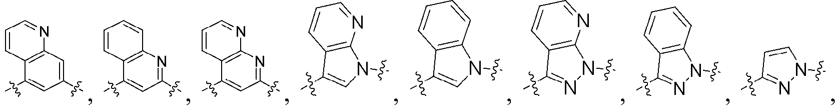 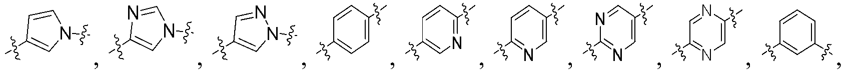 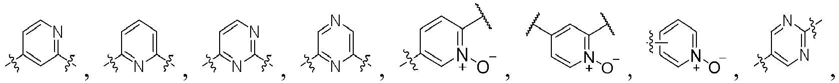 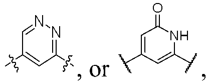 and the Q₁ is optionally substituted with 1 to 5 R^{q};
R³ and R⁴ are each independently selected from H, methyl, ethyl, CH₂F, CHF₂, or CF₃;
R¹ and R² are each independently selected from H, methyl, ethyl, CH₂F, CHF₂, or CF₃, provided that R¹ and R² are not both H;
R^{q} and R^{B} are each independently selected from H, F, Cl, Br, cyano, CH₂F, CHF₂, CF₃, -OCH₂F, -OCHF₂, -OCF₃, -OCD₃, methyl, -S-methyl, -S-CF₃, ethyl, isopropyl, ethynyl, methoxy, ethoxy, isopropyloxy, cyclopropyl, -O-cyclopropyl, - P(=O)(CH₃)₂, -P(=O)(CH₂CH₃)₂, or -P(=O)(CH₃)(cyclopropyl), and the methyl, ethyl, isopropyl, ethynyl, methoxy, ethoxy, isopropyloxy, or cyclopropyl is optionally substituted with 1 to 4 R^{k};
R^{k} is selected from deuterium, F, Cl, Br, I, CN, OH, -CH₂OH, methyl, ethyl, ethenyl, ethynyl, methoxy, ethoxy, methylthio, -O-cyclopropyl, -NH-cyclopropyl, - CH₂-cyclopropyl, -CH₂-cyclobutyl, -CH₂-cyclopentyl, -CH₂-cyclohexyl, cyclopropyl, cyclobutyl, cyclopentyl, or cyclohexyl;
the definitions of other groups are the same as those in the first, second or third embodiment of the present invention.

A fifth embodiment of the present invention provides the above-described compound as represented by general formula (I) or the stereoisomer, deuterated compound, solvate, prodrug, metabolite, pharmaceutically acceptable salt or cocrystal thereof, wherein 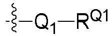
is selected from 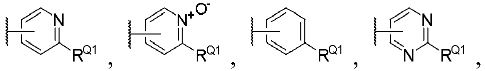 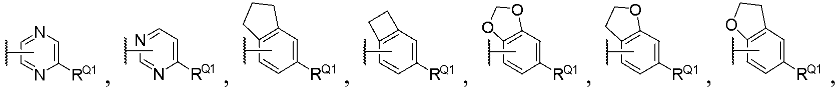 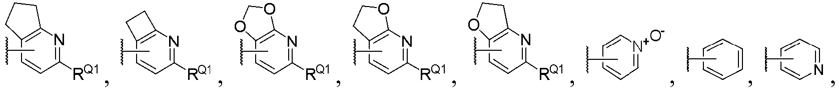 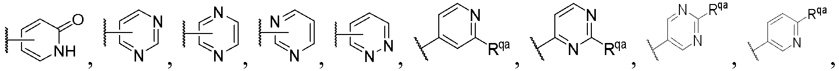 or 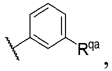 and the Q₁ is optionally substituted with 1 to 3 R^{q};
R^{qa} is selected from 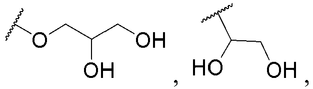 -CH₂OH, -CF₂CH₂OH, NH₂, -P(=O)(CH₃)₂, -P(=O)(CH₂CH₃)₂, or -P(=O)(CH₃)(cyclopropyl);
preferably, 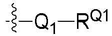 is selected from 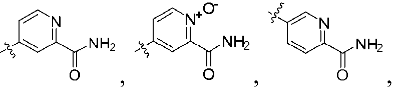 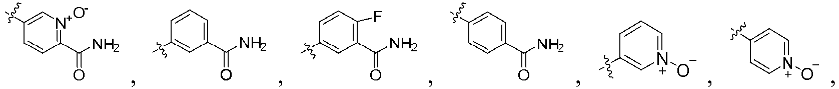 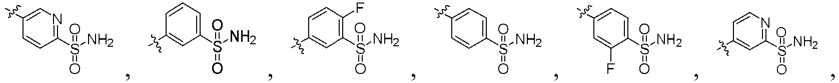 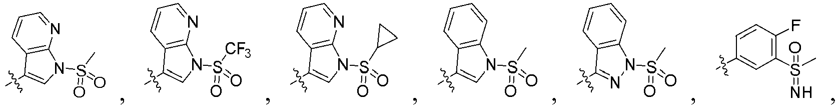 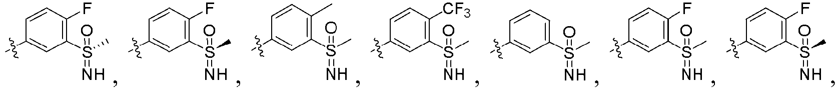 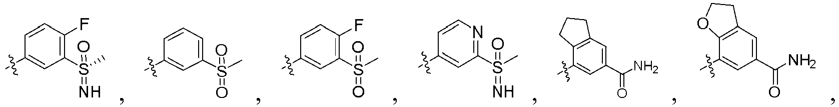 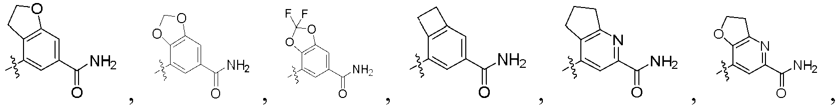 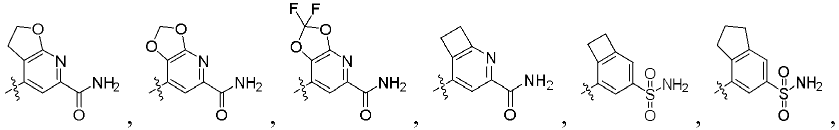 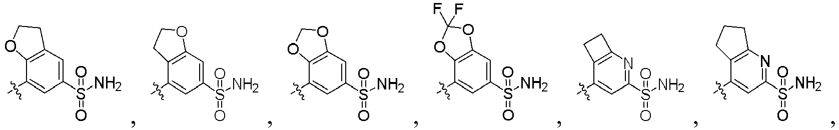 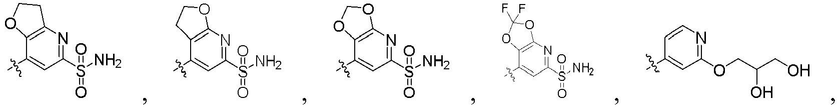 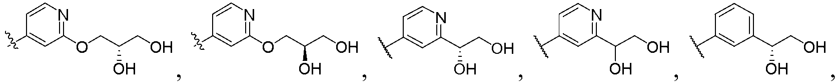 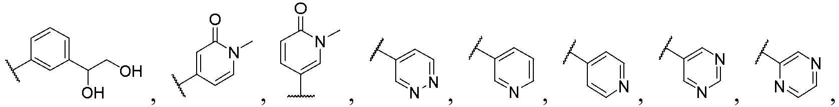 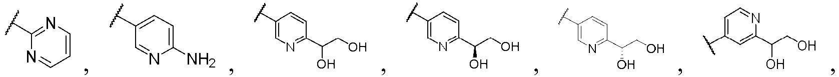 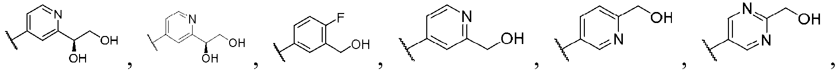 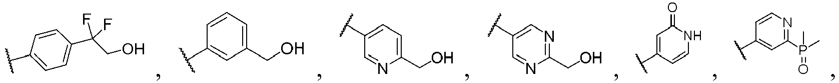 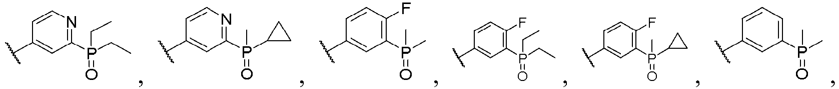 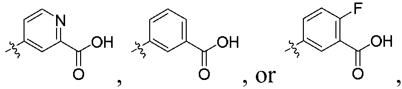 and the Q₁ is optionally substituted with 1 to 3 R^{q};
B is selected from 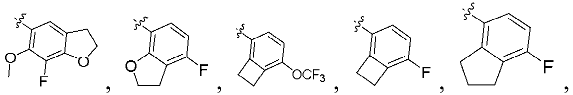 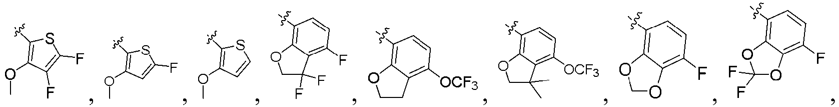 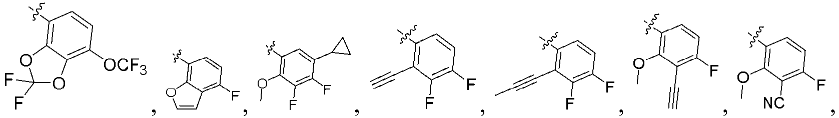 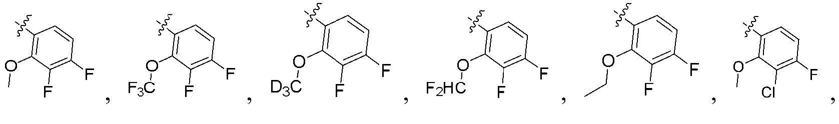 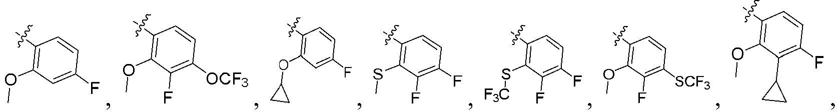 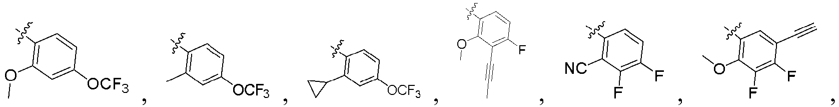 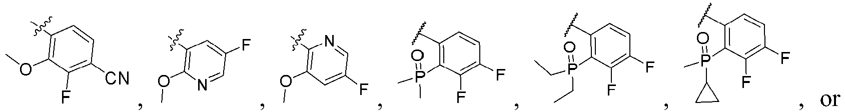 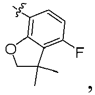 preferably 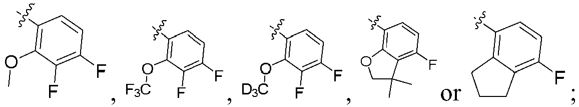
the definitions of other groups are the same as those in the first, second, third or fourth embodiment of the present invention.

A sixth embodiment of the present invention provides the above-described compound as represented by general formula (I) or the stereoisomer, deuterated compound, solvate, prodrug, metabolite, pharmaceutically acceptable salt or cocrystal thereof, wherein
the compound as represented by general formula (I) is selected from a compound as represented by general formula (Ia), (Ib), (Ic), (Id), (Ie), or (If): 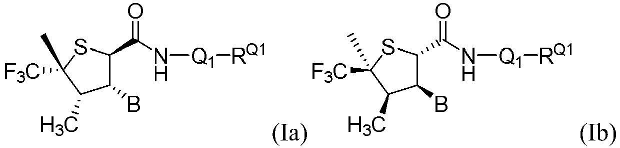 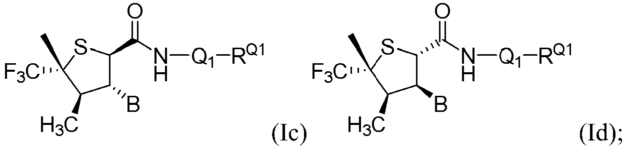 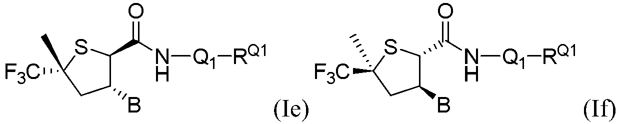
the definitions of other groups are the same as those in the first, second, third, fourth or fifth embodiment of the present invention.

A seventh embodiment of the present invention provides the above-described compound as represented by general formula (I) or the stereoisomer, deuterated compound, solvate, prodrug, metabolite, pharmaceutically acceptable salt or cocrystal thereof, wherein 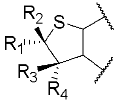
is selected from 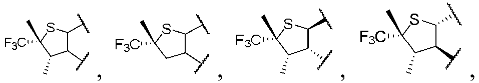 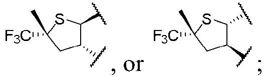
the definitions of other groups are the same as those in the first, second, third, fourth or fifth embodiment of the present invention.

The present invention relates to a compound as described below, or a stereoisomer, deuterated compound, solvate, prodrug, metabolite, pharmaceutically acceptable salt or cocrystal thereof, wherein the compound has a structure selected from one of the structures as shown in Table E.

**Table E**

| | | | | |
|---|---|---|---|---|
| 1 | 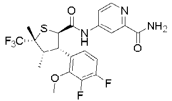 | 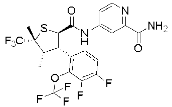 | 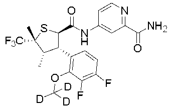 | 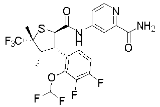 |
| 2 | 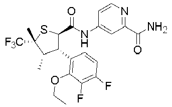 | 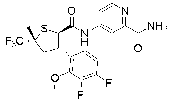 | 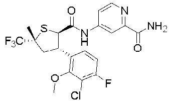 | 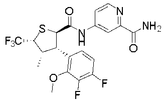 |
| 3 | 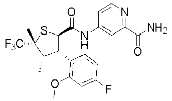 | 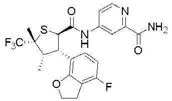 | 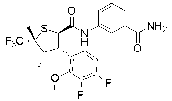 | 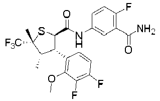 |
| 4 | 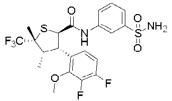 | 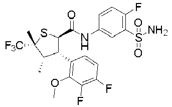 | 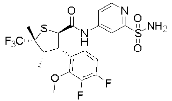 | 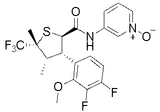 |
| 5 | 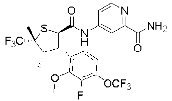 | 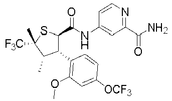 | 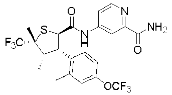 | 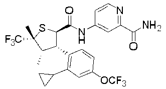 |
| 6 | 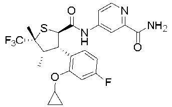 | 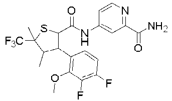 | 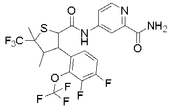 | 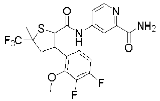 |
| 7 | 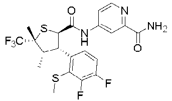 | 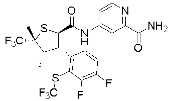 | 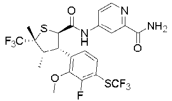 | 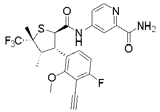 |
| 8 | 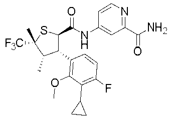 | 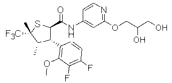 | 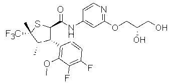 | 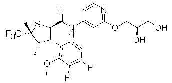 |
| 9 | 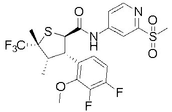 | 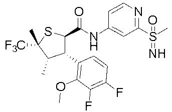 | 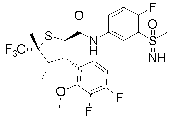 | 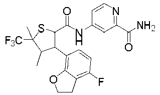 |
| 10 | 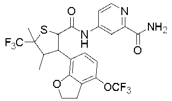 | 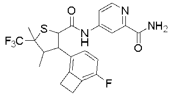 | 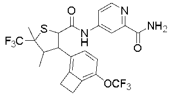 | 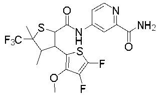 |
| 11 | 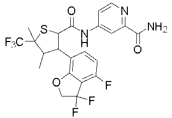 | 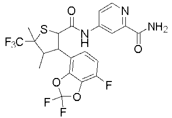 | 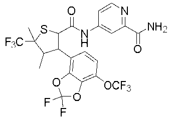 | 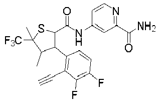 |
| 12 | 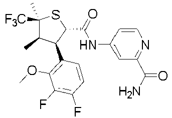 | 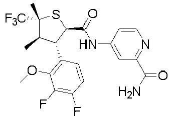 | 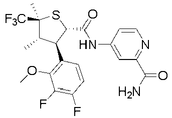 | 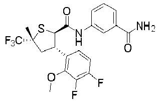 |
| 13 | 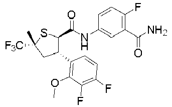 | 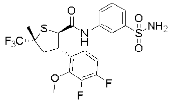 | 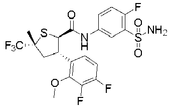 | 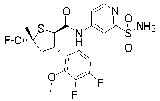 |
| 14 | 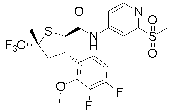 | 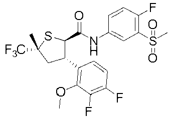 | 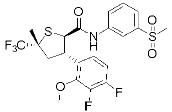 | 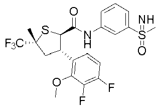 |
| 15 | 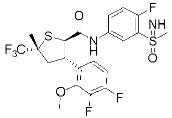 | 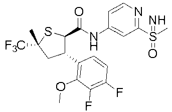 | 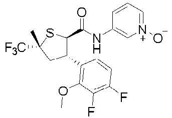 | 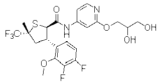 |
| 16 | 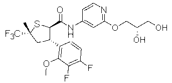 | 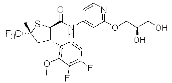 | 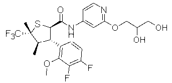 | 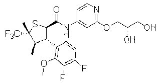 |
| 17 | 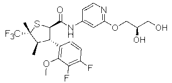 | 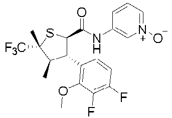 | 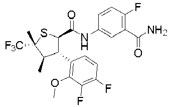 | 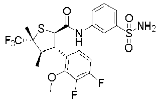 |
| 18 | 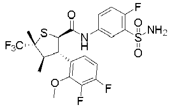 | 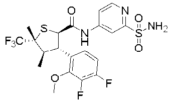 | 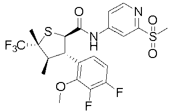 | 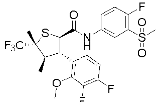 |
| 19 | 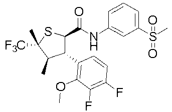 | 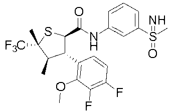 | 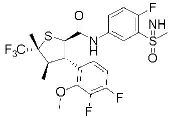 | 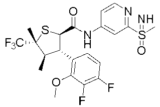 |
| 20 | 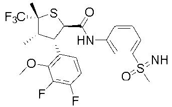 | 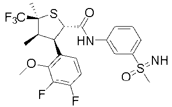 | 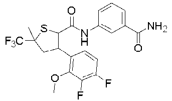 | 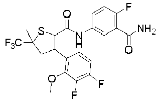 |
| 21 | 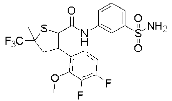 | 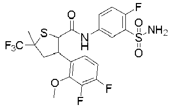 | 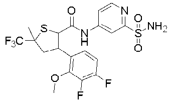 | 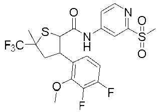 |
| 22 | 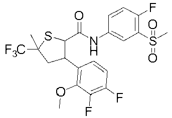 | 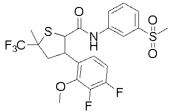 | 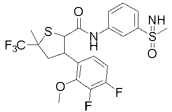 | 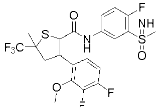 |
| 23 | 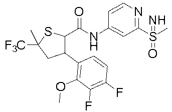 | 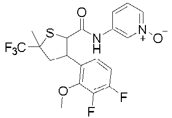 | 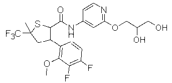 | 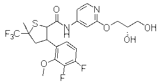 |
| 24 | 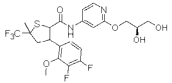 | 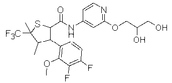 | 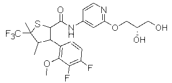 | 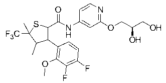 |
| 25 | 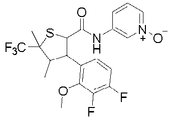 | 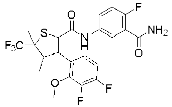 | 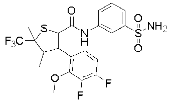 | 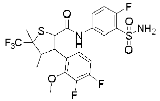 |
| 26 | 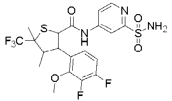 | 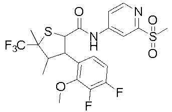 | 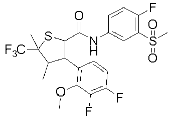 | 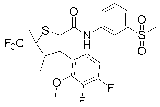 |
| 27 | 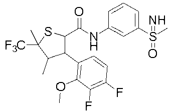 | 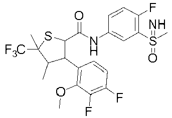 | 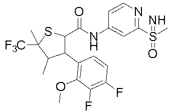 | 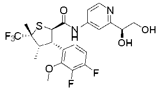 |
| 28 | 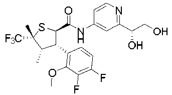 | 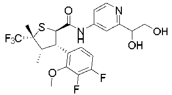 | 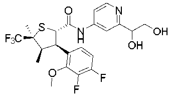 | 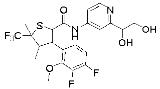 |
| 29 | 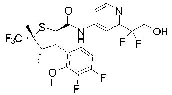 | 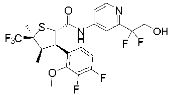 | 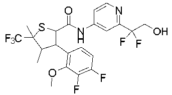 | 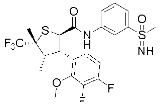 |
| 30 | 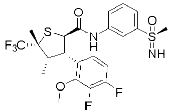 | 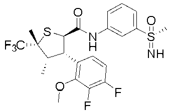 | 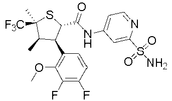 | 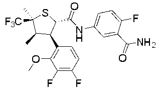 |
| 31 | 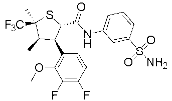 | 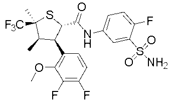 | 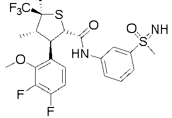 | 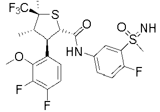 |
| 32 | 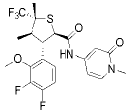 | 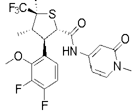 | 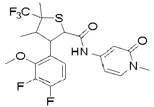 | 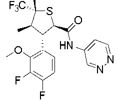 |
| 33 | 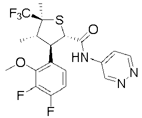 | 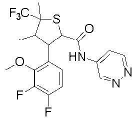 | 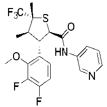 | 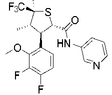 |
| 34 | 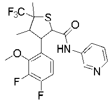 | 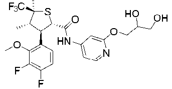 | 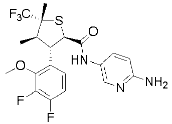 | 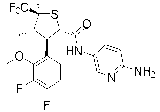 |
| 35 | 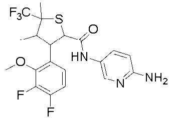 | 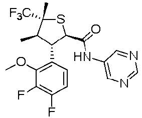 | 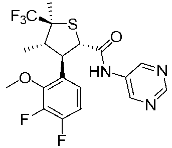 | 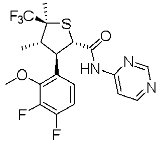 |
| 36 | 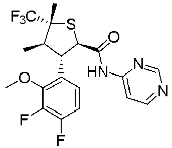 | 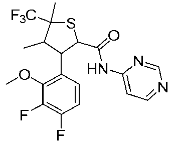 | 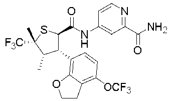 | 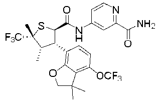 |
| 37 | 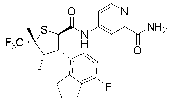 | 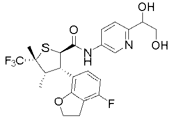 | 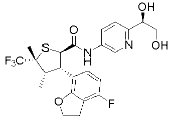 | 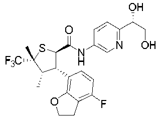 |
| 38 | 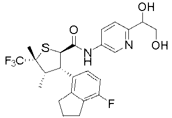 | 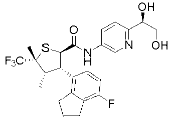 | 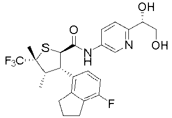 | 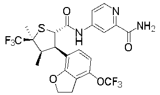 |
| 39 | 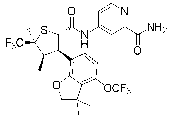 | 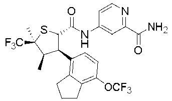 | 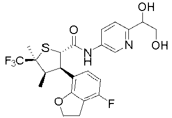 | 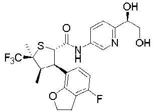 |
| 40 | 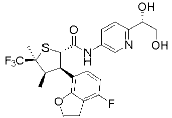 | 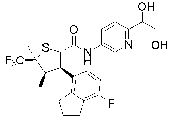 | 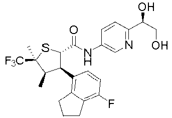 | 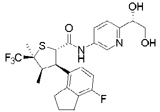 |
| 41 | 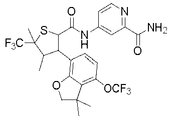 | 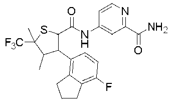 | 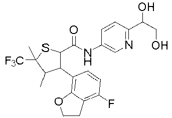 | 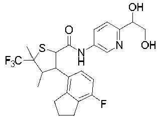 |
| 42 | 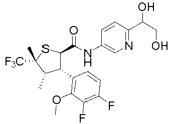 | 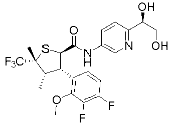 | 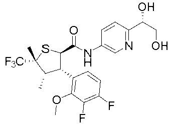 | 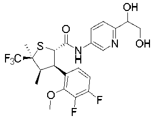 |
| 43 | 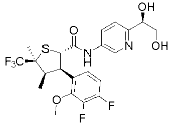 | 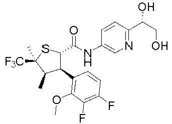 | 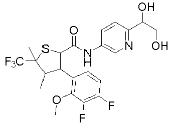 | 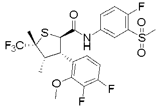 |
| 44 | 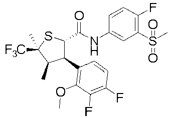 | 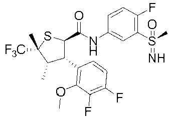 | 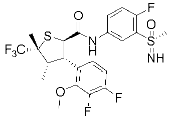 | 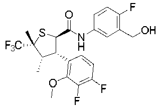 |
| 45 | 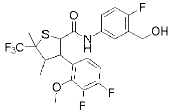 | 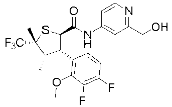 | 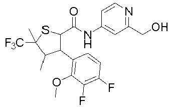 | 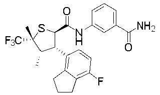 |
| 46 | 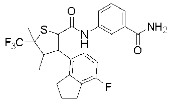 | 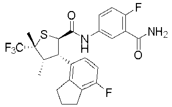 | 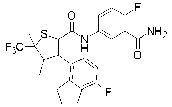 | 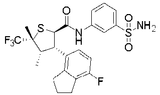 |
| 47 | 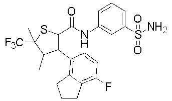 | 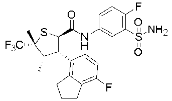 | 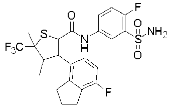 | 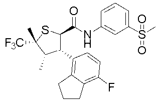 |
| 48 | 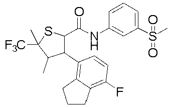 | 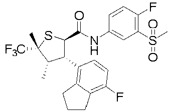 | 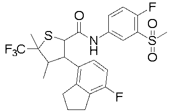 | 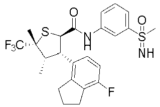 |
| 49 | 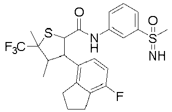 | 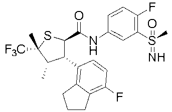 | 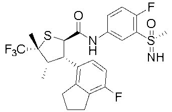 | 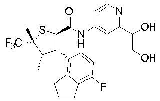 |
| 50 | 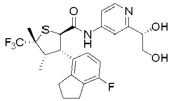 | 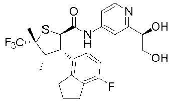 | 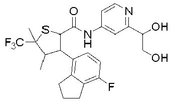 | 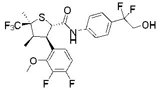 |
| 51 | 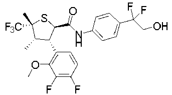 | 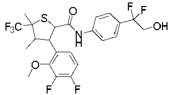 | 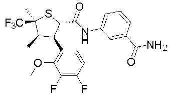 | 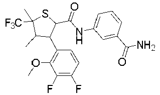 |
| 52 | 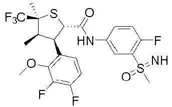 | 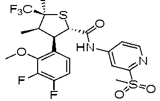 | 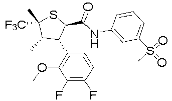 | 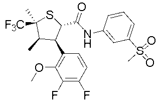 |
| 53 | 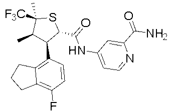 | 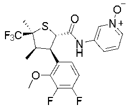 | 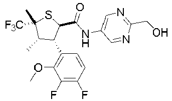 | 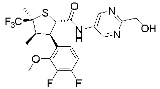 |
| 54 | 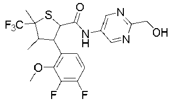 | 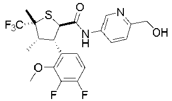 | 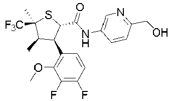 | 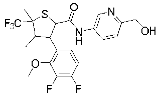 |
| 55 | 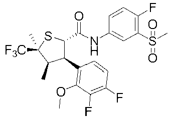 | 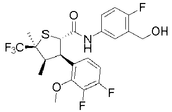 | 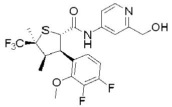 | 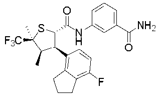 |
| 56 | 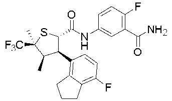 | 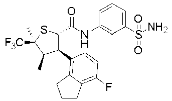 | 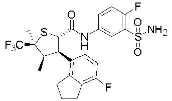 | 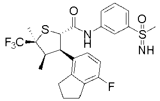 |
| 57 | 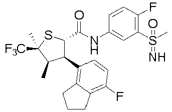 | 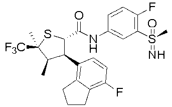 | 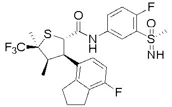 | 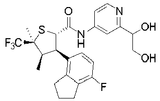 |
| 58 | 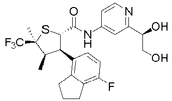 | 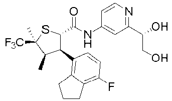 | 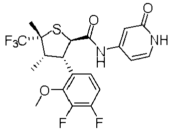 | 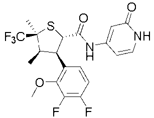 |
| 59 | 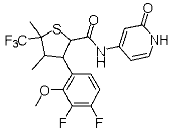 | 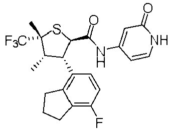 | 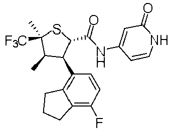 | 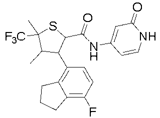 |
| 60 | 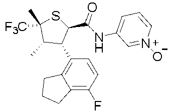 | 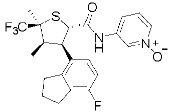 | 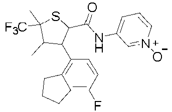 | 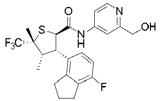 |
| 61 | 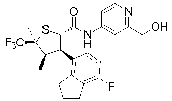 | 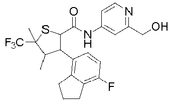 | 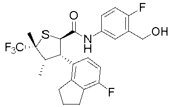 | 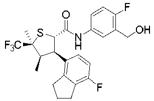 |
| 62 | 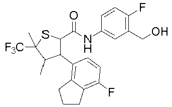 | 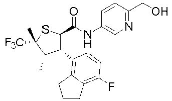 | 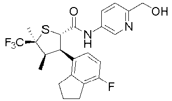 | 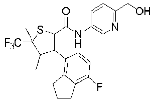 |
| 63 | 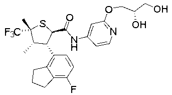 | 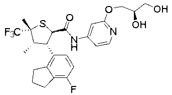 | 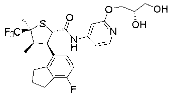 | 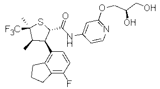 |
| 64 | 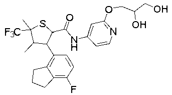 | 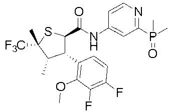 | 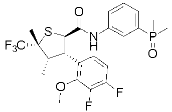 | 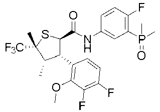 |
| 65 | 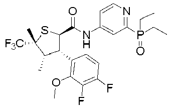 | 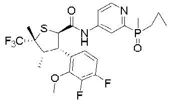 | 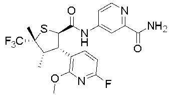 | 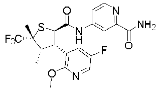 |
| 66 | 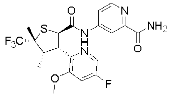 | 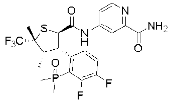 | 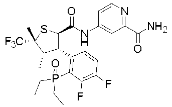 | 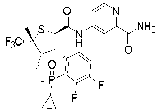 |
| 67 | 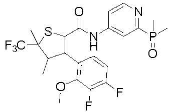 | 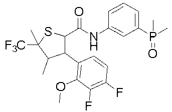 | 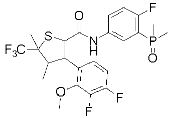 | 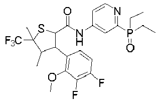 |
| 68 | 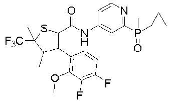 | 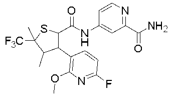 | 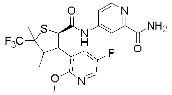 | 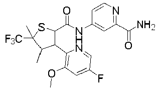 |
| 69 | 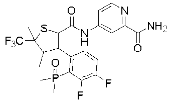 | 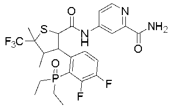 | 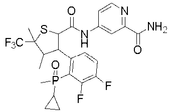 | 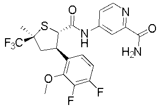 |
| 70 | 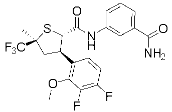 | 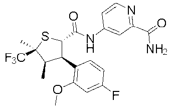 | 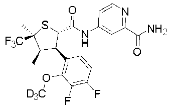 | 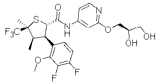 |
| 71 | 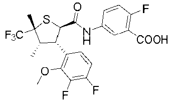 | 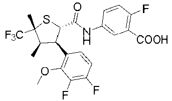 | 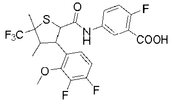 | 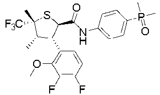 |
| 72 | 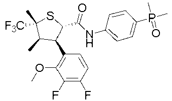 | 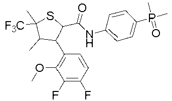 | 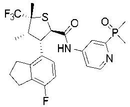 | 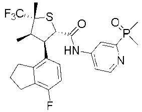 |
| 73 | 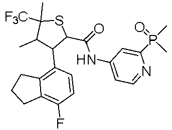 | 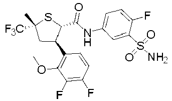 | 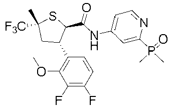 | 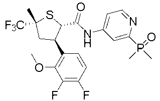 |
| 74 | 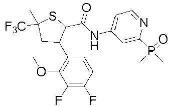 | 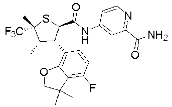 | 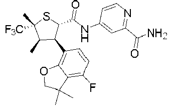 | 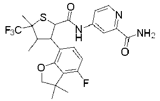 |
| 75 | 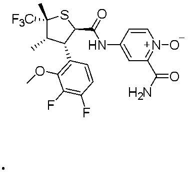 | 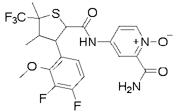 | 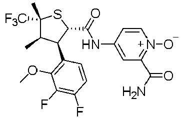 | |

The present invention relates to a pharmaceutical composition comprising any of the above-described compound or the stereoisomer, deuterated compound, solvate, prodrug, metabolite, pharmaceutically acceptable salt or cocrystal thereof, and a pharmaceutically acceptable carrier.

The present invention relates to a pharmaceutical composition comprising a therapeutically effective amount of the compound or the stereoisomer, deuterated compound, solvate, prodrug, metabolite, pharmaceutically acceptable salt or cocrystal thereof described above in the present invention, and a pharmaceutically acceptable carrier.

In some embodiments, the pharmaceutical composition of the present invention can be in a unit preparation form (the amount of the active drug in the unit preparation is also referred to as the "preparation specification").

"Effective amount" or "therapeutically effective amount" described in the present application refers to a sufficient amount of the compound disclosed in the present application that is administered to ameliorate, to some extent, one or more symptoms of the disease or condition being treated (e.g., treating and/or relieving pain). In some embodiments, the outcome is the reduction and/or remission of signs, symptoms or causes of the disease, or any other desired change in the biological system. For example, the "effective amount" in terms of the therapeutic use is an amount comprising the compound disclosed in the present application that is required to provide clinically significant reduction of the symptoms of the disease. Examples of the therapeutically effective amount include, but are not limited to 1-1500 mg, 1-1200 mg, 1-1000 mg, 1-900 mg, 1-800 mg, 1-700 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, 150-200 mg, 80-1000 mg, or 80-800 mg.

In some embodiments, the pharmaceutical composition comprises the compound or the stereoisomer, deuterated compound, solvate, prodrug, metabolite, pharmaceutically acceptable salt or cocrystal thereof of the present invention in an amount including, but not limited to 1-1000 mg, 20-800 mg, 40-800 mg, 40-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 300 mg, 320 mg, 400 mg, 480 mg, 500 mg, 600 mg, 640 mg, or 840 mg.

Provided is a method for treating a disease in a mammal, wherein the method comprises administering to a subject a therapeutically effective amount of the compound or the stereoisomer, deuterated compound, solvate, prodrug, metabolite, pharmaceutically acceptable salt or cocrystal thereof of the present invention, the therapeutically effective amount is preferably 1-1500 mg, and the disease is preferably pain to be treated or relieved.

Provided is a method for treating or alleviating a disease in a mammal, wherein the method comprises administering to a subject a drug, i.e., the compound or the stereoisomer, deuterated compound, solvate, prodrug, metabolite, pharmaceutically acceptable salt or cocrystal thereof of the present invention in a daily dose of 1-1000 mg/day, wherein the daily dose can be a single dose or divided doses. In some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day. In some embodiments, the daily dose includes, but is not limited to 10 mg/day, 20 mg/day, 25 mg/day, 50 mg/day, 80 mg/day, 100 mg/day, 125 mg/day, 150 mg/day, 160 mg/day, 200 mg/day, 300 mg/day, 320 mg/day, 400 mg/day, 480 mg/day, 600 mg/day, 640 mg/day, 800 mg/day, or 1000 mg/day.

The present invention relates to a kit, which can comprise a composition in a single dose or a multi-dose form. The kit comprises the compound or the stereoisomer, deuterated compound, solvate, prodrug, metabolite, pharmaceutically acceptable salt or cocrystal thereof of the present invention, wherein the amount of the compound or the stereoisomer, deuterated compound, solvate, prodrug, metabolite, pharmaceutically acceptable salt or cocrystal thereof of the present invention is the same as the amount thereof in the above-described pharmaceutical composition.

The present invention relates to the use of any of the above-described compound or the stereoisomer, deuterated compound, solvate, prodrug, metabolite, pharmaceutically acceptable salt or cocrystal thereof in the preparation of a drug for treating or/and relieving pain.

The present invention relates to the use of the above-described pharmaceutical composition in the preparation of a drug for treating or/and relieving pain.

The amount of the compound or the stereoisomer, deuterated compound, solvate, prodrug, metabolite, pharmaceutically acceptable salt or cocrystal thereof of the present invention is calculated in the form of a free base in each case.

### Synthesis method I:

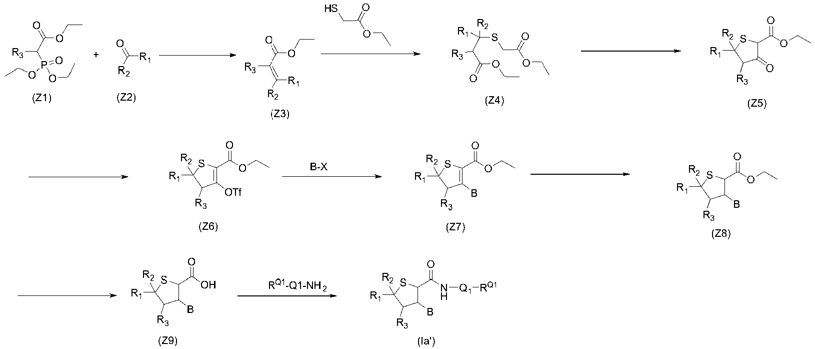

General formula (Z1) and general formula (Z2) are subjected to a Wittig reaction to obtain a corresponding general formula (Z3); the general formula (Z3) is subjected to an addition reaction with ethyl mercaptoacetate to obtain a corresponding general formula (Z4); the general formula (Z4) is subjected to an intramolecular ester condensation to obtain a corresponding general formula (Z5); the general formula (Z5) is protected by a protecting group to obtain a corresponding general formula (Z6); the general formula (Z6) and the fragment (B-X) are coupled to obtain a corresponding general formula (Z7); the general formula (Z7) is reduced to obtain a corresponding general formula (Z8); and the general formula (Z8) and the amino fragment (R^{Q1}-Q1-NH₂) are condensed to obtain a corresponding general formula (Ia').

X is halogen, boronic acid or boronate.

Unless otherwise stated, the terms used in the description and the claims have the following meanings.

The carbon, hydrogen, oxygen, sulphur, nitrogen, F, Cl, Br, I involved in the groups and compounds of the present invention all comprise their isotopes, and the carbon, hydrogen, oxygen, sulphur or nitrogen involved in the groups and compounds of the present invention is optionally replaced by one or more of their corresponding isotopes, wherein the isotopes of carbon comprise ¹²C, ¹³C and ¹⁴C, the isotopes of hydrogen comprise protium (H), deuterium (D, also known as heavy hydrogen), tritium (T, also known as superheavy hydrogen), the isotopes of oxygen comprise ¹⁶O, ¹⁷O and ¹⁸O, the isotopes of sulphur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"Halogen" refers to F, Cl, Br, or I.

"Halogen-substituted" refers to a substitution with F, Cl, Br, or I, including but not limited to a substitution with 1 to 10 substituents selected from F, Cl, Br, or I, or a substitution with 1 to 6 substituents selected from F, Cl, Br, or I, or a substitution with 1 to 4 substituents selected from F, Cl, Br, or I. "Halogen-substituted" is referred to simply as "halo".

"Alkyl" refers to substituted or unsubstituted linear or branched saturated aliphatic hydrocarbyl, including but not limited to alkyl of 1 to 20 carbon atoms, alkyl of 1 to 8 carbon atoms, alkyl of 1 to 6 carbon atoms, or alkyl of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Alkylene" refers to substituted or unsubstituted linear or branched divalent saturated hydrocarbyl, including -(CH₂)ᵥ- (v is an integer from 1 to 10), and examples of alkylene include, but are not limited to, methylene, ethylene, propylene, butylene, *etc.*

"Cycloalkyl" refers to substituted or unsubstituted saturated carbocyclic hydrocarbyl, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Heterocycloalkyl" refers to substituted or unsubstituted saturated heteroatom-containing cyclic hydrocarbyl, including but not limited to 3 to 10 atoms, 3 to 8 atoms, or 1 to 3 heteroatoms selected from N, O or S. The selectively substituted N and S in the ring of the heterocycloalkyl can be oxidized to various oxidation states. Heterocycloalkyl can be connected to a heteroatom or a carbon atom, heterocycloalkyl can be connected to an aromatic ring or a non-aromatic ring, and heterocycloalkyl can be connected to a bridged ring or a spiro ring. Non-limiting examples include oxiranyl, aziridinyl, oxetanyl, azetidinyl, tetrahydrofuryl, tetrahydro-2*H*-pyranyl, dioxolanyl, dioxanyl, pyrrolidyl, piperidyl, imidazolidinyl, oxazolidinyl, oxazinanyl, morpholinyl, hexahydropyrimidyl or piperazinyl. Heterocycloalkyl can be monovalent, divalent, trivalent or tetravalent.

"Alkenyl" refers to substituted or unsubstituted linear or branched unsaturated hydrocarbyl, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The definition of the "alkenyl" herein is consistent with this definition. Alkenyl can be monovalent, divalent, trivalent or tetravalent.

"Alkynyl" refers to substituted or unsubstituted linear or branched unsaturated hydrocarbyl, having at least 1, usually 1, 2 or 3 carbon-carbon triple bonds, with a main chain including but not limited to 2 to 10 carbon atoms, 2 to 6 carbon atoms, or 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, etc. Alkynyl can be monovalent, divalent, trivalent or tetravalent.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, 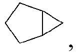 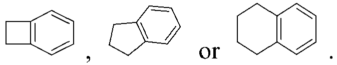 "Carbocyclyl" or "carbocyclic ring" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S. The selectively substituted N and S in the ring of the heterocyclyl can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl, 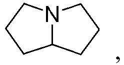 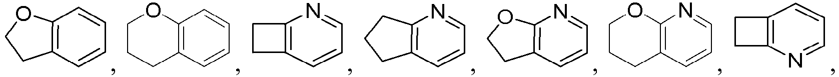 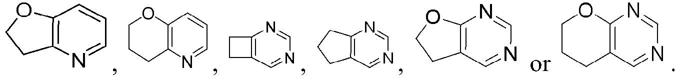 "Heterocyclyl" or "heterocyclic ring" can be monovalent, divalent, trivalent or tetravalent.

"Heteroaryl" or "heteroaromatic ring" refers to substituted or unsubstituted aromatic hydrocarbyl containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5 to 15, 5 to 10 or 5 to 6. Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring may be fused to a saturated or unsaturated carbocyclic ring or heterocyclic ring, wherein the ring connected to the parent structure is a heteroaryl ring. Non-limiting examples include 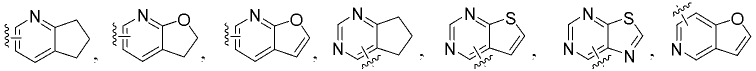 and 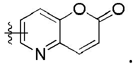 The definition of the "heteroaryl" herein is consistent with this definition. Heteroaryl can be monovalent, divalent, trivalent or tetravalent. When divalent, trivalent or tetravalent, the point of connection is on the heteroaryl ring.

"Substitution" or "substituted" refers to a substitution with 1 or more (including but not limited to 2, 3, 4 or 5) substituents including but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, -(CH₂)ₘ-C(=O)-R^{a}, -O-(CH₂)ₘ-C(=O)-R^{a}, -(CH₂)ₘ-C(=O)-NR^{b}R^{c}, -(CH₂)ₘS(=O)ₙR^{a}, -(CH₂)ₘ-alkenyl-R^{a}, OR^{d} or -(CH₂)ₘ-alkynyl-R^{a} (wherein m and n are 0, 1 or 2), arylthio, thiocarbonyl, silyl, -NR^{b}R^{c}, or other groups, wherein R^{b} and R^{c} are independently selected from H, hydroxyl, amino, carbonyl, alkyl, alkoxy, cycloalkyl, heterocyclyl, aryl, heteroaryl, sulfonyl, or trifluoromethylsulfonyl. Alternatively, R^{b} and R^{c} may form a five- or six-membered cycloalkyl or heterocyclyl; R^{a} and R^{d} are each independently selected from aryl, heteroaryl, alkyl, alkoxy, cycloalkyl, heterocyclyl, carbonyl, an ester group, a bridged ring group, a spiro ring group or a fused ring group.

"Substituted with 1 to X substituents selected from ..." refers to a substitution with 1, 2, 3 ... X substituents selected from ..., wherein X is selected from any integer between 1 and 10. For example, "substituted with 1 to 4 R^{k}" refers to a substitution with 1, 2, 3 or 4 R^{k}. For example, "substituted with 1 to 5 substituents selected from ..." refers to a substitution with 1, 2, 3, 4 or 5 substituents selected from ... For example, "bridged-heterocyclic ring is optionally substituted with 1 to 4 substituents selected from D or F" means that the bridged-heterocyclic ring is optionally substituted with 1, 2, 3 or 4 substituents selected from D or F.

An X- to Y-membered ring (X is an integer, 3 ≤ X < Y, and Y is selected from any integer between 4 and 12) includes X, X+1-, X+2-, X+3-, X+4-,...to Y-membered rings. Rings include a heterocyclic ring, a carbocyclic ring, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

"Optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or cocrystals thereof and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of a compound administered.

"Preparation specification" refers to the weight of the active drug contained in each vial, tablet or other unit preparation.

"Animal" includes mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerization and amide-imino alcohol isomerization.

### Detailed Description of Embodiments

The technical solution of the present invention will be explained in detail by the following examples; however, the scope of protection of the present invention includes but is not limited thereto.

The structures of the compounds are determined by nuclear magnetic resonance (NMR) or (and) mass spectrometry (MS). The NMR shift (δ) is given in the unit of 10⁻⁶ (ppm). NMR is determined with (Bruker Avance III 400 and Bruker Avance 300) nuclear magnetic resonance instrument; the solvents for determination are deuterated dimethyl sulfoxide (DMSO-d₆), deuterated chloroform (CDCl₃) and deuterated methanol (CD₃OD); and the internal standard is tetramethylsilane (TMS).

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM).

Prep-HPLC preparative condition (condition 1): instrument: Waters AutoP; preparative column: Sunfire C18 (19 mm×250 mm); preparation method: the sample was dissolved in methanol and filtered with a 0.45 µm filter to prepare a sample solution; mobile phase system: acetonitrile/water (0.1% trifluoroacetic acid); gradient elution, acetonitrile content from 35% to 80%; flow rate: 15 mL/min; elution time: 20 minutes.

Prep-HPLC preparative condition (condition 2): instrument: Waters 2767 preparative liquid phase chromatographic instrument; preparative column: SunFire C18 (19 mm×250 mm); the sample was dissolved in DMF and filtered with a 0.45 µm filter to prepare a sample solution; mobile phase system: acetonitrile/water (0.1% trifluoroacetic acid); gradient elution, acetonitrile content from 10% to 70%; flow rate: 17 mL/min; elution time: 20 minutes.

Prep-HPLC (neutral preparation): instrument: Waters AutoP; preparative column: XSelect C18 (19 mm×250 mm); preparation method: the crude was dissolved in methanol and filtered with a 0.45 µm filter to prepare a sample solution; mobile phase system: acetonitrile/water (5 mM ammonium acetate); gradient elution, acetonitrile content from 30% to 80%; flow rate: 15 mL/min; elution time: 20 minutes.

Yantai Huanghai HSGF254 or Qingdao GF254 silica gel plate is used as a thin layer chromatography silica plate, wherein the silica gel plate for the thin layer chromatography (TLC) is of the specification of 0.15 mm-0.20 mm, and the specification when separating and purifying a product by thin layer chromatography is 0.4 mm - 0.5 mm;

and for the column chromatography, Yantai Huanghai silica gel of 200-300 mesh silica gel is generally used as a carrier.

To achieve the objectives of the present invention, according to organic synthesis techniques known to those skilled in the art, and starting from commercially available chemicals and/or compounds described in chemical documents, the prepared compounds, "commercially available chemicals", for use in the reactions described herein are obtained from standard commercial sources, including Shanghai Aladdin Bio-Chem Technology Co., Ltd., Shanghai Macklin Biochemical Co., Ltd., Sigma-Aldrich, Alfa Aesar (China) Chemical Co., Ltd., Tokyo Chemical Industry (Shanghai) Co., Ltd., Energy Chemical Co., Ltd., Shanghai Titan Scientific Co., Ltd., Kelong Chemical Co., Ltd., J&K Scientific and the like.

TCFH: tetramethylchloroformamidinium hexafluorophosphate; THF: tetrahydrofuran; DMF: N, N-dimethylformamide; DIPEA: N,N-diisopropylethylamine; HATU: CAS 148893-10-1; T3P (50% wt in EtOAc): CAS 68957-94-8
Retention time: the retention time corresponding to the analytical method, unless otherwise specified in the examples.

### Example 1: Preparation of compound 1-1a and compound 1-1b

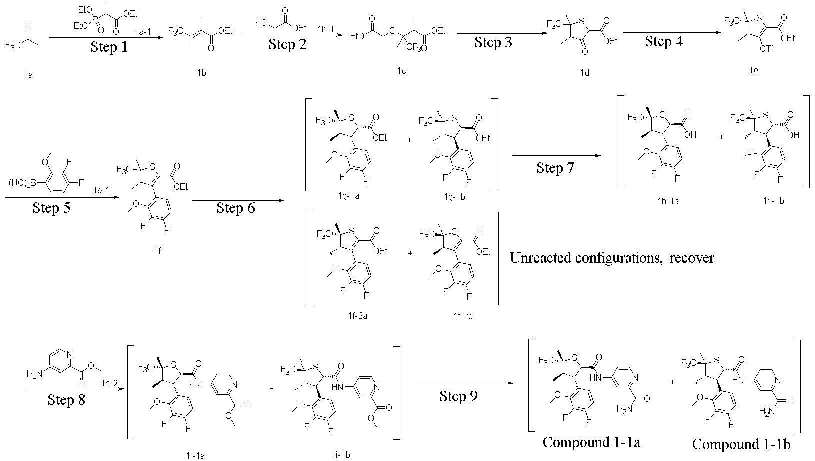

### Step 1: Preparation of compound 1b

In an ice bath, 1a-1 (30.96 g, 129.95 mmol) was dissolved in tetrahydrofuran (130 mL), and sodium hydride (5.20 g, 130 mmol) was added in batches. The mixture was reacted under nitrogen atmosphere and in an ice bath for 30 minutes. 1a (11.20 g, 100 mmol) was dissolved in tetrahydrofuran (20 mL), and the mixture was added dropwise to the reaction system. Under nitrogen atmosphere, the mixture was naturally heated to room temperature and reacted for 18 h. In an ice bath, the reaction system was adjusted to pH = 7-8 by slowly dropwise adding 1 N hydrochloric acid, and extracted with diethyl ether (150 mL × 3). The organic phases were combined, dried over anhydrous sodium sulphate, filtered and concentrated (at room temperature) to obtain a crude, which was then purified by silica gel column chromatography to obtain 1b (14.1 g, yield: 71.88%, E/Z configuration mixture).

### Step 2: Preparation of compound 1c

In an ice bath, 1b-1 (8.63 g, 71.88 mmol) was added to a round-bottom flask, piperidine (1.22 g, 14.38 mmol) was added dropwise to the system, and then 1b (6.0 g, 71.88 mmol) was added dropwise. Under nitrogen protection, the mixture was reacted at 50°C for 24 h. In an ice bath, the reaction was quenched by adding 0.1 N hydrochloric acid (100 mL), and the mixture was extracted with diethyl ether (100 mL×3). The organic phases were combined, dried over anhydrous sodium sulphate, filtered and concentrated (at 25°C) to obtain 1c as a crude, which was then purified by silica gel column chromatography to obtain 1c (6.3 g, yield: 27.96%).
LC-MS m/z = 317.1 [M+H]⁺

### Step 3: Preparation of compound 1d

In an ice bath, 1c (6.3 g, 19.92 mmol) was dissolved in diethyl ether (120 mL). Under nitrogen protection, potassium tert-butoxide (2.91 g, 25.90 mmol) was slowly added dropwise to the system, and the mixture was reacted in an ice bath for 2 h. In an ice bath, the reaction was quenched by adding glacial acetic acid (1.56 mL) and water (100 mL) to the reaction system, and the mixture was extracted with diethyl ether (100 mL×3). The organic phases were combined, dried over anhydrous sodium sulphate, filtered and concentrated to obtain 1d.

### Step 4: Preparation of compound 1e

1d (4.05 g, 15 mmol) was dissolved in dichloromethane (40 mL) and precooled at -78°C for 15 minutes. Under nitrogen atmosphere, N,N-diisopropylethylamine (2.32 g, 17.99 mmol) was added dropwise, and trifluoromethanesulfonic anhydride (4.23 g, 15 mmol) was dissolved in dichloromethane (10 mL) and slowly added dropwise to the system. After the addition, the mixture was further reacted at -78°C for 2 h. In an ice bath, the reaction was quenched by slowly adding a saturated aqueous sodium bicarbonate solution (50 mL) to the reaction system, and the mixture was extracted with dichloromethane (50 mLx3). The organic phases were combined, dried over anhydrous sodium sulphate, filtered and concentrated to obtain a crude, which was then purified by silica gel column chromatography to obtain 1e (5.2 g, yield: 86.16%).

### Step 5: Preparation of compound 1f

1e (5.2 g, 12.92 mmol) was dissolved in toluene (50 mL), and 1e-1 (2.67 g, 14.21 mmol) and Pd(PPh₃)₄ (0.75 g, 0.65 mmol) were sequentially added. Potassium phosphate (8.23 g, 38.71 mmol) was prepared into a 2 M aqueous solution and added to the system, and the mixture was reacted at 100°C under nitrogen atmosphere for 6 h. The reaction was cooled to room temperature. The reaction system was filtered, and the filter cake was washed with ethyl acetate (10 mL×2). The filtrate was allowed to stand for layering, and the organic phase was collected. The aqueous phase was extracted with ethyl acetate (30 mL×3). The organic phases were combined, dried over anhydrous sodium sulphate, filtered and concentrated to obtain a crude, which was then purified by silica gel column chromatography to obtain 1f (5.1 g, yield: 99.59%).
LC-MS m/z = 397.1 [M+H]⁺

### Step 6: Preparation of compounds 1g-1a and 1g-1b

1f (3.7 g, 9.33 mmol) was dissolved in methanol (50 mL), and palladium on carbon (1.99 g, 18.56 mmol) was added. After the addition, under hydrogen atmosphere, the pressure was increased to 2 Mpa, and the mixture was reacted at room temperature for 24 h. The reaction was filtered, and the filtrate was concentrated to obtain a crude, which was then purified by silica gel column chromatography to obtain a mixture of 1g-1a and 1g-1b (1.02 g, yield: 27.44%), and a mixture of unreacted configuration raw materials 1f-2a and 1f-2b (0.148 g, yield: 4.00%) was recovered.

### Mixture of 1g-1a and 1g-1b:

LC-MS m/z = 399.4 [M+H]⁺
¹H NMR (400 MHz, CDCl₃): δ 6.96 - 6.90 (m, 1H), 6.88 - 6.79 (m, 1H), 4.08 - 4.01 (m, 4H), 3.94 - 3.80 (m, 2H), 3.77 - 3.68 (m, 1H), 3.49 - 3.37 (m, 1H), 1.59 (s, 3H), 1.02 (t, 3H), 0.96 (d, 3H).

### Mixture of 1f-2a and 1f-2b:

LC-MS m/z = 397.1 [M+H]⁺
¹H NMR (400 MHz, CDCl₃): δ 6.90 - 6.81 (m, 1H), 6.77 - 6.71 (m, 1H), 4.13 - 4.04 (m, 2H), 3.92 (d, 3H), 3.42 - 3.34 (m, 1H), 1.77 (s, 3H), 1.13 (t, 3H), 1.09 - 1.04 (m, 3H).

### Step 7: Preparation of compounds 1h-la and 1h-1b

Under nitrogen atmosphere and in an ice bath, the mixture of 1g-la and 1g-1b (1.02 g, 2.56 mmol) was dissolved in tetrahydrofuran (25 mL) and precooled for 15 minutes. Potassium tert-butoxide (0.95 g, 8.46 mmol) was slowly added dropwise to the system (internal temperature < 13°C). After the addition, the mixture was further reacted in an ice bath for 2 h. In an ice bath, the system was adjusted to pH = 1 by slowly dropwise adding 1 N hydrochloric acid (internal temperature < 13°C), and then water (10 mL) was added. The mixture was extracted with ethyl acetate (25 mLx3). The organic phases were combined, dried over anhydrous sodium sulphate, filtered and concentrated to obtain a mixture of 1h-1a and 1h-1b.
LC-MS m/z = 369.0 [M-H]⁻

Step 8: Preparation of compounds 1i-1a and 1i-1b

The mixture of 1h-1a and 1h-1b (0.90 g, 2.44 mmol) was dissolved in DMF (20 mL), and 1h-2 (0.56 g, 3.68 mmol), TCFH (1.37 g, 4.88 mmol) and N-methylimidazole (0.40 g, 4.88 mmol) were sequentially added. After the addition, the mixture was reacted at room temperature under nitrogen atmosphere for 18 h. The reaction was quenched by adding water (20 mL) to the reaction system. The mixture was extracted with ethyl acetate (30 mL×3). The organic phases were combined, washed with a saturated sodium chloride aqueous solution (90 mL), collected, dried over anhydrous sodium sulphate, filtered and concentrated to obtain a crude, which was then purified by silica gel column chromatography to obtain a mixture of 1i-1a and 1i-1b (0.526 g, yield: 42.65%).
LC-MS m/z = 505.2 [M+H]⁺

### Step 9: Preparation of compound 1-la and compound 1-1b

The mixture of 1i-1a and 1i-1b (0.52 g, 1.04 mmol) was dissolved in a 7 M ammonia methanol solution (5 mL), and the resulting mixture was reacted at room temperature for 18 h. The reaction system was concentrated to obtain a crude, which was then subjected to silica gel column chromatography to obtain a mixture of compound 1-1a and compound 1-1b (0.38 g, yield: 74.50%).
LC-MS m/z = 490.2 [M+H]⁺;
¹H NMR (400 MHz, DMSO-*d₆*): δ 10.74 (s, 1H), 8.45 (d, 1H), 8.14 (d, 1H), 8.07 - 7.99 (m, 1H), 7.67 - 7.63 (m, 1H), 7.62 - 7.56 (m, 1H), 7.21 - 7.12 (m, 2H), 4.47 (d, 1H), 4.00 - 3.82 (m, 4H), 2.84 - 2.72 (m, 1H), 1.61 (s, 3H), 0.83 (d, 3H).

### Example 2: Preparation of compound 1-2a and compound 1-2b

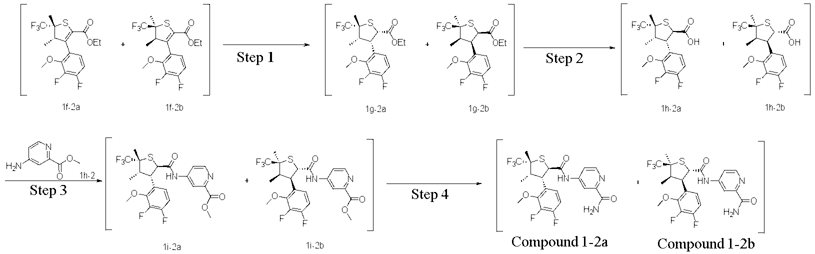

### Step 1: Preparation of compounds 1g-2a and 1g-2b

The mixture of 1f-2a and 1f-2b (0.148 g, 0.38 mmol) was dissolved in methanol (10 mL), and palladium on carbon (0.15 g, 1.41 mmol) was added. After the addition, under hydrogen atmosphere, the pressure was increased to 2.5 Mpa, and the mixture was reacted at 90°C for 24 h. The reaction was filtered, and the filtrate was concentrated to obtain a crude, which was then purified by silica gel column chromatography to obtain a mixture of 1g-2a and 1g-2b (0.052 g, yield: 34.35%).

### Step 2: Preparation of compounds 1h-2a and 1h-2b

Under nitrogen atmosphere and in an ice bath, the mixture of 1g-2a and 1g-2b (0.052 g, 0.13 mmol) was dissolved in tetrahydrofuran (5 mL) and precooled for 15 minutes. Potassium tert-butoxide (0.048 g, 0.43 mmol) was slowly added dropwise to the system (internal temperature < 13°C). After the addition, the mixture was reacted in an ice bath for 2 h. In an ice bath, the system was adjusted to pH = 1 by slowly dropwise adding 1 N hydrochloric acid (internal temperature < 13°C), and water (5 mL) was added. The mixture was extracted with ethyl acetate (10 mLx3). The organic phases were combined, dried over anhydrous sodium sulphate, filtered and concentrated to obtain a mixture of 1h-2a and 1h-2b.
LC-MS m/z = 369.0 [M-H]⁻

### Step 3: Preparation of compounds 1i-2a and 1i-2b

The mixture of 1h-2a and 1h-2b (0.048 g, 0.13 mmol) was dissolved in DMF (3 mL), and 1h-2 (0.03 g, 0.20 mmol), TCFH (0.073 g, 0.26 mmol) and N-methylimidazole (0.022 g, 0.26 mmol) were sequentially added. The mixture was reacted at room temperature under nitrogen atmosphere for 18 h. The reaction was quenched by adding water (10 mL) to the reaction system. The mixture was extracted with ethyl acetate (10 mL×3). The organic phases were combined, washed with a saturated sodium chloride aqueous solution (30 mL), collected, dried over anhydrous sodium sulphate, filtered and concentrated to obtain a crude, which was then purified by silica gel column chromatography to obtain a mixture of 1i-2a and 1i-2b (0.01 g, yield: 15.25%).
LC-MS m/z = 505.2 [M+H]⁺

### Step 4: Preparation of compound 1-2a and compound 1-2b

The mixture of 1i-2a and 1i-2b (0.01 g, 0.02 mmol) was dissolved in a 7 M ammonia methanol solution (3 mL), and the resulting mixture was reacted at room temperature for 8 h. The reaction system was concentrated to obtain a crude, which was then subjected to silica gel column chromatography to obtain a mixture of compound 1-2a and compound 1-2b (0.006 g, yield: 61.91%).
LC-MS m/z = 490.2 [M+H]⁺;
¹H NMR (400 MHz, DMSO-*d₆*): δ 10.92 (s, 1H), 8.47 (d, 1H), 8.17 (d, 1H), 8.06 - 8.00 (m, 1H), 7.70 - 7.64 (m, 1H), 7.63 - 7.57 (m, 1H), 7.22 - 7.06 (m, 2H), 4.86 (d, 1H), 4.54 - 4.46 (m, 1H), 4.01 (d, 3H), 2.66 - 2.58 (m, 1H), 1.85 (s, 3H), 0.83 - 0.75 (m, 3H).

### SFC preparative conditions:

instrument: Waters 150 Prep-SFC C; preparative column: Chiralcel OX Column; preparation method: the crude was dissolved in acetonitrile to prepare a 10 mg/ml sample solution; mobile phase system: carbon dioxide/methanol, with methanol content of 30%; elution time: 8 min;
the mixture of compounds 1-2a and 1-2b (504 mg) was subjected to preparative SFC and lyophilized to obtain compound 1-2P1 (193.8 mg, retention time: 1.137 min) and compound 1-2P2 (218 mg, retention time: 1.526 min); chiral testing method: (instrument: SHIMADZU LC-30 AD sf; chiral column: Chiralcel OX Column; preparation method: the crude was dissolved in acetonitrile to prepare a sample solution; mobile phase system: carbon dioxide/0.05% DEA in methanol; elution gradient: 5%-40%; flow rate: 3.0 mL/min; elution time: 3 min).

Compound 1-2P1 has a structure as shown in either formula 1-2a or formula 1-2b, and is an enantiomer of compound 1-2P2, that is, when compound 1-2P1 has a structure as shown in formula 1-2a, compound 1-2P2 has a structure as shown in formula 1-2b; and when compound 1-2P1 has a structure as shown in formula 1-2b, compound 1-2P2 has a structure as shown in formula 1-2a. The nuclear magnetic resonance and mass spectrometry of compound 1-2P1 and compound 1-2P2 show no differences, and are consistent with those of the mixture of compound 1-2a and compound 1-2b.

### Example 3: Preparation of compound 3-2a and compound 3-2b

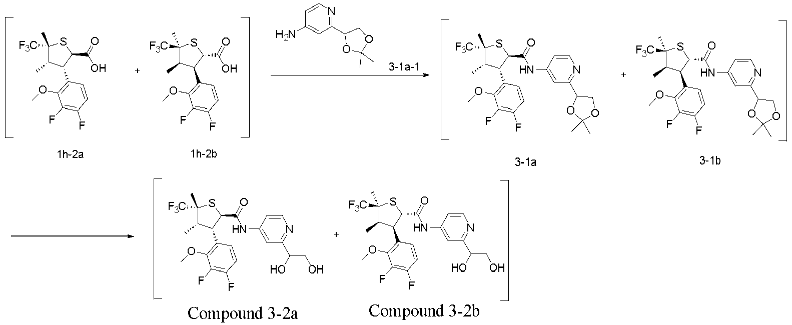

### Step 1: Preparation of compounds 3-1a and 3-1b

The mixture of 1h-2a and 1h-2b (0.052 g, 0.14 mmol) was dissolved in ethyl acetate (3 mL), and triethylamine (0.042 g, 0.42 mmol) and T3P (50% wt in EtOAc 0.36 g; containing 1-propylphosphoric anhydride 0.18 g, 0.56 mmol) were sequentially added. A solution of 3-1a-1 (0.041 g, 0.21 mmol) in ethyl acetate (1 mL) was added dropwise, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then purified by silica gel column chromatography to obtain a mixture of 3-1a and 3-1b (0.03 g, yield: 39.21%).
LCMS m/z = 547.1 [M+H]⁺

### Step 2: Preparation of compound 3-2a and compound 3-2b

The mixture of 3-1a and 3-1b (0.03 g, 0.027 mmol) was dissolved in dichloromethane (3 mL), and trifluoroacetic acid (0.6 mL) was added dropwise. The mixture was reacted at room temperature for 2 h. The reaction system was concentrated to obtain a crude, which was then subjected to prep-HPLC (condition 1) to obtain the trifluoroacetate of a mixture of 3-2a and compound 3-2b (0.016 g, yield: 58.5%).
LCMS m/z = 507.4 [M+H]⁺;

### Example 4: Preparation of compound 4-1a and compound 4-1b

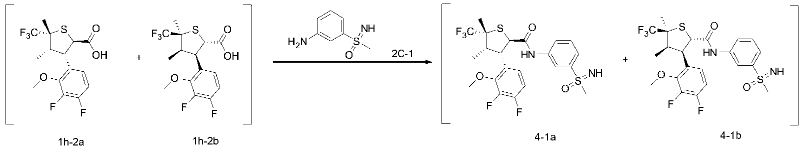

### Step 1: Preparation of compounds 4-1a and 4-1b

The mixture of 1h-2a and 1h-2b (0.052 g, 0.14 mmol) was dissolved in ethyl acetate (3 mL), and triethylamine (0.042 g, 0.42 mmol) and T3P (50% wt in EtOAc 0.36 g; containing 1-propylphosphoric anhydride 0.18 g, 0.56 mmol) were sequentially added. 2C-1 (0.048 g, 0.28 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then purified by prep-HPLC (condition 1) to obtain the trifluoroacetate of a mixture of 4-1a and 4-1b (0.006 g, yield: 8.2%).
LCMS m/z = 523.5 [M+H]⁺
¹H NMR (400 MHz, DMSO-*d₆*) δ 10.78 (d, 1H), 8.31 - 8.25 (m, 1H), 7.78 - 7.72 (m, 1H), 7.69 - 7.64 (m, 1H), 7.62 - 7.56 (m, 1H), 7.22 - 7.13 (m, 1H), 7.11 - 7.05 (m, 1H), 4.87 (d, 1H), 4.54 - 4.45 (m, 1H), 4.01 (d, 3H), 3.27 (d, 3H), 2.68 - 2.59 (m, 1H), 1.86 (s, 3H), 0.83 - 0.74 (m, 3H).

### Example 5: Preparation of compound 5-3a and compound 5-3b

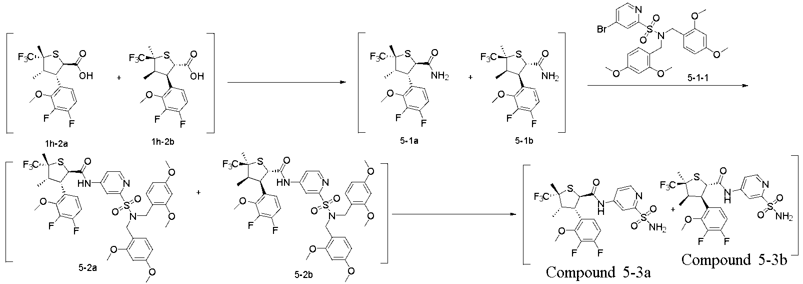

### Step 1: Preparation of compounds 5-1a and 5-1b

The mixture of 1h-2a and 1h-2b (0.022 g, 0.06 mmol) was dissolved in ethyl acetate (3 mL), and triethylamine (0.036 g, 0.36 mmol) and T3P (50% wt in EtOAc 0.152 g; containing 1-propylphosphoric anhydride 0.076 g, 0.24 mmol) were sequentially added. Aqueous ammonia (0.008 g, 0.12 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a mixture of 5-1a and 5-1b.
LCMS m/z = 370.1 [M+H]⁺

### Step 2: Preparation of compounds 5-2a and 5-2b

Under nitrogen atmosphere and in an ice bath, the mixture of 5-1a and 5-1b (0.06 mmol) was dissolved in 1,4-dioxane (3 mL), and 5-1-1 (0.048 g, 0.09 mmol), Xantphos Pd G2 (CAS: 1375325-77-1) (0.006 g, 0.006 mmol) and caesium carbonate (0.057 g, 0.18 mmol) were sequentially added. The mixture was reacted at 100°C under nitrogen atmosphere for 16 h. The reaction was cooled to room temperature, and the reaction system was quenched by adding water (15 mL). The mixture was extracted with ethyl acetate (15 mLx3). The organic phases were combined, dried over anhydrous sodium sulphate, filtered and concentrated to obtain a crude, which was then purified by silica gel column chromatography to obtain a mixture of 5-2a and 5-2b (0.026 g, yield: 52.47%).

### Step 3: Preparation of compounds 5-3a and 5-3b

The mixture of 5-2a and 5-2b (0.026 g, 0.016 mmol) was dissolved in dichloromethane (3 mL), and trifluoroacetic acid (0.6 mL) was added dropwise. The mixture was reacted at room temperature for 2 h. In an ice bath, a saturated sodium bicarbonate solution (15 mL) was slowly added dropwise to the reaction system, and the mixture was extracted with dichloromethane (15 mL×3). The organic phases were combined, dried over anhydrous sodium sulphate, filtered and concentrated to obtain a crude, which was then purified by silica gel column chromatography to obtain a mixture of 5-3a and 5-3b (0.012 g, yield: 71.36%).
LCMS m/z = 526.0 [M+H]⁺;

### Example 6: Preparation of compound 6-2a and compound 6-2b

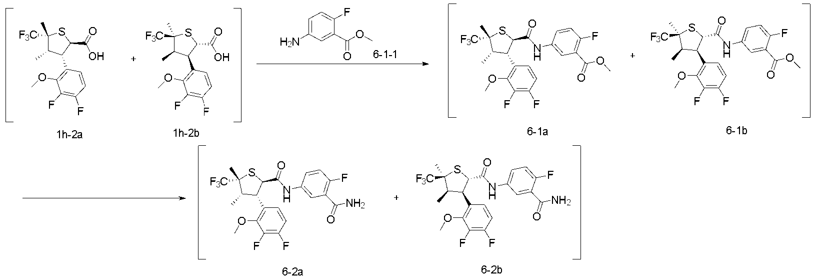

### Step 1: Preparation of compounds 6-1a and 6-1b

The mixture of 1h-2a and 1h-2b (0.049 g, 0.13 mmol) was dissolved in ethyl acetate (3 mL), and triethylamine (0.079 g, 0.78 mmol) and T3P (50% wt in EtOAc 0.33 g; containing 1-propylphosphoric anhydride 0.165 g, 0.52 mmol) were sequentially added. 6-1-1 (0.033 g, 0.20 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then purified by silica gel column chromatography to obtain a mixture of 6-1a and 6-1b (0.056 g, yield: 82.61%).
LCMS m/z = 522.2 [M+H]⁺

### Step 2: Preparation of compounds 6-2a and 6-2b

The mixture of 6-1a and 6-1b (0.056 g, 0.054 mmol) was dissolved in a 7 M ammonia methanol solution (5 mL), and the resulting mixture was reacted at room temperature for 18 h. The reaction system was concentrated to obtain a crude, which was then subjected to prep-HPLC (condition 1) to obtain a mixture of 6-2a and 6-2b (0.036 g, yield: 65.82%).
LCMS m/z = 507.2 [M+H]⁺;
¹H NMR (400 MHz, DMSO-*d₆*) δ 10.52 (s, 1H), 7.87 - 7.81 (m, 1H), 7.69 - 7.56 (m, 3H), 7.25 - 7.13 (m, 2H), 7.12 - 7.04 (m, 1H), 4.83 (d, 1H), 4.52 - 4.45 (m, 1H), 4.00 (d, 3H), 2.66 - 2.55 (m, 1H), 1.85 (s, 3H), 0.84 - 0.72 (m, 3H).

### Example 7: Preparation of compound 7-1a and compound 7-1b

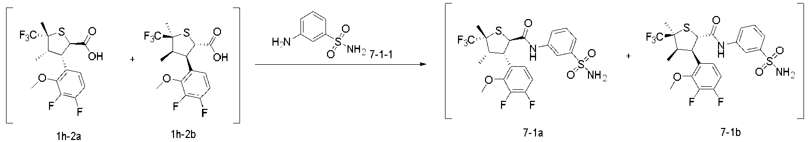

### Preparation of compounds 7-1a and 7-1b

The mixture of 1h-2a and 1h-2b (0.050 g, 0.14 mmol) was dissolved in ethyl acetate (3 mL), and triethylamine (0.042 g, 0.42 mmol) and T3P (50% wt in EtOAc 0.35 g; containing 1-propylphosphoric anhydride 0.175 g, 0.55 mmol) were sequentially added. 7-1-1 (0.033 g, 0.20 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then subjected to prep-HPLC (condition 1) and lyophilized to obtain a mixture of 7-1a and 7-1b (0.020 g, yield: 27.24%).
LCMS m/z = 525.2 [M+H]⁺

### Example 8: Preparation of compound 8-1a and compound 8-1b

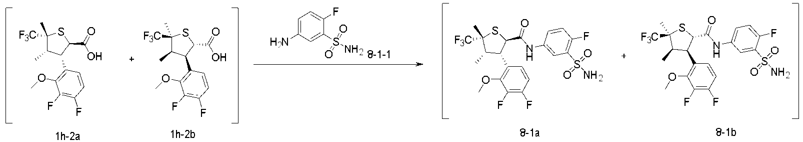

### Preparation of compounds 8-1a and 8-1b

The mixture of 1h-2a and 1h-2b (0.050 g, 0.14 mmol) was dissolved in ethyl acetate (3 mL), and triethylamine (0.042 g, 0.42 mmol) and T3P (50% wt in EtOAc 0.35 g; containing 1-propylphosphoric anhydride 0.175 g, 0.55 mmol) were sequentially added. 8-1-1 (0.040 g, 0.21 mmol, reference: WO 2020/028724) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then subjected to prep-HPLC (condition 1) and lyophilized to obtain a mixture of 8-1a and 8-1b (0.007 g, yield: 9.22%).
LCMS m/z = 543.2 [M+H]⁺
¹H NMR (400 MHz, CDCl₃) δ 8.11 (s, 1H), 7.98 - 7.92 (m, 1H), 7.81 - 7.76 (m, 1H), 7.17 (t, 1H), 7.05 - 6.98 (m, 1H), 6.91 - 6.83 (m, 1H), 5.04 (s, 2H), 4.57 (d, 1H), 4.46 (dd, 1H), 4.04 (d, 3H), 2.68 - 2.59 (m, 1H), 1.92 (s, 3H), 0.91 - 0.85 (m, 3H).

### Example 9: Preparation of compound 9-1a and compound 9-1b

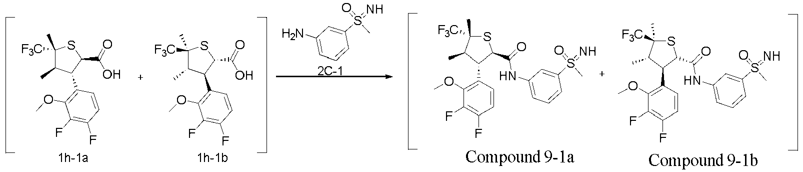

The mixture of 1h-1a and 1h-1b (50 mg, 0.14 mmol) was dissolved in DMF (3 mL), and 2C-1 (36 mg, 0.21 mmol), TCFH (79 mg, 0.28 mmol) and N-methylimidazole (34 mg, 0.41 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 9-1a and compound 9-1b (15 mg, yield: 20.95%).
LCMS m/z = 523.5 [M+H]⁺

### Example 10: Preparation of compound 10-1a and compound 10-1b

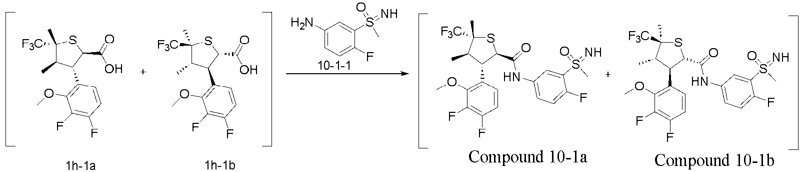

The mixture of 1h-1a and 1h-1b (50 mg, 0.14 mmol) was dissolved in DMF (3 mL), and 10-1-1 (40 mg, 0.21 mmol), TCFH (79 mg, 0.28 mmol) and N-methylimidazole (34 mg, 0.42 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 10-1a and compound 10-1b (9.73 mg, yield: 12.86%).
LCMS m/z = 541.5 [M+H]⁺

### Example 11: Preparation of compound 11-1a and compound 11-1b

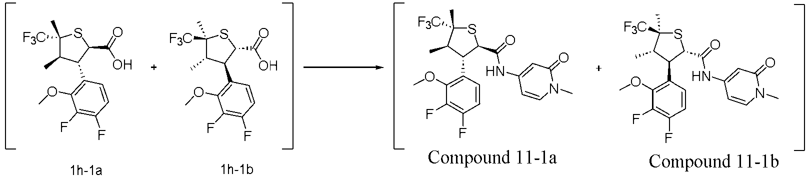

The mixture of 1h-1a and 1h-1b (100 mg, 0.27 mmol) was dissolved in EA (3 mL), and 4-amino-1-methylpyridine-ethanone (40 mg, 0.32 mmol), T3P (50% wt in EtOAc 680 mg; containing 1-propylphosphoric anhydride 340 mg, 1.08 mmol) and Et₃N (82 mg, 0.81 mmol) were sequentially added. The mixture was reacted at room temperature under nitrogen atmosphere for 18 h. Water (5 mL) was added. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 11-1a and compound 11-1b (30 mg, yield: 23.32%).
LCMS m/z = 477.1 [M+H]⁺

### Example 12: Preparation of compound 12-1a and compound 12-1b

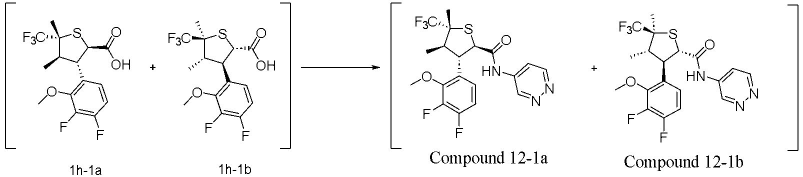

The mixture of 1h-1a and 1h-1b (100 mg, 0.27 mmol) was dissolved in EA (3 mL), and 4-aminopyridazine (31 mg, 0.33 mmol), T3P (50% wt in EtOAc 680 mg; containing 1-propylphosphoric anhydride 340 mg, 1.08 mmol) and Et₃N (82 mg, 0.81 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 12-1a and compound 12-1b (93.92 mg, yield: 77.75%).
LCMS m/z = 448.2 [M+H]⁺

### Example 13: Preparation of compound 13-1a and compound 13-1b

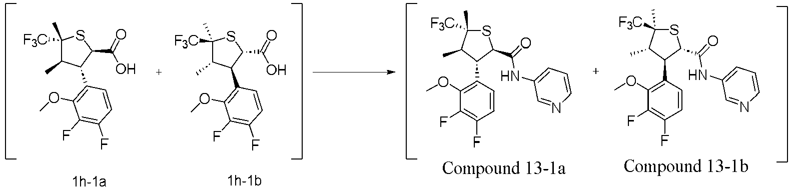

The mixture of 1h-1a and 1h-1b (100 mg, 0.27 mmol) was dissolved in EA (3 mL), and 3-aminopyridine (30 mg, 0.32 mmol), T3P (50% wt in EtOAc 680 mg; containing 1-propylphosphoric anhydride 340 mg, 1.08 mmol) and Et₃N (82 mg, 0.81 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 13-1a and compound 13-1b (110 mg, yield: 91.26%).
LCMS m/z = 447.1 [M+H]⁺

### Example 14: Preparation of compound 14-2a and compound 14-2b

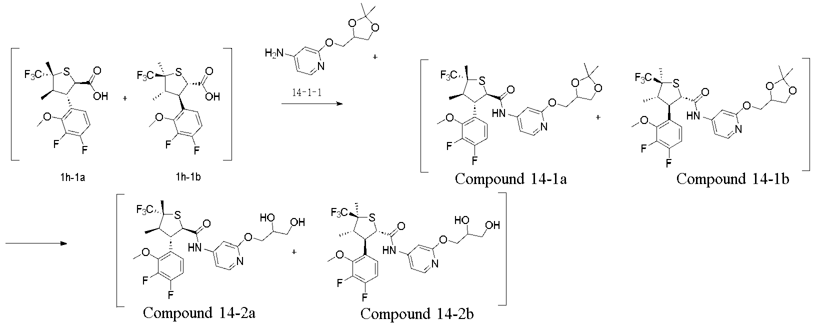

### Step 1: Preparation of compound 14-1a and compound 14-1b

The mixture of 1h-1a and 1h-1b (100 mg, 0.27 mmol) was dissolved in DMF (3 mL), and 14-1-1 (prepared with reference to WO 2022121517 A1) (91 mg, 0.41 mmol), TCFH (150 mg, 0.53 mmol) and N-methylimidazole (67 mg, 0.82 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (10 mL), collected, dried over anhydrous sodium sulphate, filtered and concentrated to obtain a crude, which was then purified by silica gel column chromatography to obtain a mixture of compound 14-1a and compound 14-1b (90 mg, yield: 57.81%).
LCMS m/z = 577.2 [M+H]⁺

### Step 2: Preparation of compound 14-2a and compound 14-2b

Hydrogen chloride in dioxane (4 M, 2 mL) was added to the mixture of compound 14-1a and compound 14-1b (40 mg, 0.07 mmol). After the addition, the mixture was reacted at room temperature under nitrogen atmosphere for 18 h. The reaction mixture was concentrated to obtain a crude, which was then purified by prep-HPLC (condition 1) to obtain a mixture of compound 14-2a and compound 14-2b (19.32 mg, yield: 52.19 %).
LCMS m/z = 537.2 [M+H]⁺

### Example 15: Preparation of compound 15-2a and compound 15-2b

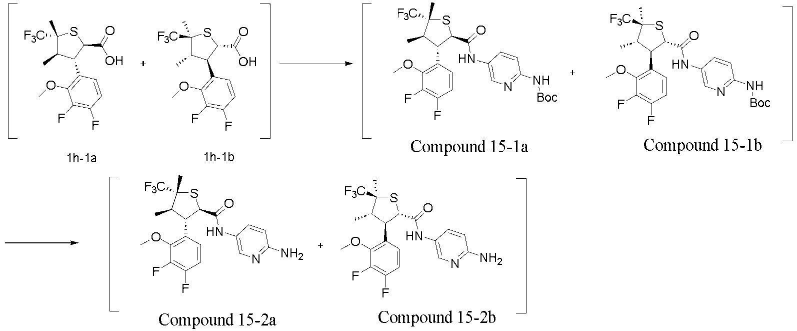

### Step 1: Preparation of compound 15-1a and compound 15-1b

The mixture of 1h-1a and 1h-1b (100 mg, 0.27 mmol) was dissolved in EA (3 mL), and tert-butyl 5-aminopyridine-2-carbamate (68 mg, 0.32 mmol), T3P (50% wt in EtOAc 680 mg; containing 1-propylphosphoric anhydride 340 mg, 1.08 mmol) and Et₃N (82 mg, 0.81 mmol) were sequentially added. After the addition, the mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 15-1a and compound 15-1b (140 mg, yield: 92.34%).
LCMS m/z = 562.2 [M+H]⁺

### Step 2: Preparation of compound 15-2a and compound 15-2b

Hydrogen chloride in dioxane (4 M, 2 mL) was added to the mixture of compound 15-1a and compound 15-1b (100 mg, 0.18 mmol). After the addition, the mixture was reacted at room temperature under nitrogen atmosphere for 18 h. The reaction mixture was concentrated to obtain a crude, which was then purified by prep-HPLC (condition 1) to obtain a mixture of compound 15-2a and compound 15-2b (81.11 mg, yield: 97.65 %).
LCMS m/z =462.2 [M+H]⁺

### Example 16: Preparation of compound 16-1a and compound 16-1b

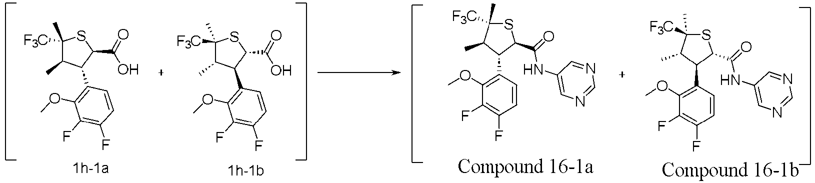

The mixture of 1h-1a and 1h-1b (100 mg, 0.27 mmol) was dissolved in EA (3 mL), and 5-aminopyrimidine (31 mg, 0.32 mmol), T3P (50% wt in EtOAc 680 mg; containing 1-propylphosphoric anhydride 340 mg, 1.08 mmol) and Et₃N (82 mg, 0.81 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 16-1a and compound 16-1b (28.78 mg, yield: 23.82%).
LCMS m/z = 448.4 [M+H]⁺

### Example 17: Preparation of compound 17-1a and compound 17-1b

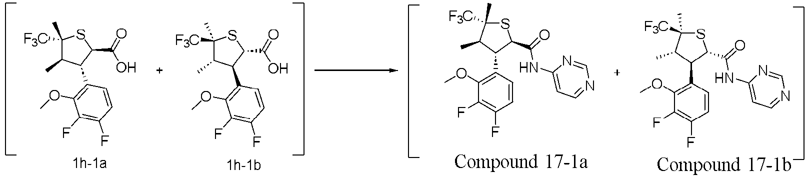

With reference to the synthesis method of Example 16, by using 5-aminopyrimidine as the raw material, a mixture of compound 17-1a and compound 17-1b (63.24 mg, yield: 52.34%) was obtained.
LCMS m/z = 448.4 [M+H]⁺

### Example 18: Preparation of compound 18-2a and compound 18-2b

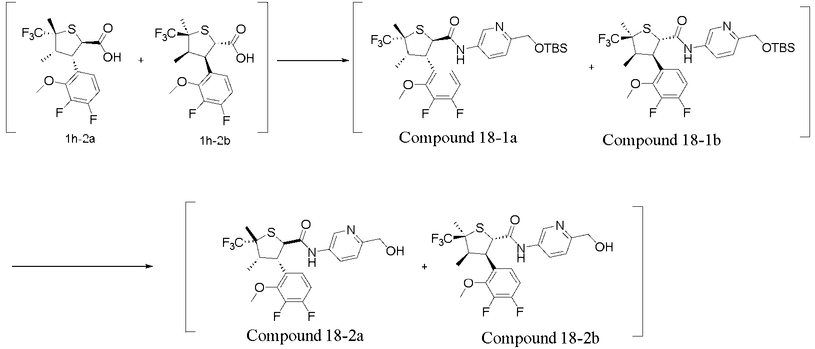

### Step 1: Preparation of compound 18-1a and compound 18-1b

With reference to the synthesis method of Example 16, a mixture of compound 18-1a and compound 18-1b (40 mg, yield: 48.37%) was obtained.
LCMS m/z = 591.9 [M+H]⁺

### Step 2: Preparation of compound 18-2a and compound 18-2b

Methanol (1 mL) and hydrochloric acid (0.3 mL) were added to the mixture of compound 18-1a and compound 18-1b (40 mg, 0.068 mmol). After the addition, the mixture was reacted at room temperature under nitrogen atmosphere for 18 h. The reaction mixture was concentrated to obtain a crude, which was then purified by prep-HPLC (condition 1) to obtain a mixture of compound 18-2a and compound 18-2b (16 mg, yield: 49.38 %).
LCMS m/z =477.1 [M+H]⁺

### Example 19: Preparation of compound 19-2a and compound 19-2b

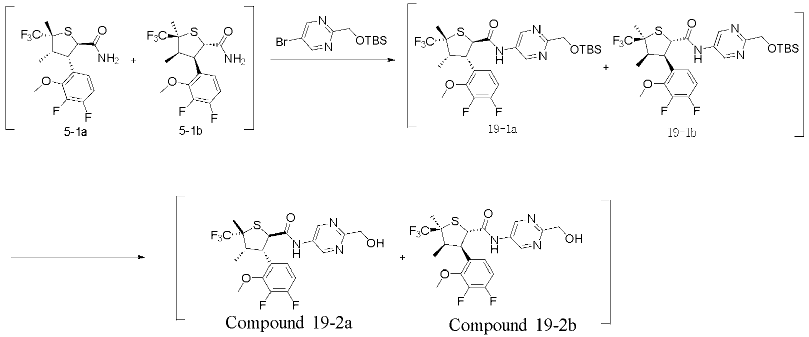

### Step 1: Preparation of compound 19-1a and compound 19-1b

With reference to the synthesis method of Example 5, a mixture of compound 19-1a and compound 19-1b (34 mg, yield: 52.24%) was obtained.
LCMS m/z = 592.2 [M+H]⁺

### Step 2: Preparation of compound 19-2a and compound 19-2b

Methanol (1 mL) and hydrochloric acid (0.3 mL) were added to the mixture of compound 19-1a and compound 19-1b (34 mg, 0.057 mmol). After the addition, the mixture was reacted at room temperature under nitrogen atmosphere for 18 h. The reaction mixture was concentrated to obtain a crude, which was then purified by prep-HPLC (condition 1) to obtain a mixture of compound 19-2a and compound 19-2b (20 mg, yield: 73.49 %).
LCMS m/z =478.50 [M+H]⁺

### Example 20: Preparation of compound 20-1a and compound 20-1b

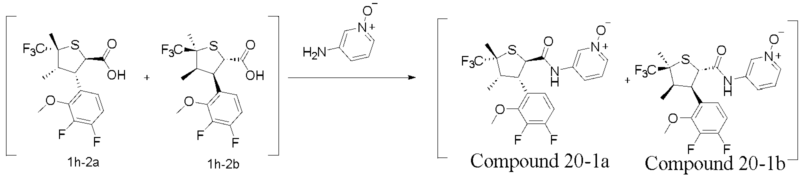

With reference to the synthesis method of Example 16, a mixture of compound 20-1a and compound 20-1b (3 mg, yield: 4.63%) was obtained.
LCMS m/z = 463.1 [M+H]⁺

### Example 21: Preparation of compound 21-1a and compound 21-1b

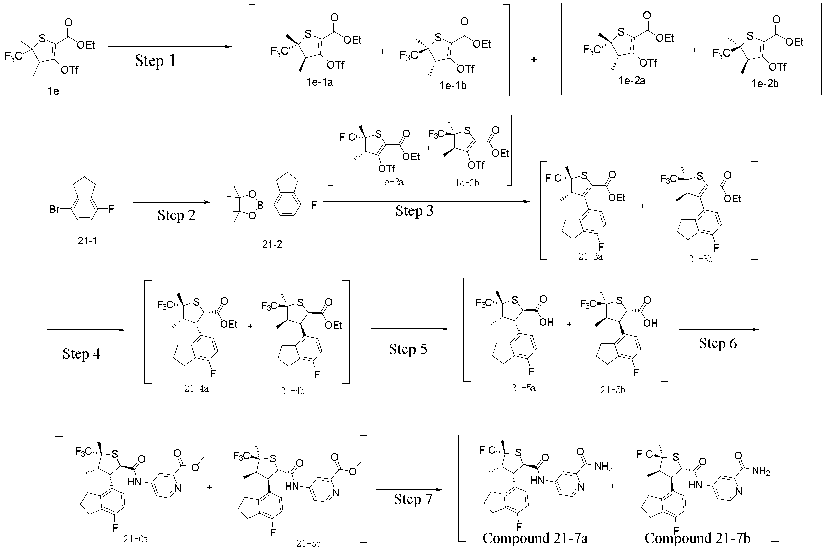

### Step 1: Preparation of compounds 1e-2a and 1e-2b

1e (51.0 g, 126.86 mmol) was purified by column chromatography to obtain a mixture of 1e-2a and 1e-2b (17.6 g, yield: 34%).

### Step 2: Preparation of compound 21-2

21-1 (350 mg, 1.63 mmol) (prepared with reference to WO 2021093820 A1), (Bpin)₂ (496 mg, 1.96 mmol), potassium acetate (480 mg, 4.89 mmol) and Pd(dppf)Cl₂•DCM (119 mg, 0.16 mmol) were added to 1,4-dioxane (10 mL), and the mixture was reacted at 100°C under nitrogen atmosphere for 5 h. The reaction solution was used directly in the next step.

### Step 3: Preparation of compounds 21-3a and 21-3b

1e-2a and 1e-2b (655 mg, 1.63 mmol), Pd(PPh₃)₄ (94 mg, 0.08 mmol), potassium phosphate (1.04 g, 4.90 mmol) and water (0.5 mL) were added to the reaction solution from the previous step, and the mixture was reacted at 100°C under nitrogen atmosphere for 16 h. The reaction solution was cooled to room temperature and then concentrated under reduced pressure. The residue was separated and purified by silica gel column chromatography to obtain a mixture of 21-3a and 21-3b (420 mg, yield: 66.34%).
LCMS m/z =389.2 [M+H]⁺

### Step 4: Preparation of 21-4a and 21-4b

The mixture of 21-3a and 21-3b (220 mg, 0.57 mmol) was dissolved in methanol (8 mL), and platinum oxide (52 mg) was added. The mixture was reacted at room temperature under hydrogen atmosphere for 18 h. The mixture was filtered through celite, and the filtrate was concentrated. The residue was separated and purified by silica gel column chromatography to obtain a mixture of 21-4a and 21-4b (88 mg, yield: 39.54%).

### Step 5: Preparation of 21-5a and 21-5b

The mixture of 21-4a and 21-4b (50 mg, 0.13 mmol) was dissolved in anhydrous ethanol (3 mL), and caesium carbonate (64 mg, 0.2 mmol) was added. The mixture was reacted at 50°C for 3 h. The reaction solution was cooled to room temperature, and added to 1 M hydrochloric acid (5 mL) to quench the reaction. The reaction mixture was extracted with ethyl acetate (50 mL×3). The organic phases were combined, dried over anhydrous sodium sulphate and then filtered. The filtrate was concentrated, and the residue was separated and purified by silica gel column chromatography to obtain a mixture of 21-5a and 21-5b (40 mg, yield: 84.91%).
LCMS m/z =361.1 [M-H]⁻

### Step 6: Preparation of 21-6a and 21-6b

The mixture of 21-5a and 21-5b (40 mg, 0.11 mmol) was dissolved in ethyl acetate (3 mL), and triethylamine (33 mg, 0.33 mmol), T3P (50% wt in EtOAc 280 mg; containing 1-propylphosphoric anhydride 140 mg, 0.44 mmol) and methyl 4-aminopyridine-2-carboxylate (40 mg, 0.11 mmol) were sequentially added. The mixture was reacted at room temperature under nitrogen atmosphere for 18 h. The reaction was quenched by adding an aqueous sodium bicarbonate solution (10 mL), and the mixture was extracted with ethyl acetate (10 mL×3). The organic phases were combined, dried over anhydrous sodium sulphate, filtered and concentrated to obtain a crude, which was then separated and purified by silica gel column chromatography to obtain a mixture of 21-6a and 21-6b (8 mg, yield: 14.65%).
LCMS m/z =497.6 [M+H]⁺

### Step 7: Preparation of compound 21-7a and compound 21-7b

The mixture of 21-6a and 21-6b (8 mg, 0.039 mmol) was dissolved in a 7 M ammonia methanol solution (1 mL), and the resulting mixture was reacted at room temperature for 18 h. The reaction system was concentrated to obtain a crude, which was then subjected to silica gel column chromatography to obtain a mixture of compound 21-7a and compound 21-7b (1 mg, yield: 13.01%).
LCMS m/z =482.2 [M+H]⁺

The mixture of compound 21-7a and compound 21-7b (110 mg) was used as raw materials, and subjected to chiral resolution to obtain **compound 21-7P1**(38.3 mg, retention time: 1.576 min) and **compound 21-7P2** (38 mg, retention time: 1.723 min).

Compound 21-7P1 is either 21-7a or 21-7b shown above, and is an enantiomer of compound 21-7P2, that is, when compound 21-7P1 has a structure as shown in formula 21-7a, compound 21-7P2 has a structure as shown in formula 21-7b; and when compound 21-7P1 has a structure as shown in formula 21-7b, compound 21-7P2 has a structure as shown in formula 21-7a.

### SFC preparative conditions:

instrument: Waters 150 Prep-SFC C; preparative column: Chiral IC column; preparation method: the crude was dissolved in acetonitrile to prepare a 10 mg/ml sample solution; mobile phase system: carbon dioxide/0.1% NH3•H2O in methanol, with methanol content of 15%; elution time: 15 min;
chiral testing method: (instrument: SHIMADZU LC-30 AD sf; preparative column: Chiral IC column; preparation method: the crude was dissolved in acetonitrile to prepare a sample solution; mobile phase system: carbon dioxide/0.05% DEA in methanol; elution gradient: 5%-40%; flow rate: 3.0 mL/min; elution time: 3 min).

### Example 22: Preparation of compound 22-1a and compound 22-1b

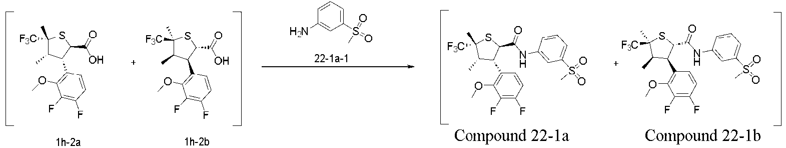

The mixture of 1h-2a and 1h-2b (50 mg, 0.135 mmol) was dissolved in ethyl acetate (3 mL), and triethylamine (42 mg, 0.42 mmol) and T3P (50% wt in EtOAc 350 mg; containing 1-propylphosphoric anhydride 175 mg, 0.55 mmol) were sequentially added. 22-1a-1 (34 mg, 0.20 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then separated and purified by silica gel column chromatography to obtain a mixture of 22-1a and 22-1b (42 mg, yield: 59%).
LCMS m/z =524.0 [M+H]⁺;
¹H NMR (400 MHz, CDCl₃) δ 8.44 (s, 1H), 8.02 (d, 1H), 7.95 (t, 1H), 7.67 (d, 1H), 7.52 (t, 1H), 7.04 - 6.95 (m, 1H), 6.89 - 6.80 (m, 1H), 4.63 (d, 1H), 4.56 - 4.45 (m, 1H), 4.05 (t, 3H), 3.10 (s, 3H), 2.72 - 2.56 (m, 1H), 1.93 (s, 3H) , 0.94 - 0.80 (m, 3H).

### Example 23: Preparation of compounds 23-8a and 23-8b

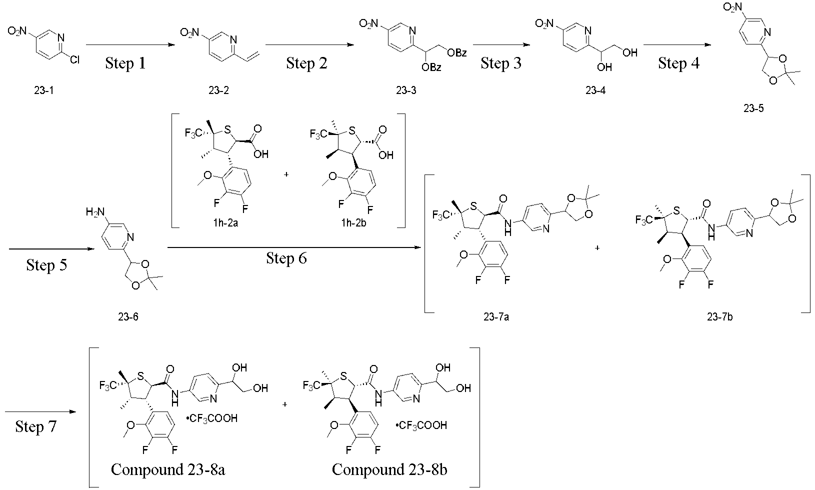

### Step 1: Preparation of compound 23-2

23-1 (7.9 g, 50.0 mmol), vinylboronic acid pinacol ester (8.47 g, 55.0 mmol), potassium carbonate (13.8 g, 100.0 mmol) and Pd(dppf)Cl₂•DCM (0.51 g, 0.75 mmol) were added to a mixed solvent of 1,4-dioxane (120 mL) and water (30 mL), and the mixture was reacted at 100°C under nitrogen atmosphere for 4 h. The reaction solution was cooled to room temperature, and then ethyl acetate (200 mL) was added. The mixture was washed with a saturated sodium chloride aqueous solution (50 mL×3). The organic layers were combined, dried over anhydrous sodium sulphate and filtered. The filtrate was concentrated under reduced pressure, and the residue was purified by column chromatography to obtain 23-2 (4.6 g, yield: 61%).
LCMS m/z =151.0 [M+H]⁺

### Step 2: Preparation of compound 23-3

23-2 (4.6 g, 30.66 mmol), silver benzoate (21.0 g, 91.98 mmol), and iodine (12.4 g, 49.05 mmol) were added to toluene (150 mL), and the mixture was refluxed under nitrogen atmosphere for 20 h. The reaction solution was cooled to room temperature and filtered through celite. The filtrate was concentrated under reduced pressure, and the residue was purified by column chromatography to obtain 23-3 (5.88 g, yield: 49%).
LCMS m/z = 393.1 [M+H]⁺

### Step 3: Preparation of compound 23-4

23-3 (5.88 g, 15.0 mmol) and lithium hydroxide monohydrate (2.52 g, 60.0 mmol) were added to a mixed solvent of tetrahydrofuran (80 mL) and water (20 mL), and the mixture was reacted at room temperature for 3 h. The reaction solution was extracted with ethyl acetate (300 mL). The organic phase was dried over anhydrous sodium sulphate and then filtered. The filtrate was concentrated, and the residue was separated and purified by silica gel column chromatography to obtain 23-4 (117 mg, yield: 4%).
LCMS m/z = 185.1 [M+H] ⁺

### Step 4: Preparation of compound 23-5

23-4 (117 mg, 0.635 mmol), 2,2-dimethoxypropane (198 mg, 1.9 mmol) and p-toluenesulfonic acid monohydrate (18 mg, 0.095 mmol) were added to tetrahydrofuran (5 mL), and the mixture was reacted at room temperature for 4 h. The reaction solution was concentrated under reduced pressure, and the residue was separated and purified by silica gel column chromatography to obtain 23-5 (110 mg, yield: 76%).

### Step 5: Preparation of compound 23-6

23-5 (110 mg, 0.486 mmol) and palladium on carbon (10%) (22 mg) were added to ethyl acetate (3 mL) and ethanol (3 mL), and the mixture was reacted at room temperature under hydrogen atmosphere for 16 h. The reaction solution was filtered through celite. The filtrate was concentrated under reduced pressure, and the residue was separated and purified by silica gel column chromatography to obtain 23-6 (89 mg, yield: 94%).
LCMS m/z = 195.1 [M+H] ⁺

### Step 6: Preparation of 23-7a and 23-7b

The mixture of 1h-2a and 1h-2b (50 mg, 0.135 mmol) was dissolved in ethyl acetate (3 mL), and triethylamine (42 mg, 0.42 mmol), T3P (50% wt in EtOAc 350 mg; containing 1-propylphosphoric anhydride 175 mg, 0.55 mmol) and 23-6 (40 mg, 0.20 mmol) were sequentially added. The mixture was reacted at room temperature under nitrogen atmosphere for 18 h. The reaction was quenched by adding an aqueous sodium bicarbonate solution (10 mL), and the mixture was extracted with ethyl acetate (10 mL×3). The organic phases were combined, dried over anhydrous sodium sulphate, filtered and concentrated to obtain a crude, which was then separated and purified by silica gel column chromatography to obtain a mixture of 23-7a and 23-7b (68 mg, yield: 92%).
LCMS m/z =547.1 [M+H]⁺

### Step 7: Preparation of compound 23-8a and compound 23-8b

The mixture of 23-7a and 23-7b (68 mg, 0.124 mmol) was dissolved in dichloromethane (5 mL), and trifluoroacetic acid (1 mL) was added. The mixture was reacted at room temperature for 1 h. The reaction solution was concentrated under reduced pressure and then subjected to prep-HPLC (condition 1) to obtain the trifluoroacetate of a mixture of compounds 23-8a and 23-8b (40 mg, yield: 52%).
LCMS m/z =507.1 [M+H]⁺ ;

### Example 24: Preparation of compounds 24-9a and 24-9b

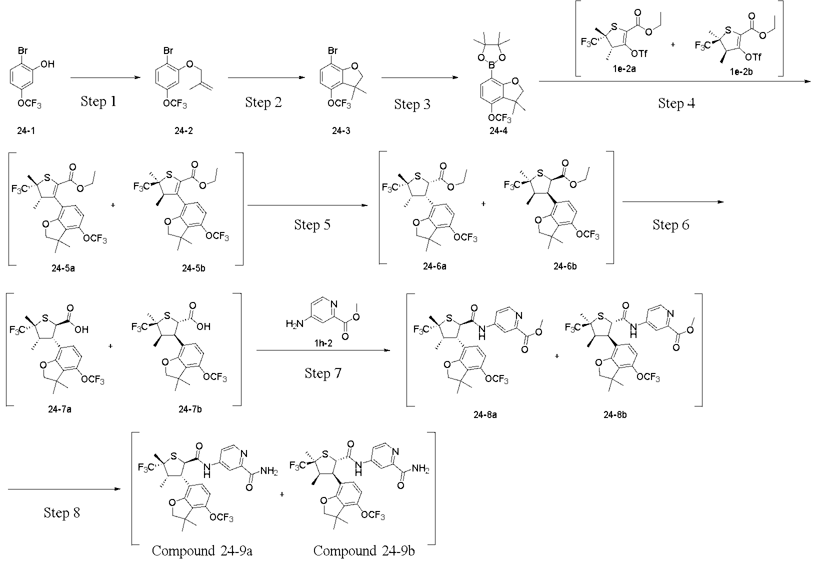

### Step 1: Preparation of compound 24-2

24-1 (4.0 g, 15.6 mmol), 3-bromo-2-methylpropene (2.53 g, 18.74 mmol) and potassium carbonate (4.3 g, 31.2 mmol) were added to acetonitrile (60 mL), and the mixture was reacted at 60°C under nitrogen atmosphere for 12 h. The reaction solution was cooled to room temperature and filtered through celite. The filtrate was concentrated under reduced pressure, and the residue was purified by column chromatography to obtain 24-2 (4.8 g, yield: 99%).

### Step 2: Preparation of compound 24-3

24-2 (4.8 g, 15.4 mmol) was added to dichloromethane (150 mL), and placed in an ice bath, and aluminium chloride (107 mg, 0.8 mmol) was added. The mixture was further reacted in the ice bath for 1 h. The reaction was quenched by adding the reaction solution to an ice-cold aqueous sodium bicarbonate solution, and the mixture was extracted with ethyl acetate (200 mL). The organic phase was dried over anhydrous sodium sulphate and then filtered. The filtrate was concentrated, and the residue was separated and purified by silica gel column chromatography to obtain 24-3 (4.0 g, yield: 83%).

### Step 3: Preparation of compound 24-4

24-3 (2.02 g, 6.51 mmol), (Bpin)₂(CAS:73183-34-3) (2.15 g, 8.46 mmol), potassium acetate (1.28 g, 13.0 mmol) and Pd(dppf)Cl₂•DCM (265 mg, 0.32 mmol) were added to 1,4-dioxane (40 mL), and the mixture was reacted at 100°C under nitrogen atmosphere for 5 h. The reaction solution was cooled to room temperature and concentrated under reduced pressure, and the residue was separated and purified by silica gel column chromatography to obtain 24-4 (660 mg, yield: 30%).

### Step 4: Preparation of compounds 24-5a and 24-5b

24-4 (286 mg, 0.8 mmol), 1e-2a and 1e-2b (300 mg, 0.746 mmol), Pd(PPh₃)₄(43 mg, 0.04 mmol) and potassium phosphate (474 mg, 2.4 mmol) were added to toluene (9 mL) and water (3 mL), and the mixture was reacted at 100°C under nitrogen atmosphere for 16 h. The reaction solution was cooled to room temperature and then concentrated under reduced pressure. The residue was separated and purified by silica gel column chromatography to obtain a mixture of 24-5a and 24-5b (300 mg, yield: 77%).

### Step 5: Preparation of 24-6a and 24-6b

The mixture of 24-5a and 24-5b (300 mg, 0.62 mmol) was dissolved in methanol (30 mL), and palladium on carbon (10%) (100 mg) was added. The mixture was reacted at 90°C under hydrogen atmosphere at a pressure of 2.5 MPa for 70 h. The reaction solution was cooled to room temperature and filtered through celite, and the filtrate was concentrated. The residue was separated and purified by silica gel column chromatography to obtain a mixture of 24-6a and 24-6b (180 mg, yield: 60%).

### Step 6: Preparation of 24-7a and 24-7b

The mixture of 24-6a and 24-6b (130 mg, 0.267 mmol) was dissolved in anhydrous ethanol (5 mL), and caesium carbonate (160 mg, 0.4 mmol) was added. The mixture was reacted at 50°C for 3 h. The reaction solution was cooled to room temperature, and added to 1 M hydrochloric acid (5 mL) to quench the reaction. The reaction mixture was extracted with ethyl acetate (50 mL×3). The organic phases were combined, dried over anhydrous sodium sulphate and then filtered. The filtrate was concentrated, and the residue was separated and purified by silica gel column chromatography to obtain a mixture of 24-7a and 24-7b (120 mg, yield: 98%).
LCMS m/z =457.0 [M-H]⁻

### Step 7: Preparation of 24-8a and 24-8b

The mixture of 24-7a and 24-7b (46 mg, 0.1 mmol) was dissolved in ethyl acetate (2 mL), and triethylamine (42 mg, 0.42 mmol), T3P (50% wt in EtOAc 350 mg; containing 1-propylphosphoric anhydride 175 mg, 0.55 mmol) and 1h-2 (40 mg, 0.20 mmol) were sequentially added. The mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then separated and purified by silica gel column chromatography to obtain a mixture of 24-8a and 24-8b (23 mg, yield: 39%).
LCMS m/z =593.1 [M+H]⁺

### Step 8: Preparation of compound 24-9a and compound 24-9b

The mixture of 24-8a and 24-8b (23 mg, 0.039 mmol) was dissolved in a 7 M ammonia methanol solution (3 mL), and the resulting mixture was reacted at room temperature for 18 h. The reaction system was concentrated to obtain a crude, which was then subjected to silica gel column chromatography to obtain a mixture of compound 24-9a and compound 24-9b (14 mg, yield: 62%).
LCMS m/z =578.1 [M+H]⁺
¹H NMR (400 MHz, CDCl₃) δ 9.82(s, 1H), 8.46 (d, 1H), 8.39 - 8.27 (m, 1H), 8.16 (d, 1H), 8.00 (d, 1H), 7.06 (d, 1H), 6.68 (d, 1H), 5.88 (d, 1H), 5.09 (d, 1H), 4.43 - 4.29 (m, 1H), 3.02 (s, 2H), 2.83 - 2.70 (m, 1H), 1.90 (s, 3H), 1.48 (s, 3H), 1.42 (s, 3H), 1.00 - 0.90 (m, 3H).

### Example 25: Preparation of compound 25-1a and compound 25-1b

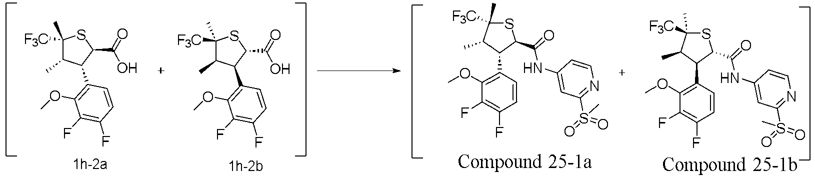

The mixture of 1h-1a and 1h-1b (50 mg, 0.14 mmol) was dissolved in EA (1.5 mL), and 2-(methylsulfonyl)pyridin-4-amine (45 mg, 0.26 mmol), T3P (50% wt in EtOAc 340 mg; containing 1-propylphosphoric anhydride 170 mg, 0.54 mmol) and Et₃N (82 mg, 0.81 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 25-1a and compound 25-1b (7.23 mg, yield: 18.62%).
LCMS m/z = 525.60 [M+H]⁺

### Example 26: Preparation of compound 26-1a and compound 26-1b

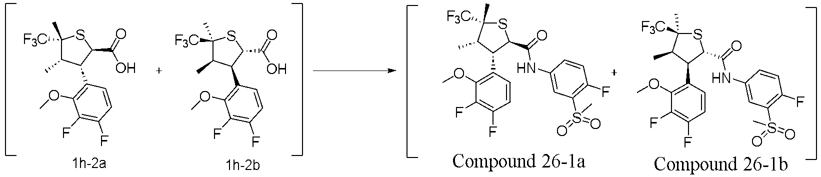

The mixture of 1h-1a and 1h-1b (50 mg, 0.13 mmol) was dissolved in ethyl acetate (1.5 mL), and 4-fluoro-3-(methylsulfonyl)aniline (51 mg, 0.26 mmol), T3P (50% wt in EtOAc 340 mg; containing 1-propylphosphoric anhydride 170 mg, 0.54 mmol) and Et₃N (82 mg, 0.81 mmol) were sequentially added. The mixture was reacted at room temperature under nitrogen atmosphere for 18 h. The reaction was quenched by adding water (5 mL) to the reaction system. The mixture was extracted with ethyl acetate (5 mL×3). The organic phases were combined, washed with a saturated sodium chloride aqueous solution (5 mL), collected, dried over anhydrous sodium sulphate, filtered and concentrated to obtain a crude, which was then purified by prep-HPLC (condition 1) to obtain a mixture of compound 26-1a and compound 26-1b (37.21 mg, yield: 33.95%).
LCMS m/z = 542.60 [M+H]⁺
¹H NMR (400 MHz, CDCl₃) δ 8.84 (s, 1H), 8.42 - 8.30 (m, 1H), 7.82 - 7.73 (m, 1H), 7.21 (t, 1H), 6.99 - 6.90 (m, 1H), 6.87 - 6.76 (m, 1H), 4.70 (d, 1H), 4.61 - 4.52 (m, 1H), 4.05 (d, 3H), 3.34 (s, 3H), 2.67 - 2.58 (m, 1H), 1.92 (s, 3H), 0.95 - 0.85 (m, 3H).

### Example 27: Preparation of compound 27-1a and compound 27-1b

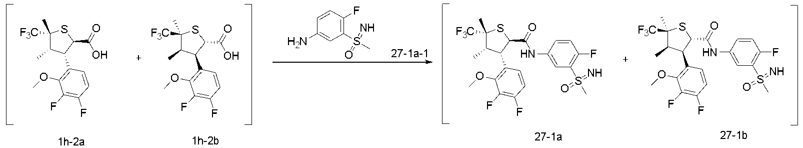

The mixture of 1h-2a and 1h-2b (0.052 g, 0.14 mmol) was dissolved in ethyl acetate (3 mL), and triethylamine (0.085 g, 0.84 mmol) and T3P (50% wt in EtOAc 0.36 g; containing 1-propylphosphoric anhydride 0.18 g, 0.56 mmol) were sequentially added. 27-1a-1 (0.040 g, 0.21 mmol) was added to the system, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then purified by prep-HPLC (condition 1) to obtain the trifluoroacetate of a mixture of 27-1a and 27-1b.
LCMS m/z = 541.1 [M+H]⁺
¹H NMR (400 MHz, DMSO-*d₆*) δ 10.72 (s, 1H), 8.17 - 8.09 (m, 1H), 7.81 - 7.72 (m, 1H), 7.37 (t, 1H), 7.22 - 7.14 (m, 1H), 7.11 - 7.04 (m, 1H), 4.83 (d, 1H), 4.53 - 4.45 (m, 1H), 4.00 (d, 3H), 3.19 (s, 3H), 2.65 - 2.57 (m, 1H), 1.85 (s, 3H), 0.83 - 0.74 (m, 3H).

### Example 28: Preparation of compound 28-2a and compound 28-2b

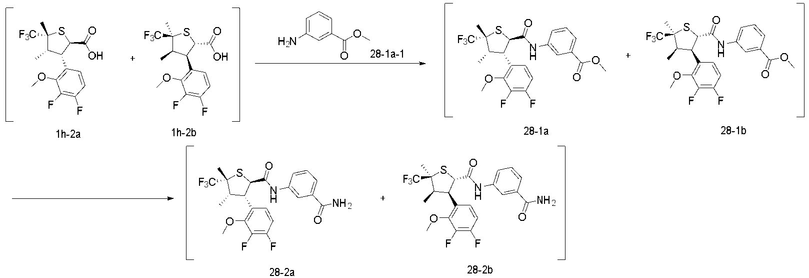

### Step 1: Preparation of compounds 28-1a and 28-1b

The mixture of 1h-2a and 1h-2b (0.052 g, 0.14 mmol) was dissolved in ethyl acetate (3 mL), and triethylamine (0.085 g, 0.84 mmol) and T3P (50% wt in EtOAc 0.36 g; containing 1-propylphosphoric anhydride 0.18 g, 0.56 mmol) were sequentially added. 28-1a-1 (0.032 g, 0.21 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then purified by silica gel column chromatography to obtain a mixture of 28-1a and 28-1b (0.06 g, yield: 85.12%).
LCMS m/z = 504.1 [M+H]⁺

### Step 2: Preparation of compounds 28-2a and 28-2b

The mixture of 28-1a and 28-1b (0.06 g, 0.12 mmol) was dissolved in a 7 M ammonia methanol solution (5 mL), and the resulting mixture was heated to 40°C in a sealed tube and reacted for 18 h. The reaction system was concentrated to obtain a crude, which was then purified by silica gel column chromatography to obtain a mixture of 28-2a and 28-2b (0.02 g, yield: 34.12%).
LCMS m/z = 489.3 [M+H]⁺;
¹H NMR (400 MHz, DMSO-*d₆*) δ 10.46 (s, 1H), 7.99 (t, 1H), 7.90 (s, 1H), 7.66 - 7.61 (m, 1H), 7.56 - 7.51 (m, 1H), 7.39 - 7.27 (m, 2H), 7.23 - 7.06 (m, 2H), 4.87 (d, 1H), 4.53 - 4.45 (m, 1H), 4.00 (d, 3H), 2.65 - 2.56 (m, 1H), 1.86 (s, 3H), 0.82 - 0.75 (m, 3H).

### Example 29: Preparation of compound 29-1a and compound 29-1b

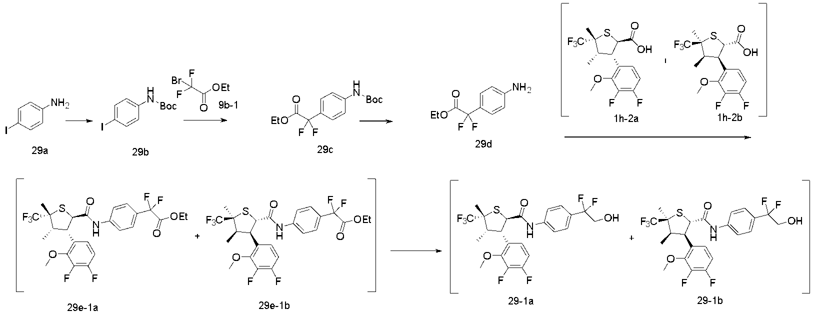

### Step 1: Preparation of compound 29b

29a (3.28 g, 15.0 mmol) was dissolved in methanol (50 mL), and triethylamine (1.52 g, 14.99 mmol) and (Boc)₂O (6.48 g, 29.98 mmol) were sequentially added. The mixture was reacted at 50°C for 18 h. The reaction system was concentrated to obtain a crude, which was then purified by silica gel column chromatography to obtain 29b (4.47 g, yield: 93.38%).
LCMS m/z = 264.0 [M-55]⁺

### Step 2: Preparation of compound 29c

29b (4.46 g, 13.96 mmol) was dissolved in DMSO (50 mL), and 29b-1 (7.08 g, 34.90 mmol) and copper powder (2.22 g, 34.90 mmol) were sequentially added. The mixture was reacted at 80°C under nitrogen atmosphere for 18 h. The reaction was cooled to room temperature, and the reaction system was filtered. The filter cake was washed with ethyl acetate (10 mL×3). The filtrate was quenched by adding water (500 mL) and extracted with ethyl acetate (50 mL×3). The organic phases were combined, washed with a saturated sodium chloride solution (200 mL), collected, dried over anhydrous sodium sulphate, filtered and concentrated to obtain a crude, which was then purified by silica gel column chromatography to obtain 29c (3.3 g, yield: 74.97%).

### Step 3: Preparation of compound 29d

29c (0.50 g, 1.6 mmol) was dissolved in ethyl acetate (10 mL), and p-toluenesulfonic acid (0.69 g, 4.0 mmol) was added. The mixture was reacted at 45°C under nitrogen atmosphere for 18 h. The reaction system was concentrated to obtain a crude, and the crude was dissolved in water (10 mL). In an ice bath, the mixture was adjusted to pH = 9-10 by slowly dropwise adding a saturated sodium carbonate solution, and extracted with dichloromethane : methanol = 10 : 1 (15 mL × 4). The organic phases were combined, dried over anhydrous sodium sulphate, filtered and concentrated to obtain 29d (0.32 g, yield: 92.94%).
LCMS m/z = 216.1 [M+1]⁺

### Step 4: Preparation of compounds 29e-1a and 29e-1b

The mixture of 1h-2a and 1h-2b (0.052 g, 0.14 mmol) was dissolved in ethyl acetate (3 mL), and triethylamine (0.085 g, 0.84 mmol) and T3P (50% wt in EtOAc 0.36 g; containing 1-propylphosphoric anhydride 0.18 g, 0.56 mmol) were sequentially added. 29d (0.045 g, 0.21 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then purified by silica gel column chromatography to obtain a mixture of 29e-1a and 29e-1b (0.048 g, yield: 60.41%).

### Step 5: Preparation of compounds 29-1a and 29-1b

The mixture of 29e-1a and 29e-1b (0.048 g, 0.084 mmol) was dissolved in methanol (3 mL), and precooled in an ice bath for 10 minutes. Sodium borohydride (0.0032 g, 0.084 mmol) was added, and the mixture was reacted in an ice bath for 2 h. In an ice bath, the reaction was quenched by adding water (10 mL) to the reaction system, and the mixture was extracted with ethyl acetate (10 mL×3). The organic phases were combined, dried over anhydrous sulphuric acid, filtered and concentrated to obtain a crude, which was then purified by silica gel column chromatography to obtain a mixture of 29-1a and 29-1b (0.02 g, yield: 45.31%).
LCMS m/z = 524.1 [M+H]⁻;
¹H NMR (400 MHz, DMSO-*d₆*) δ 10.52 (s, 1H), 7.60 (d, 2H), 7.43 (d, 2H), 7.22 - 7.13 (m, 1H), 7.11 - 7.05 (m, 1H), 5.52 (t, 1H), 4.87 (d, 1H), 4.52 - 4.44 (m, 1H), 3.99 (d, 3H), 3.84 - 3.73 (m, 2H), 2.65 - 2.57 (m, 1H), 1.85 (s, 3H), 0.82 - 0.74 (m, 3H).

### Example 30: Preparation of compound 30

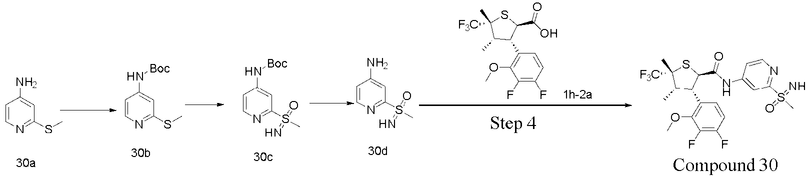

### Step 1: Preparation of compound 30b

In an ice bath, 30a (0.88 g, 6.28 mmol) was dissolved in tetrahydrofuran (15 mL), and LiHMDS (2.53 g, 13.82 mmol) was added dropwise. Di-tert-butyl dicarbonate (1.51 g, 6.91 mmol) was dissolved in tetrahydrofuran (5 mL) and slowly added dropwise to the system. Under nitrogen atmosphere, the mixture was naturally heated to room temperature and reacted for 18 h. In an ice bath, the reaction was quenched by adding a saturated aqueous ammonium chloride solution (30 mL) to the reaction system, and the mixture was extracted with ethyl acetate (20 mL×3). The organic phases were combined, dried over anhydrous sodium sulphate, filtered and concentrated to obtain a crude, which was then purified by silica gel column chromatography to obtain 30b (1.4 g, yield: 92.76%).

### Step 2: Preparation of compound 30c

30b (1.41 g, 5.87 mmol) was dissolved in methanol (10 mL), and ammonium carbamate (1.83 g, 23.48 mmol) and iodobenzene acetate (5.67 g, 17.61 mmol) were sequentially added. The mixture was reacted at room temperature for 18 h. The reaction mixture was concentrated under reduced pressure to remove the reaction solution, and the crude was purified by silica gel column chromatography to obtain 30c (1.14 g, yield: 71.57%).

### Step 3: Preparation of compound 30d

30c (0.054 g, 0.2 mmol) was dissolved in dichloromethane (2.5 mL), and trifluoroacetic acid (0.5 mL) was added. The mixture was reacted at room temperature for 2 h. The solvent was removed by concentration under reduced pressure to obtain 30d as a crude.
LCMS m/z = 172.1 [M+1]⁺

### Step 4: Preparation of compound 30

1h-2a (0.037 g, 0.1 mmol) was dissolved in ethyl acetate (3 mL), and triethylamine (0.061 g, 0.60 mmol) and T3P (50% wt in EtOAc 0.25 g; containing 1-propylphosphoric anhydride 0.125 g, 0.40 mmol) were sequentially added. 30d (0.026 g, 0.15 mmol) was added, and the mixture was reacted at room temperature under nitrogen atmosphere for 18 h. An aqueous sodium bicarbonate solution (10 mL) was added to the reaction system, and the mixture was extracted with ethyl acetate (15 mL×3). The organic phases were combined, dried over anhydrous sodium sulphate, filtered and concentrated to obtain a crude, which was then purified by prep-HPLC (condition 1) and then lyophilized to obtain compound 30 (0.003 g, yield: 5.73%).
LCMS m/z = 524.1 [M+H]⁺;

### Example 31: Preparation of compound 31-1a and compound 31-1b

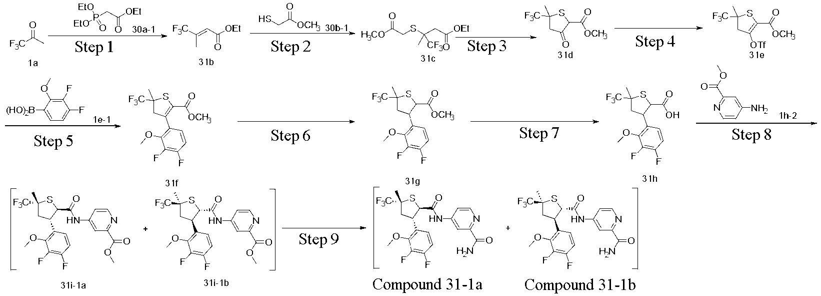

### Step 1: Preparation of compound 31b

In an ice bath, 31a-1 (29.14 g, 130 mmol) was dissolved in tetrahydrofuran (100 mL), sodium hydride (5.2 g, 130 mmol) was added in batches, and the mixture was reacted under nitrogen atmosphere and in an ice bath for 30 minutes. A solution of 1a (11.20 g, 100 mmol) in tetrahydrofuran (20 mL) was added dropwise. Under nitrogen atmosphere, the mixture was naturally heated to room temperature and reacted for 18 h. In an ice bath, the reaction system was adjusted to pH = 7-8 by slowly dropwise adding 1 N hydrochloric acid, and extracted with diethyl ether (100 mL × 3). The organic phases were combined, dried over anhydrous sodium sulphate, filtered and concentrated (at room temperature) to obtain a crude, which was then purified by silica gel column chromatography to obtain 31b (9.5 g, yield: 52.16%).

### Step 2: Preparation of compound 31c

In an ice bath, 31b (4.8 g, 26.37 mmol) was added to a round-bottom flask, piperidine (0.45 g, 5.27 mmol) was added dropwise to the system, and then 31b-1 (2.80 g, 26.37 mmol) was added dropwise. Under nitrogen protection, the mixture was reacted at 30°C for 48 h. In an ice bath, the reaction was quenched by adding 0.1 N hydrochloric acid (50 mL) to the reaction system, and the mixture was extracted with diethyl ether (50 mL×3). The organic phases were combined, dried over anhydrous sodium sulphate, filtered and concentrated (at 25°C) to obtain a target product as a crude, which was then purified by silica gel column chromatography to obtain 31c (2.57 g, yield: 33.81%).
LCMS m/z = 289.1 [M+H]⁺

### Step 3: Preparation of compound 31d

Under nitrogen atmosphere, 31c (2.57 g, 8.91 mmol) was dissolved in diethyl ether (30 mL) and precooled in an ice bath for 10 min. A solution of potassium tert-butoxide in tetrahydrofuran (1.43 mL, 1 M) was slowly added dropwise, and the mixture was further reacted in an ice bath for 2 h. In an ice bath, the reaction was quenched by slowly dropwise adding glacial acetic acid (0.7 mL) and water (30 mL) to the system, and the mixture was extracted with ethyl acetate (30 mLx3). The organic phases were combined, washed with a saturated aqueous sodium bicarbonate solution (100 mL), collected, dried over anhydrous sodium sulphate, filtered and concentrated to obtain 31d as a crude.

### Step 4: Preparation of compound 31e

31d (1.97 g, 8.13 mmol) was dissolved in dry dichloromethane (20 mL), and precooled at -78°C for 15 minutes. Under nitrogen atmosphere, N,N-diisopropylethylamine (1.26 g, 9.76 mmol) and a solution of trifluoromethanesulfonic anhydride (2.29 g, 8.13 mmol) in dichloromethane (5 mL) were added dropwise, and the mixture was reacted at -78°C for 2 h. In an ice bath, the reaction was quenched by slowly adding a saturated aqueous sodium bicarbonate solution (30 mL) to the reaction system, and the mixture was extracted with dichloromethane (30 mL×3). The organic phases were combined, dried over anhydrous sodium sulphate, filtered and concentrated to obtain a crude, which was then purified by silica gel column chromatography to obtain 31e (0.680 g, yield: 22.35%).

### Step 5: Preparation of compound 31f

31e (0.68 g, 1.82 mmol) was dissolved in toluene (15 mL), and le-1 (0.38 g, 2.00 mmol) and tetrakis(triphenylphosphine)palladium (0.11 g, 0.091 mmol) were sequentially added. Potassium phosphate (1.16 g, 5.46 mmol) was prepared into a 2 M aqueous solution and added to the system. After the addition, the mixture was reacted at 100°C under nitrogen atmosphere for 3 h. The reaction was cooled to room temperature. The reaction system was filtered, and the filter cake was washed with ethyl acetate (10 mL×2). The filtrate was allowed to stand for layering, and the organic phase was collected. The aqueous phase was extracted with ethyl acetate (30 mL×3). The organic phases were combined, dried over anhydrous sodium sulphate, filtered and concentrated to obtain a crude, which was then purified by silica gel column chromatography to obtain 31f (0.66 g, yield: 98.46%).

### Step 6: Preparation of compound 31g

31f (0.55 g, 1.49 mmol) was dissolved in ethanol (15 mL), and platinum dioxide (0.12 g, 0.51 mmol) was added. The mixture was reacted at 25°C under hydrogen atmosphere for 18 h. The reaction system was filtered, and the filter cake was washed with ethyl acetate (5 mL×2). The filtrate was collected and concentrated to obtain a crude, which was then purified by silica gel column chromatography to obtain 31 g (0.53 g, yield: 96.05%).

### Step 7: Preparation of compound 31h

31 g (0.53 g, 1.43 mmol) was dissolved in ethanol (15 mL), caesium carbonate (0.70 g, 2.15 mmol) was added, and the mixture was reacted at 50°C for 2 h. The reaction system was concentrated to obtain a crude, and the crude was dissolved in water (15 mL). The mixture was adjusted to pH = 1-2 with 1 N hydrochloric acid and extracted with ethyl acetate (20 mLx3). The organic phases were combined, dried over anhydrous sodium sulphate, filtered and concentrated to obtain 31h as a crude.
LCMS m/z =355.3 [M-H]⁻

### Step 8: Preparation of compounds 31i-1a and 31i-1b

31h (0.51 g, 1.43 mmol) was dissolved in ethyl acetate (15 mL), and triethylamine (0.87 g, 8.59 mmol) and T3P (50% wt in EtOAc 3.64 g; containing 1-propylphosphoric anhydride 1.82 g, 5.72 mmol) were sequentially added. 1h-2 (0.33 g, 2.15 mmol) was added, and the mixture was reacted at room temperature under nitrogen atmosphere for 18 h. An aqueous sodium bicarbonate solution (15 mL) was added to the reaction system, and the mixture was extracted with ethyl acetate (20 mLx3). The organic phases were combined, dried over anhydrous sodium sulphate, filtered and concentrated to obtain a crude, which was then purified by silica gel column chromatography to obtain a mixture of 31i-1a and 31i-1b (0.34 g, yield: 48.43%).
LCMS m/z = 491.1 [M+H]⁺

### Step 9: Preparation of compound 31-1a and compound 31-1b

The mixture of 31i-1a and 31i-1b (0.34 g, 0.70 mmol) was dissolved in a 7 M ammonia methanol solution (10 mL), and the resulting mixture was reacted at room temperature for 18 h. The reaction system was concentrated to obtain a crude, which was then subjected to prep-HPLC (neutral preparation) and lyophilized to obtain a mixture of compound 31-1a and compound 31-1b (0.21 g, yield: 63.10%).
LCMS m/z = 476.1 [M+H]⁺;
¹H NMR (400 MHz, CDCl₃): δ 9.84 (s, 1H), 8.45 (d, 1H), 8.36 - 8.29 (m, 1H), 8.13 (d, 1H), 8.08 - 7.96 (m, 1H), 7.05 - 6.96 (m, 1H), 6.89 - 6.78 (m, 1H), 6.07 - 5.95 (m, 1H), 4.74 (d, 1H), 4.38 - 4.25 (m, 1H), 4.06 (d, 3H), 2.60 (t, 1H), 2.33 - 2.22 (m, 1H), 1.79 (s, 3H).

The mixture of compounds 31-1a and 31-1b (0.21 g) was subjected to preparative SFC and lyophilized to obtain compound 31-1P1 (86.0 mg, retention time: 5.345 min) and compound 31-1P2 (90.0 mg, retention time: 7.364 min). chiral testing method: (instrument: SHIMADZU LC-20AT, chiral column: DAICEL AD-H; preparation method: the crude was dissolved in acetonitrile to prepare a sample solution; mobile phase system: n-hexane/ethanol, with ethanol content of 20%; flow rate: 1.0 mL/min; elution time: 30 min).

SFC preparative conditions:
instrument: SFC Prep 150 AP; preparative column: DAICEL IC-H (19 mm×250 mm); preparation method: the crude was dissolved in methanol and filtered with a 0.45 µm filter to prepare a sample solution; mobile phase system: carbon dioxide/methanol:isopropanol (1 : 1), with methanol:isopropanol (1 : 1) content of 18%; flow rate: 35 mL/min.

Compound 31-1P1 has a structure of either formula 31-1a or formula 31-1b shown above, and is an enantiomer of compound 31-1P2, that is, when compound 31-1P1 has a structure as shown in formula 31-1a, compound 31-1P2 has a structure as shown in formula 31-1b; and when compound 31-1P1 has a structure as shown in formula 31-1b, compound 31-1P2 has a structure as shown in formula 31-1a.

### Example 32: Preparation of compound 32-1a and compound 32-1b

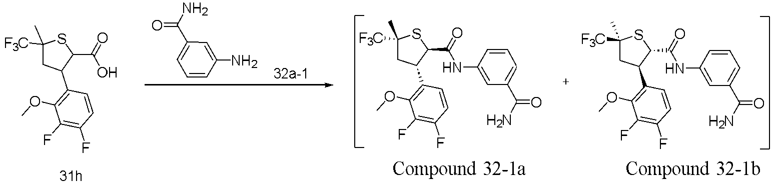

The mixture of 31h (0.18 g, 0.5 mmol) was dissolved in ethyl acetate (15 mL), and triethylamine (0.30 g, 3 mmol) and T3P (50% wt in EtOAc 1.28 g; containing 1-propylphosphoric anhydride 0.64 g, 2 mmol) were sequentially added. 32a-1 (0.10 g, 0.75 mmol) was added, and the mixture was reacted at room temperature under nitrogen atmosphere for 18 h. The reaction was quenched by adding an aqueous sodium bicarbonate solution (15 mL) to the reaction system, and the mixture was extracted with ethyl acetate (20 mLx3). The organic phases were combined, dried over anhydrous sodium sulphate, filtered and concentrated to obtain a crude, which was then purified by silica gel column chromatography to obtain a mixture (120 mg) of compound 32-1a and compound 32-1b.
LCMS m/z = 475.1 [M+H]⁺
¹H NMR (400 MHz, CDCl₃): δ 8.46 (s, 1H), 7.99 - 7.94 (m, 1H), 7.93 - 7.87 (m, 1H), 7.51 - 7.45 (m, 1H), 7.38 (t, 1H), 7.04 - 6.96 (m, 1H), 6.90 - 6.80 (m, 1H), 6.36 - 5.53 (m, 2H), 4.52 (d, 1H), 4.27 - 4.14 (m, 1H), 4.00 (d, 3H), 2.59 (t, 1H), 2.29 - 2.20 (m, 1H), 1.81 (s, 3H).

The mixture of compounds 32-1a and 32-1b (120.0 mg) was subjected to preparative SFC and lyophilized to obtain compound 32-1P1 (48.0 mg, retention time: 5.732 min) and compound 32-1P2 (50.0 mg, retention time: 5.168 min). chiral testing method: (instrument: SHIMADZU LC-20AT, chiral column: DAICEL AD-H; preparation method: the crude was dissolved in acetonitrile to prepare a sample solution; mobile phase system: n-hexane/ethanol, with ethanol content of 20%; flow rate: 1.0 mL/min; elution time: 30 min).

SFC preparative conditions:
instrument: SFC Prep 150 AP; preparative column: DAICEL IC-H (19 mm×250 mm); preparation method: the crude was dissolved in methanol and filtered with a 0.45 µm filter to prepare a sample solution; mobile phase system: carbon dioxide/methanol:isopropanol (9 : 1), with methanol:isopropanol (9 : 1) content of 20%; flow rate: 40 mL/min.

Compound 32-1P1 has a structure of either formula 32-1a or formula 32-1b shown above, and is an enantiomer of compound 32-1P2, that is, when compound 32-1P1 has a structure as shown in formula 32-1a, compound 32-1P2 has a structure as shown in formula 32-1b; and when compound 32-1P1 has a structure as shown in formula 32-1b, compound 32-1P2 has a structure as shown in formula 32-1a.

### Example 33: Preparation of compound 33-1a and compound 33-1b

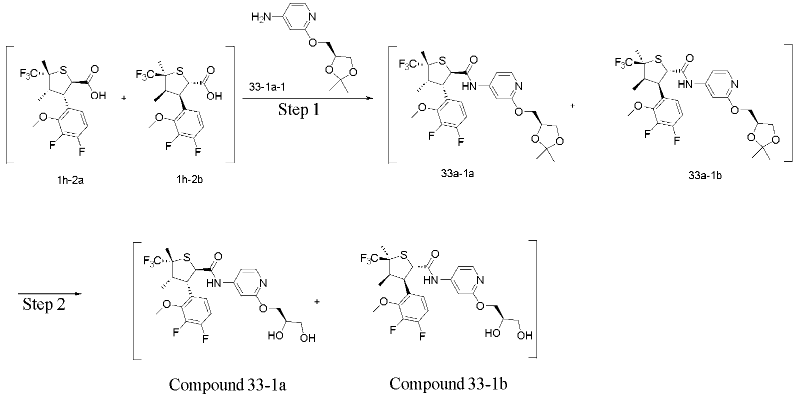

### Step 1: Preparation of compound 33-1a and compound 33-1b

The mixture of 1h-2a and 1h-2b (0.05 g, 0.13 mmol) was dissolved in oxalyl chloride (1 mL). In an ice bath, DMF (0.002 g, 0.028 mmol) was added dropwise, and the mixture was reacted at room temperature for 2 h. After concentration, the crude was added to a solution of 33-1a-1 (prepared with reference to WO 2022121517 A1) (0.044 g, 0.20 mmol) and triethylamine (0.039 g, 0.39 mmol) in dichloromethane (1 mL), and the mixture was reacted overnight at room temperature. The reaction mixture was concentrated to obtain a crude, which was then purified by silica gel column chromatography to obtain a mixture of 33a-1a and 33a-1b (0.036 g, yield: 48.03%).

### Step 2: Preparation of compound 33-1a and compound 33-1b

Hydrogen chloride in dioxane (4 M, 1 mL) was added to the mixture of 33a-1a and 33a-1b (0.036 g, 0.062 mmol). The mixture was reacted at room temperature under nitrogen atmosphere for 18 h. The reaction mixture was concentrated to obtain a crude, which was then purified by prep-HPLC (condition 1) to obtain a mixture of compound 33-1a and compound 33-1b (0.01 g, yield: 30.06%).
LCMS m/z = 537.5 [M+H]⁺

### Example 34: Preparation of compound 34-1a and compound 34-1b

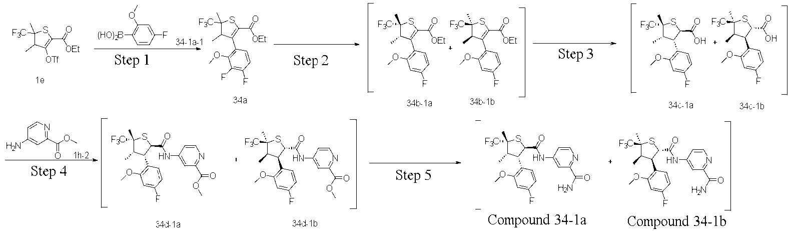

### Step 1: Preparation of compound 34a

1e (0.5 g, 1.29 mmol) was dissolved in toluene (5 mL), and 34-1a-1 (0.24 g, 1.42 mmol) and Pd(PPh₃)₄ (0.075 g, 0.065 mmol) were sequentially added. Potassium phosphate (0.82 g, 3.87 mmol) was prepared into a 2 M aqueous solution and added to the system, and the mixture was reacted at 100°C under nitrogen atmosphere for 6 h. The reaction was cooled to room temperature. The reaction system was filtered, and the filter cake was washed with ethyl acetate (10 mL×2). The filtrate was allowed to stand for layering, and the organic phase was collected. The aqueous phase was extracted with ethyl acetate (30 mL×3). The organic phases were combined, dried over anhydrous sodium sulphate, filtered and concentrated to obtain a crude, which was then purified by silica gel column chromatography to obtain 34a (0.45 g, yield: 91.72%).

### Step 2: Preparation of compounds 34b-1a and 34b-1b

34a (0.45 g, 1.24 mmol) was dissolved in methanol (5 mL), and palladium on carbon (0.3 g, 0.29 mmol) was added. Under hydrogen atmosphere, the pressure was increased to 2.5 Mpa, and the mixture was reacted at 90°C for 72 h. The reaction was filtered, and the filtrate was concentrated to obtain a crude, which was then purified by silica gel column chromatography to obtain a mixture of 34b-1a and 34b-1b (0.26 g, yield: 57.55%).

### Step 3: Preparation of compounds 34c-1a and 34c-1b

The mixture of 34b-1a and 34b-1b (0.25 g, 0.69 mmol) was dissolved in ethanol (3 mL), and caesium carbonate (0.34 g, 1.03 mmol) was added. The mixture was reacted at 50°C for 4 h. In an ice bath, the system was adjusted to pH = 1 by slowly dropwise adding 1 N hydrochloric acid, and water (5 mL) was added. The mixture was extracted with ethyl acetate (10 mLx3). The organic phases were combined, dried over anhydrous sodium sulphate, filtered and concentrated to obtain a crude mixture of 34c-1a and 34c-1b.

### Step 4: Preparation of compounds 34d-1a and 34d-1b

The mixture of 34c-1a and 34c-1b (0.1 g, 0.28 mmol) was dissolved in dichloromethane (1 mL). In an ice bath, oxalyl chloride (0.36 g, 2.80 mmol) was slowly added dropwise, and DMF (0.002 g, 0.028 mmol) was added dropwise. The mixture was reacted at room temperature for 2 h. After concentration, the crude was added to a solution of 1h-2 (0.062 g, 0.41 mmol) and triethylamine (0.082 g, 0.81 mmol) in dichloromethane (1 mL), and the mixture was reacted overnight at room temperature. The reaction mixture was concentrated to obtain a crude, which was then purified by silica gel column chromatography to obtain a mixture of 34d-1a and 34d-1b (0.078 g, yield: 59.38%).

### Step 5: Preparation of compound 34-1a and compound 34-1b

The mixture of 34d-1a and 34d-1b (0.078 g, 0.16 mmol) was dissolved in a 7 M ammonia methanol solution (5 mL), and the resulting mixture was reacted at room temperature for 18 h. The reaction system was concentrated to obtain a crude, which was then subjected to prep-HPLC (condition 1) and lyophilized to obtain a mixture of compound 34-1a and compound 34-1b (0.034 g, yield: 45.07%).
LCMS m/z = 472.1 [M+H]⁺
¹H NMR (400 MHz, CDCl₃): δ 9.60 (s, 1H), 8.45 (d, 1H), 8.30 (d, 1H), 8.11 (s, 1H), 8.06 (s, 1H), 7.25 - 7.18 (m, 1H), 6.62 (ddd, 2H), 5.88 (s, 1H), 4.87 (d, 1H), 4.55 (dd, 1H), 3.85 (s, 3H), 2.76 (q, 1H), 1.93 (s, 3H), 0.86 (dd, J = 6.9, 2.3 Hz, 3H).

### Example 35: Preparation of compound 35-8a and compound 35-8b

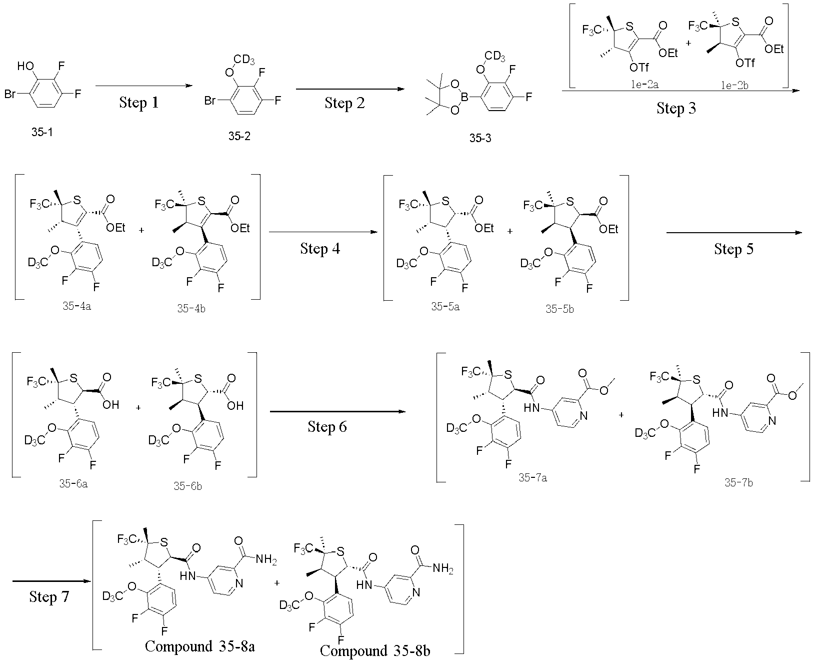

### Step 1: Preparation of compound 35-2

35-1 (2 g, 9.57 mmol) was dissolved in DMF (40 mL), and NaH (574 mg, 14.36 mmol) was added in an ice bath. The mixture was further reacted for 30 minutes. Deuterated iodomethane (2.77 g, 19.14 mmol) was added, and the mixture was reacted at room temperature under nitrogen atmosphere for 5 h. Water (100 mL) was added, and the mixture was extracted with ethyl acetate (100 mL×3). The organic phases were combined, dried over anhydrous sodium sulphate and then filtered. The filtrate was concentrated, and the residue was separated and purified by silica gel column chromatography to obtain 35-2 (1.1 g, yield: 50.85%)

### Step 2: Preparation of compound 35-3

35-1 (400 mg, 1.77 mmol), (Bpin)₂ (539.4 mg, 2.12 mmol), potassium acetate (521.1 mg, 5.31 mmol) and Pd(dppf)Cl₂•DCM (114.6 mg, 0.18 mmol) were added to 1,4-dioxane (10 mL), and the mixture was reacted at 100°C under nitrogen atmosphere for 5 h. A reaction solution of 35-3 was obtained.

### Step 3: Preparation of compounds 35-4a and 35-4b

1e-2a and 1e-2b (713 mg, 1.77 mmol), Pd(PPh₃)₄ (102.3 mg, 0.09 mmol), potassium phosphate (1.13 g, 5.33 mmol) and water (1 mL) were added to the reaction solution of 35-3 from the previous step, and the mixture was reacted at 100°C under nitrogen atmosphere for 16 h. The reaction solution was cooled to room temperature and then concentrated under reduced pressure. The residue was separated and purified by silica gel column chromatography to obtain a mixture of 35-4a and 35-4b (500 mg, yield: 70.73%).
LCMS m/z =400.1 [M+H]⁺

### Step 4: Preparation of compounds 35-5a and 35-5b

The mixture of 35-4a and 35-4b (300 mg, 0.75 mmol) was dissolved in methanol (8 mL), and platinum oxide (52 mg) was added. The mixture was reacted at room temperature under hydrogen atmosphere for 18 h. The mixture was filtered through celite, and the filtrate was concentrated. The residue was separated and purified by silica gel column chromatography to obtain a mixture of 35-5a and 35-5b (80 mg, yield: 26.57%).

### Step 5: Preparation of compounds 35-6a and 35-6b

The mixture of 35-5a and 35-5b (80 mg, 0.20 mmol) was dissolved in anhydrous ethanol (3 mL), and caesium carbonate (98 mg, 0.3 mmol) was added. The mixture was reacted at 50°C for 3 h. The reaction solution was cooled to room temperature, and added to 1 M hydrochloric acid (5 mL) to quench the reaction. The reaction mixture was extracted with ethyl acetate (50 mL×3). The organic phases were combined, dried over anhydrous sodium sulphate and then filtered. The filtrate was concentrated, and the residue was separated and purified by silica gel column chromatography to obtain a mixture of 35-6a and 35-6b (69 mg, yield: 92.41 %).
LCMS m/z =372.3 [M-H]⁻

### Step 6: Preparation of compounds 35-7a and 35-7b

The mixture of 35-6a and 35-6b (50 mg, 0.13 mmol) was dissolved in a solution of oxalyl chloride in dichloromethane (2 M, 2 mL), and DMF (0.002 g, 0.028 mmol) was added dropwise in an ice bath. The mixture was reacted at room temperature for 2 h. After concentration, the crude was added to a solution of methyl 4-aminopyridine-2-carboxylate (30 mg, 0.20 mmol) and triethylamine (39 mg, 0.39 mmol) in dichloromethane (2 mL), and the mixture was reacted overnight at room temperature. The reaction mixture was concentrated to obtain a crude, which was then purified by silica gel column chromatography to obtain a mixture of 35-7a and 35-7b (15 mg, yield: 22.74%).
LCMS m/z =508.3 [M+H]⁺

### Step 7: Preparation of compounds 35-8a and 35-8b

The mixture of 35-7a and 35-7b (15 mg, 0.03 mmol) was dissolved in a 7 M ammonia methanol solution (1 mL), and the resulting mixture was reacted at room temperature for 18 h. The reaction system was concentrated to obtain a crude, which was then subjected to silica gel column chromatography to obtain a mixture of compound 35-8a and compound 35-8b (5.64 mg, yield: 38.17%).
LCMS m/z =493.4 [M+H]⁺

### Example 36: Preparation of compound 36-1a and compound 36-1b

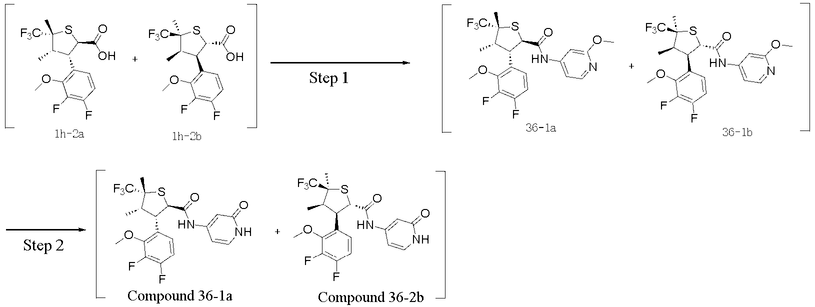

### Step 1: Preparation of compounds 36-1a and 36-1b

The mixture of 1h-2a and 1h-2b (50 mg, 0.14 mmol) was dissolved in a solution of oxalyl chloride in dichloromethane (2 M, 2 mL), and DMF (0.002 g, 0.028 mmol) was added dropwise in an ice bath. The mixture was reacted at room temperature for 2 h. After concentration, the crude was added to a solution of 4-amino-2-methoxypyridine (26 mg, 0.21 mmol) and triethylamine (42 mg, 0.42 mmol) in dichloromethane (2 mL), and the mixture was reacted overnight at room temperature. The reaction mixture was concentrated to obtain a crude, which was then purified by silica gel column chromatography to obtain a mixture of 36-1a and 36-1b (55 mg, yield: 82.45%).
LCMS m/z =477.1 [M+H]⁺

### Step 2: Preparation of compounds 36-2a and 36-2b

The mixture of 36-1a and 36-1b (45 mg, 0.10 mmol) was dissolved in THF (1 mL) and HCl (1 mL), and the mixture was reacted at 60°C for 18 h. The reaction system was concentrated to obtain a crude, which was then subjected to prep-HPLC (condition 1) to obtain a mixture of compound 36-2a and compound 36-2b (4 mg, yield: 8.65%).
LCMS m/z =463.1 [M+H]⁺

### Example 37: Preparation of compound 37-1a and compound 37-1b

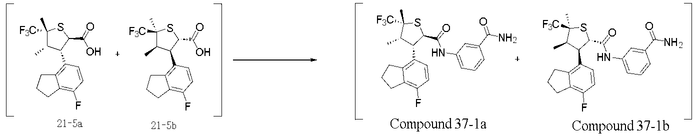

The mixture of 21-5a and 21-5b (70 mg, 0.19 mmol) was dissolved in tetrahydrofuran (3 mL), and triethylamine (120 mg, 1.14 mmol) and T3P (50% wt in EtOAc 480 mg; containing 1-propylphosphoric anhydride 240 mg, 0.76 mmol) were sequentially added. 3-aminobenzamide (26 mg, 0.19 mmol) was added, and the mixture was reacted at room temperature under nitrogen atmosphere for 18 h. The reaction was quenched by adding an aqueous sodium bicarbonate solution (10 mL), and the mixture was extracted with ethyl acetate (10 mL×3). The organic phases were combined, dried over anhydrous sodium sulphate, filtered and concentrated to obtain a crude, which was then subjected to prep-HPLC (condition 1) and lyophilized to obtain a mixture of compound 37-1a and compound 37-1b (43 mg, yield: 47.1%).
LCMS m/z =481.2 [M+H]⁺

### Example 38: Preparation of compound 1-2a

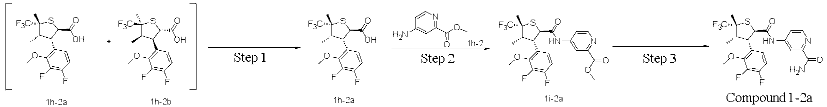

### Step 1: Preparation of compound 1h-2a

The mixture of compounds 1h-2a and 1h-2b (3.0 g) was subjected to preparative SFC and lyophilized to obtain compound 1h-2a (1.28 g, retention time for chiral HPLC: 0.760 min) and compound 1h-2b (1.11 g, retention time for chiral HPLC: 0.966 min). Chiral HPLC testing method: (instrument: SHIMADZU LC-30 AD; chiral column: Chiralcel IG column; preparation method: the crude was dissolved in acetonitrile to prepare a sample solution; mobile phase system: carbon dioxide/0.05% DEA in ethanol; elution gradient: 5%-40%; elution time: 3 min).

SFC preparative conditions: instrument: Waters 150 Prep-SFC A; preparative column: Chiralcel IG column; preparation method: the crude was dissolved in acetonitrile to prepare a sample solution at a concentration of 2 mg/mL; mobile phase system: carbon dioxide/ethanol, with ethanol content of 10%; flow rate: 100 mL/min; elution time: 2 min.

### Compound 1h-2a:

¹HNMR(400MHz,CDCl₃): δ6.91-6.78(m,2H),4.53 (d,1H),4.46-4.34(m,1H),4.02(d,3H),2.68-2.54 (m,1H),1.85(s,3H),0.88-0.75(m,3H).

### Step 2: Preparation of compound 1i-2a

1h-2a (1.0 g, 2.7 mmol) was dissolved in tetrahydrofuran (20 mL), and triethylamine (1.64 g, 16.20 mmol) and T3P (50% wt in EtOAc 6.88 g; containing 1-propylphosphoric anhydride 3.44 g, 10.79 mmol) were sequentially added. The mixture was stirred at room temperature for 15 minutes, and 1h-2 (0.62 g, 4.07 mmol) was added. The resulting mixture was reacted at room temperature under nitrogen atmosphere for 18 h. A saturated aqueous sodium bicarbonate solution (40 mL) was added, and the mixture was extracted with ethyl acetate (30 mL×3). The organic phases were combined, dried over anhydrous sodium sulphate, filtered and concentrated to obtain a crude, which was then purified by silica gel column chromatography to obtain compound 1i-2a (0.919 g, yield: 67.47%)

### Step 3: Preparation of compound 1-2a

With reference to step 4 of Example 2, compound 1-2a (0.87 g) was obtained.

The absolute configuration of compound 1-2a was verified by Micro-ED.

### Example 39: Preparation of compound 6-2a

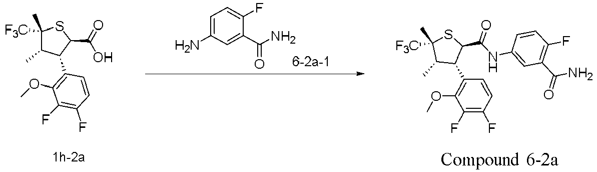

1h-2a (0.037 g, 0.1 mmol) was dissolved in tetrahydrofuran (3 mL), and triethylamine (0.061 g, 0.60 mmol), T3P (50% wt in EtOAc 0.26 g; containing 1-propylphosphoric anhydride 0.13 g, 0.40 mmol) and 6-2a-1 (0.023 g, 0.15 mmol) were sequentially added. The mixture was reacted at room temperature under nitrogen atmosphere for 18 h. An aqueous sodium bicarbonate solution (10 mL) was added, and the mixture was extracted with ethyl acetate (10 mL×3). The organic phases were combined, dried over anhydrous sodium sulphate, filtered and concentrated to obtain a crude, which was then purified by silica gel column chromatography to obtain compound 6-2a (0.036 g, yield: 71.08%).

¹HNMR(400MHz,CDCl₃): δ9.69 (s,1H),8.45-8.37 (m,lH),8.10-8.04 (m,1H),7.15-7.06 (m,1H), 6.98 -6.88 (m,2H) , 6.87-6.78 (m,1H), 6.47(s,1H),4.84(d,1H), 4.63-4.54 (m,1H),4.08(d,3H),2.73-2.63(m,1H),1.93(s,3H),0.93-0.83(m,3H).
LCMS m/z = 507.2 [M+H]⁺

### Example 40: Preparation of compound 28-2a

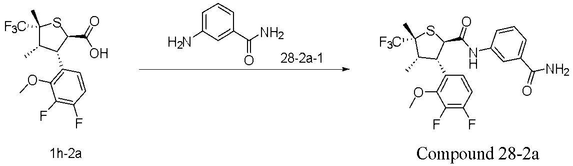

1h-2a (0.037 g, 0.1 mmol) was dissolved in tetrahydrofuran (5 mL), and triethylamine (0.061 g, 0.60 mmol) and T3P (50% wt in EtOAc 0.26 g; containing 1-propylphosphoric anhydride 0.13 g, 0.40 mmol) were sequentially added. 28-2a-1 (0.055 g, 0.41 mmol) was added, and the mixture was reacted at room temperature under nitrogen atmosphere for 18 h. An aqueous sodium bicarbonate solution (10 mL) was added, and the mixture was extracted with ethyl acetate (15 mL×3). The organic phases were combined, dried over anhydrous sodium sulphate, filtered and concentrated to obtain a crude, which was then purified by silica gel column chromatography to obtain compound 28-2a (0.106 g, yield: 80.37%).

### Example 41: Preparation of compound 8-1a

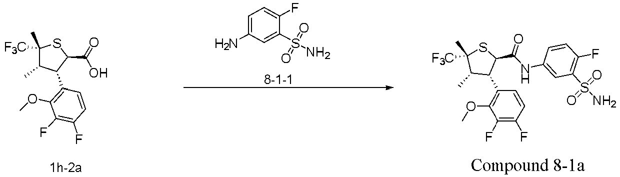

By using 1h-2a (100 mg, 0.27 mmol), according to the synthesis method of Example 8, separation and purification by silica gel column chromatography was performed to obtain compound 8-1a (95 mg, yield: 62.9%).
LCMS m/z = 543.2 [M+H]⁺
¹H NMR (400 MHz, CDCl₃): δ 8.54 (s, 1H), 8.14 - 8.05 (m, 1H), 7.74 - 7.65 (m, 1H), 7.13 (t, 1H), 7.04 - 6.95 (m, 1H), 6.89 - 6.79 (m, 1H), 5.33 (s, 2H), 4.67 (d, 1H), 4.59 - 4.47 (m, 1H), 4.04 (d, 3H), 2.69 - 2.55 (m, 1H), 1.91 (s, 3H), 0.93 - 0.85 (m, 3H).

### Example 42: Preparation of compound 26-1a

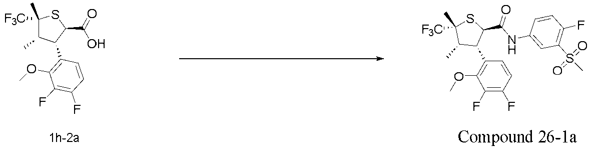

1h-2a (100 mg, 0.27 mmol) was dissolved in tetrahydrofuran (5 mL), and triethylamine (164 mg, 1.62 mmol), T3P (50% wt in EtOAc 686 mg; containing 1-propylphosphoric anhydride 343 mg, 1.08 mmol), and 4-fluoro-3-(methylsulfonyl)aniline (76 mg, 0.405 mmol) were sequentially added. The mixture was reacted at room temperature under nitrogen atmosphere for 18 h. The reaction was quenched by adding an aqueous sodium bicarbonate solution (10 mL) to the reaction system, and the mixture was extracted with ethyl acetate (10 mL×3). The organic phases were combined, dried over anhydrous sodium sulphate, filtered and concentrated to obtain a crude, which was then separated and purified by silica gel column chromatography to obtain compound 26-1a (132 mg, yield: 90%).

### Example 43: Preparation of compound 27-1a

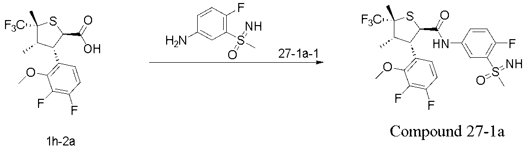

By using 1h-2a as the substrate, with reference to the synthesis condition of Example 27, 27-1a was obtained.
LCMS m/z = 541.1 [M+H]⁺

### Example 44: Preparation of compound 25-1a

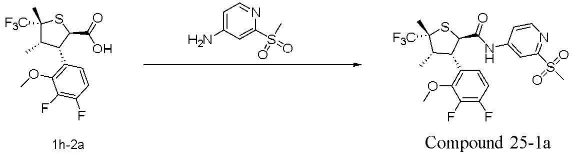

1h-2a (0.10 g, 0.27 mmol) was dissolved in tetrahydrofuran (5 mL), and triethylamine (0.16 g, 1.62 mmol) and T3P (50% wt in EtOAc 0.69 g; containing 1-propylphosphoric anhydride 0.345 g, 1.08 mmol) were sequentially added. 2-(methylsulfonyl)pyridin-4-amine (0.070 g, 0.41 mmol) was added, and the mixture was reacted at room temperature under nitrogen atmosphere for 18 h. The reaction was quenched by adding an aqueous sodium bicarbonate solution (10 mL) to the reaction system, and the mixture was extracted with ethyl acetate (15 mL×3). The organic phases were combined, dried over anhydrous sodium sulphate, filtered and concentrated to obtain a crude, which was then purified by silica gel column chromatography to obtain 25-1a (0.05 g, yield: 35.31%).

### Example 45: Preparation of compound 5-3a

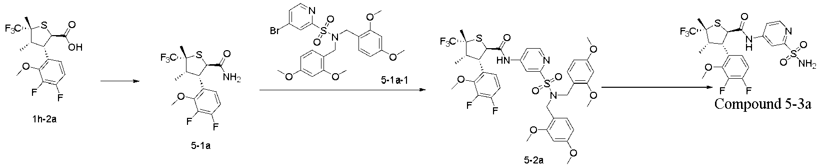

By using 1h-2a as the substrate, with reference to the synthesis method of Example 5, 5-3a was obtained.
LCMS m/z = 526.0 [M+H]⁺

### Example 46: Preparation of compound 7-1a

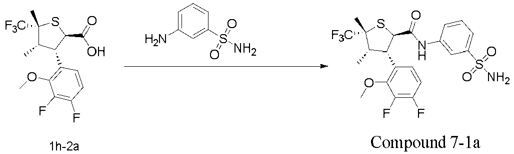

By using 1h-2a as the substrate, with reference to the synthesis method of Example 7, compound 7-1a was obtained.

### Example 47: Preparation of compound 19-2a

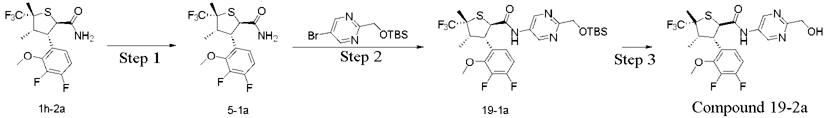

By using 1h-2a as the substrate, with reference to the synthesis method of Example 19, compound 19-2a was obtained.
LCMS m/z =478.50 [M+H]⁺

### Example 48: Preparation of compound 48-1a

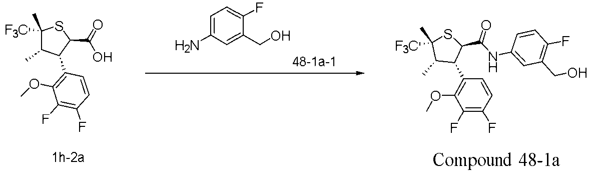

1h-2a (0.037 g, 0.1 mmol) was dissolved in tetrahydrofuran (3 mL), and triethylamine (0.061 g, 0.60 mmol) and T3P (50% wt in EtOAc 0.25 g; containing 1-propylphosphoric anhydride 0.125 g, 0.40 mmol) were sequentially added. 48-1a-1 (0.021 g, 0.15 mmol) was added, and the mixture was reacted at room temperature under nitrogen atmosphere for 18 h. An aqueous sodium bicarbonate solution (10 mL) was added, and the mixture was extracted with ethyl acetate (10 mLx3). The organic phases were combined, dried over anhydrous sodium sulphate, filtered and concentrated to obtain a crude, which was then purified by silica gel column chromatography to obtain 48-1a (0.02 g, yield: 40.53%).
LCMS m/z = 494.2 [M+H]⁺
¹H NMR (400 MHz, DMSO-*d₆*): δ 10.38 (s, 1H), 7.66 - 7.62 (m, 1H), 7.45 - 7.38 (m, 1H), 7.22 - 7.14 (m, 1H), 7.11 - 7.01 (m, 1H), 5.26 (t, 1H), 4.83 (d, 1H), 4.52 - 4.44 (m, 3H), 3.99 (d, 3H), 2.65 - 2.56 (m, 1H), 1.85 (s, 3H), 0.82 - 0.74 (m, 3H).

### Example 49: Preparation of compound 18-2a

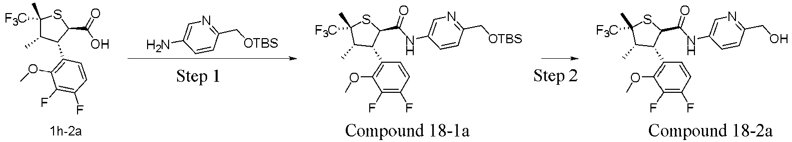

By using 1h-2a as the substrate, with reference to the synthesis method of Example 18, compound 18-2a was obtained.
LCMS m/z =477.1 [M+H]⁺

### Example 50: Preparation of compound 50

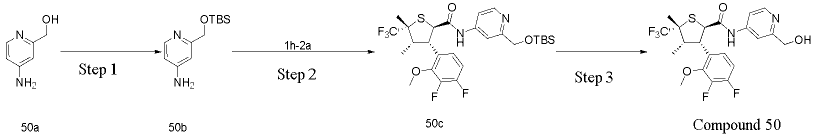

### Step 1: Preparation of compound 50b

50a (524 mg, 4.26 mmol) and imidazole (0.44 g, 6.39 mmol) were added to DMF (10 mL), and TBSCl (0.77 g, 5.11 mmol) was added. The mixture was reacted at room temperature for 3 h. The reaction solution was directly concentrated under reduced pressure, and the residue was separated and purified by silica gel column chromatography to obtain 50b (451 mg, yield: 44.4%).
LCMS m/z = 239.3 [M+H]⁺

### Step 2: Preparation of compound 50c

1h-2a (50 mg, 0.14 mmol), 50b (67 mg, 0.28 mmol), T3P (50% wt in EtOAc 362 mg; containing 1-propylphosphoric anhydride 181 mg, 0.57 mmol), and TEA (85 mg, 0.84 mmol) were added to ethyl acetate (5 mL), and the mixture was reacted at room temperature for 16 h. The reaction was quenched by adding the reaction solution to an aqueous sodium bicarbonate solution, and the mixture was extracted with ethyl acetate. The organic phase was dried over anhydrous sodium sulphate and then filtered. The filtrate was concentrated, and the residue was separated and purified by silica gel column chromatography to obtain 50c (24 mg, yield: 29.02%).
LCMS m/z =591.5 [M+H]⁺

### Step 3: Preparation of compound 50

Compound 50c (24 mg, 0.041 mmol) was added to methanol (5 mL), and concentrated hydrochloric acid (1 mL) was slowly added. The mixture was reacted at room temperature for 3 h. The reaction solution was concentrated under reduced pressure. Ethyl acetate (50 mL) was added, and then a saturated aqueous sodium bicarbonate solution was added for extraction. The organic phase was separated, dried over anhydrous sodium sulphate and then filtered. The filtrate was concentrated, and the residue was separated and purified by silica gel column chromatography to obtain compound 50 (8 mg, yield: 38.90%).
LCMS m/z =477.2 [M+H]⁺

### Example 51: Preparation of compound 33-1a

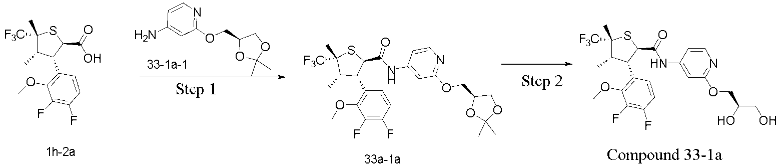

### Step 1: Preparation of 33a-1a

1h-2a (100 mg, 0.27 mmol) was dissolved in a solution of oxalyl chloride in dichloromethane (2 M, 2 mL), and DMF (0.002 g, 0.028 mmol) was added dropwise in an ice bath. The mixture was reacted at room temperature for 2 h. After concentration, the crude was added to a solution of 33-1a-1 (prepared with reference to WO 2022121517 A1) (61 mg, 0.27 mmol) and triethylamine (82 mg, 0.81 mmol) in dichloromethane (2 mL), and the mixture was reacted overnight at room temperature. The reaction mixture was concentrated to obtain a crude, which was then purified by silica gel column chromatography to obtain 33a-1a (100 mg, yield: 64.24%).

### Step 2: Preparation of compound 33-1a

Hydrogen chloride in dioxane (4 M, 1 mL) was added to compound 33a-1a (100 mg, 0.17 mmol). The mixture was reacted at room temperature under nitrogen atmosphere for 18 h. The reaction mixture was concentrated to obtain a crude, which was then purified by prep-HPLC (condition 2) to obtain compound 33-1a (70 mg, yield: 76.75%).

### Example 52: Preparation of compound 36-2a

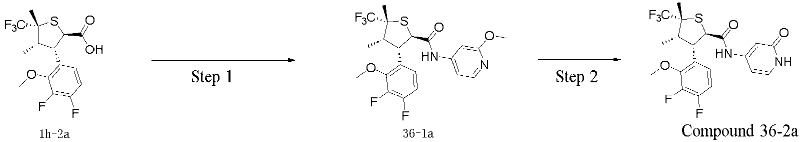

By using 1h-2a as the substrate, with reference to the synthesis method of Example 36, compound 36-2a was obtained.
LCMS m/z =463.1 [M+H]⁺

### Example 53: Preparation of compound 20-1a

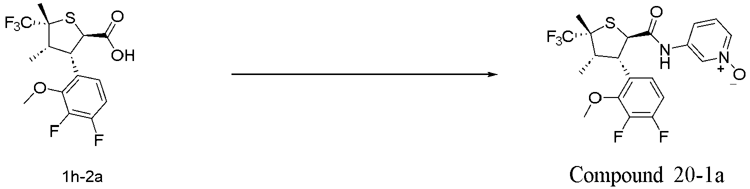

1h-2a (100 mg, 0.27 mmol) was dissolved in tetrahydrofuran (5 mL), and triethylamine (164 mg, 1.62 mmol) and T3P (50% wt in EtOAc 686 mg; containing 1-propylphosphoric anhydride 343 mg, 1.08 mmol) were sequentially added. 3-aminopyridine oxide (60 mg, 0.53 mmol) was added, and the mixture was reacted at room temperature under nitrogen atmosphere for 18 h. The reaction was quenched by adding the reaction solution to an aqueous sodium bicarbonate solution, and the mixture was extracted with ethyl acetate. The organic phase was dried over anhydrous sodium sulphate and then filtered. The filtrate was concentrated. The residue was separated and purified by silica gel column chromatography to obtain compound 20-1a (25 mg, yield: 19.84%).
LCMS m/z = 463.1 [M+H]⁺

### Example 54: Preparation of compound 54

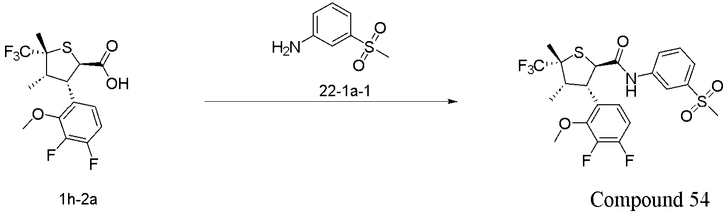

By using 1h-2a (100 mg, 0.27 mmol) and 54-1 (69 mg, 0.40 mmol), according to the synthesis method of Example 22, compound 54 (90 mg, yield: 63.4%) was obtained.
LCMS m/z =524.1 [M+H]⁺;

### Example 55: Preparation of compound 55

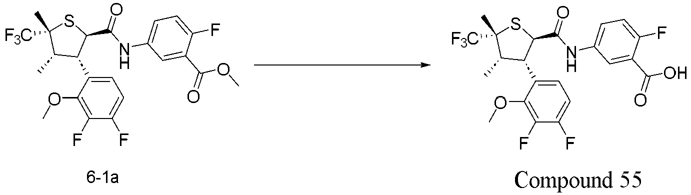

6-1a (40 mg, 0.077 mmol) was added to an aqueous solution (1 ml) of lithium hydroxide (5 mg, 0.19 mmol), and methanol (1 ml) was added. The mixture was reacted at 40°C for 16 h. The reaction solution was concentrated under reduced pressure to obtain a crude, which was then subjected to prep-HPLC (condition 2) to obtain compound 55 (10 mg, yield: 25.56%).
LCMS m/z =508.1 [M+H]⁺

### Example 56: Preparation of compound 56

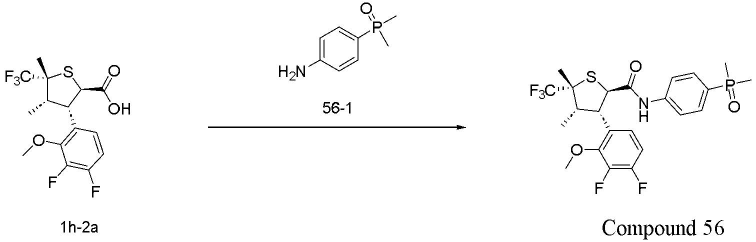

By using 1h-2a (100 mg, 0.27 mmol) and 56-1 (68 mg, 0.405 mmol), according to the synthesis method of Example 22, separation and purification by silica gel column chromatography was performed to obtain compound 56 (137 mg, yield: 94.4%).
LCMS m/z =522.5 [M+H]⁺;

### Example 57: Preparation of compound 57

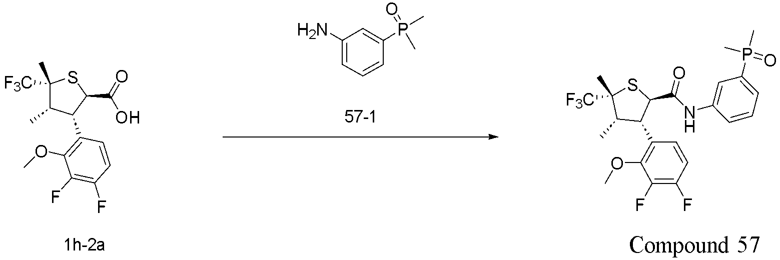

### Preparation of compound 57

By using 1h-2a (74 mg, 0.2 mmol) and 57-1 (50.7 mg, 0.3 mmol), according to the synthesis method of Example 22, separation and purification by silica gel column chromatography was performed to obtain compound 57 (87 mg, yield: 81.7%).
LCMS m/z =522.5 [M+H]⁺;

### Example 58: Preparation of compound 58

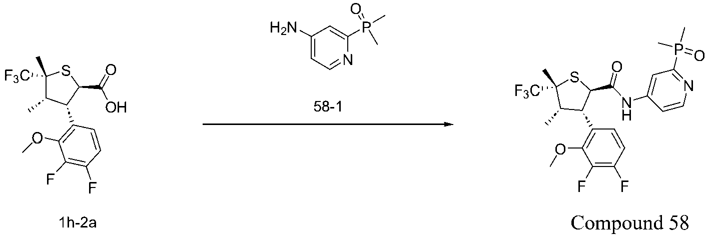

### Preparation of compound 58

By using 1h-2a (100 mg, 0.27 mmol) and 58-1 (69 mg, 0.41 mmol), according to the synthesis method of Example 22, prep-HPLC (condition 2) was performed to obtain the trifluoroacetate of compound 58 (36 mg, yield: 25.5%).
LCMS m/z =523.1 [M+H]⁺;
¹H NMR (400 MHz, CDCl₃) δ 11.37 (s, 1H), 8.71 (d, 1H), 8.61 - 8.52 (m, 1H), 8.16 - 8.07 (m, 1H), 6.91- 6.75 (m, 2H), 4.81 (d, 1H), 4.65 - 4.57 (m, 1H), 4.09 (d, 3H), 2.78 - 2.65 (m, 1H), 2.06 - 1.96 (m, 6H), 1.93 (s, 3H), 0.94 - 0.82 (m, 3H).

1h-2a (254 mg, 0.69 mmol), 58-1 (180 mg, 1.03 mmol), triethylamine (420 mg, 4.14 mmol), T3P (50% wt in EtOAc 1.73 g; containing 1-propylphosphoric anhydride 0.87 g, 2.76 mmol) were added to tetrahydrofuran (10 mL), and the mixture reacted at room temperature for 16 hours. An aqueous sodium bicarbonate solution (20 mL) was added to the reaction system, and the mixture was extracted with ethyl acetate (20 mL×3). The organic phases were combined, dried over anhydrous sodium sulphate, filtered and concentrated to obtain a crude, which was then separated and purified by silica gel column chromatography to obtain compound 58 (220 mg, yield: 60.4%).

### Example 59: Preparation of compound 59-a

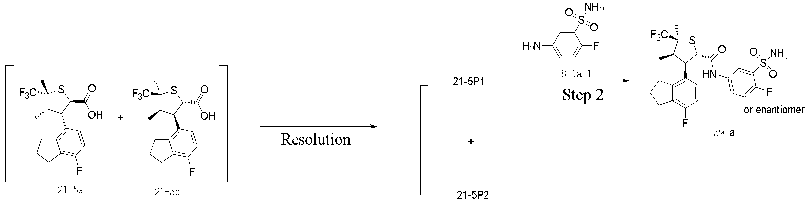

### Step 1: Preparation of compounds 21-5P1 and 21-5P2

The mixture of compound 21-5a and compound 21-5b (1.3 g) was subjected to resolution by preparative SFC to obtain compound 21-5P1 (552 mg, retention time for chiral HPLC: 0.736 min) and compound 21-5P2 (504 mg, retention time for chiral HPLC: 1.033 min).

Compound 21-5P1 is either 21-5a or 21-5b shown above, and is an enantiomer of compound 21-5P2, that is, when compound 21-5P1 has a structure as shown in formula 21-5a, compound 21-5P2 has a structure as shown in formula 21-5b; and when compound 21-5P1 has a structure as shown in formula 21-5b, compound 21-5P2 has a structure as shown in formula 21-5a.

### SFC preparative conditions:

instrument: Waters 150 Prep-SFC C; preparative column: Chiral IC column; preparation method: the crude was dissolved in acetonitrile to prepare a 10 mg/ml sample solution; mobile phase system: carbon dioxide/0.1% NH3•H2O in methanol, with methanol content of 15%; elution time: 8 min;
chiral testing method: (instrument: SHIMADZU LC-30 AD sf; preparative column: Chiral IC column; preparation method: the crude was dissolved in acetonitrile to prepare a sample solution; mobile phase system: carbon dioxide/0.05% DEA in methanol; elution gradient: 5%-40%; flow rate: 3.0 mL/min; elution time: 3 min).

### Step 2: Preparation of compound 59-a

21-5P1 (50 mg, 0.14 mmol) was dissolved in a solution of oxalyl chloride in dichloromethane (2 M, 2 mL), and DMF (0.002 g, 0.028 mmol) was added dropwise in an ice bath. The mixture was reacted at room temperature for 2 h. After concentration, the crude was added to a solution of 8-1a-1 (40 mg, 0.21 mmol) and triethylamine (42.5 mg, 0.42 mmol) in dichloromethane (2 mL), and the mixture was reacted overnight at room temperature. The reaction mixture was concentrated to obtain a crude, which was then purified by silica gel column chromatography to obtain 59-a (50 mg, yield: 66.8%). The chirality orientation of compound 59-a is identical to that of 21-5P1.
LCMS m/z = 535.0 [M+H]⁺

With reference to the method for preparing 59-a (no configuration change occurs during the amidation reaction), the target compounds listed in the table below were prepared with their configurations consistent with those of the substrates.

| Substrate | | Target compound | LCMS or NMR for target compound |
|---|---|---|---|
| 21-5P2 | 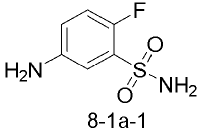 | 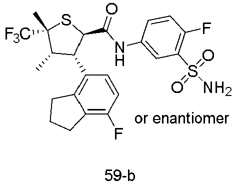 | LCMS m/z = 535.0 [M+H]⁺ |
| 21-5P1 | 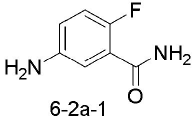 | 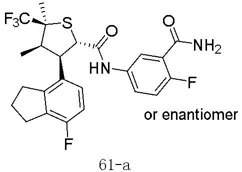 | LCMS m/z =499.5 [M+H]⁺ |
| 21-5P2 | 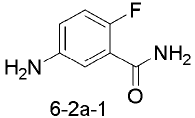 | 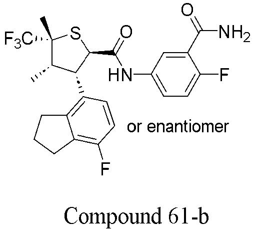 | ¹H NMR (400 MHz, CDCl₃) δ 9.30 (s, 1H), 8.41 - 8.25 (m, 1H), 8.01 (dd, 1H), 7.14 - 6.99 (m, 2H), 6.93 - 6.72 (m, 2H), 6.37 (s, 1H), 4.85 (d, 1H), 4.38 (dd, 1H), 3.11 (dt, 1H), 2.96 (t, 2H), 2.87 (dt, 1H), 2.54 (p, 1H), 2.15 (p, 2H), 2.00 (s, 3H), 0.93 (d, 3H). |
| | | | LCMS m/z =499.5 [M+H]⁺ |
| 21-5P1 | 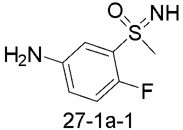 | 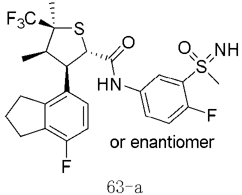 | LCMS m/z =533.0 [M+H]⁺ |
| 21-5P2 | 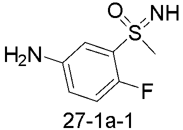 | 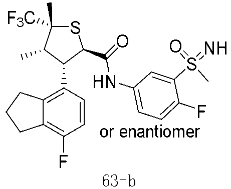 | LCMS m/z =533.0 [M+H]⁺ |
| 21-5P1 | 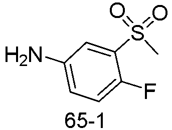 | 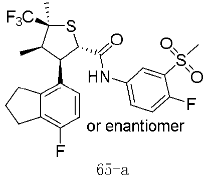 | LCMS m/z =534.0 [M+H]⁺ |
| 21-5P2 | 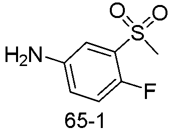 | 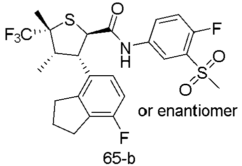 | LCMS m/z = 534.0 [M+H]⁺ |
| 21-5P1 | 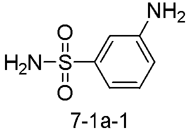 | 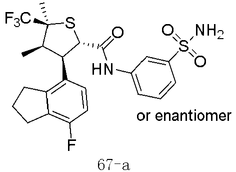 | LCMS m/z =517.1 [M+H]⁺ |
| 21-5P1 | 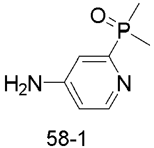 | 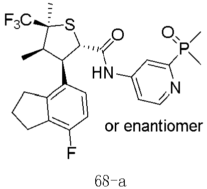 | LCMS m/z =515.3 [M+H]⁺ |
| 21-5P2 | 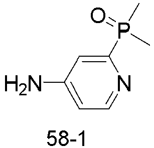 | 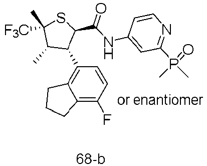 | LCMS m/z = 515.0 [M+H]⁺ |

### Example 70: Preparation of compound 70-a

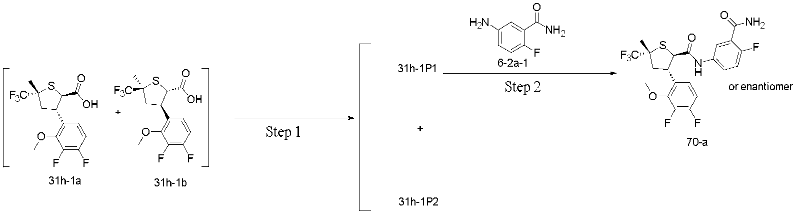

### Step 1: Compounds 31h-1P1 and 31h-1P2

The mixture of compound 31h-1a and 31h-1b (1 gg) was subjected to resolution by preparative SFC to obtain compound 31h-1P1 (412 mg, retention time: 1.589 min) and compound 31h-1P2 (430 mg, retention time: 1.778 min).

Compound 31h-1P1 is either 31h-1a or 31h-1b shown above, and is an enantiomer of compound 31h-1P2, that is, when compound 31h-1P1 has a structure as shown in formula 31h-1a, compound 31h-1P2 has a structure as shown in formula 31h-1b; and when compound 31h-1P1 has a structure as shown in formula 31h-1b, compound 31h-1P2 has a structure as shown in formula 31h-1a.

SFC preparative conditions:
instrument: SFC Prep 150 AP; preparative column: DAICEL AD-H (19 mm×250 mm); preparation method: The sample was dissolved in methanol and filtered with a 0.45 µm filter to prepare a sample solution. mobile phase system: carbon dioxide/ethanol, with ethanol content of 8%; flow rate: 40 ml/min.

### Step 2: Preparation of compound 70-a

31h-1P1 (50 mg, 0.14 mmol) was dissolved in ethyl acetate (3 mL), and triethylamine (85 mg, 0.84 mmol), T3P (50% wt in EtOAc 360 mg; containing 1-propylphosphoric anhydride 180 mg, 0.56 mmol), and 6-2a-1 (43 mg, 0.28 mmol) were sequentially added. The mixture was reacted at room temperature under nitrogen atmosphere for 18 h. An aqueous sodium bicarbonate solution (10 mL) was added, and the mixture was extracted with ethyl acetate (10 mL×3). The organic phases were combined, dried over anhydrous sodium sulphate, filtered and concentrated to obtain a crude, which was then separated and purified by silica gel column chromatography to obtain 70-a (6.28 mg, yield: 9.11%).
LCMS m/z =493.2 [M+H]⁺

With reference to the method for preparing 70-a (no configuration change occurs during the amidation reaction), the target compounds listed in the table below were prepared with their configurations consistent with those of the substrates.

| Substrate | | Target compound | LCMS |
|---|---|---|---|
| 31h-1P2 | 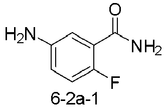 | 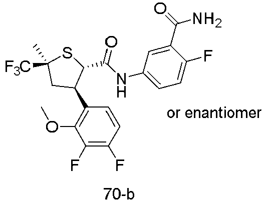 | LCMS m/z =493.1 [M+H]⁺ |
| 31h-1P1 | 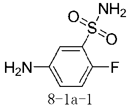 | 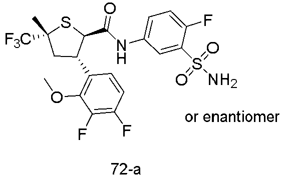 | LCMS m/z =528.9 [M+H]⁺ |
| 31h-1P2 | 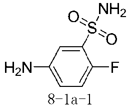 | 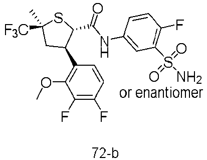 | LCMS m/z = 528.9 [M+H]⁺ |
| 31h-1P1 | 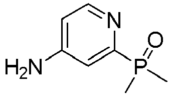 | 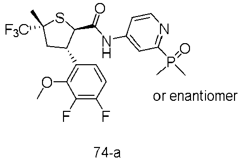 | LCMS m/z =509.2 [M+H]⁺ |
| 31h-1P2 | 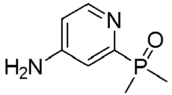 | 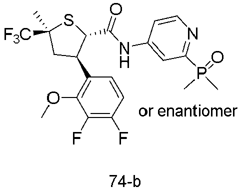 | LCMS m/z = 509.3 [M+H]⁺ |

### Example 76: Preparation of compounds 76-8a and 76-8b

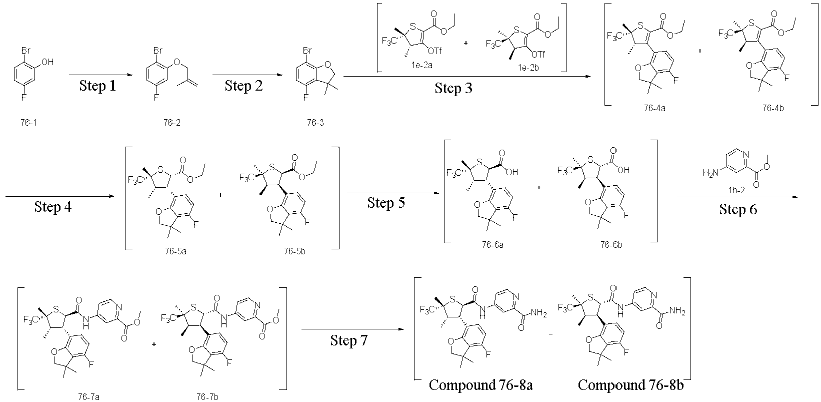

### Step 1: Preparation of compound 76-2

76-1 (3.8 g, 20.0 mmol), 3-bromo-2-methylpropene (3.24 g, 24.0 mmol) and potassium carbonate (5.53 g, 40.09 mmol) were added to acetonitrile (80 mL), and the mixture was reacted at 60°C under nitrogen atmosphere for 12 h. The reaction solution was cooled to room temperature and filtered through celite. The filtrate was concentrated under reduced pressure, and the residue was purified by column chromatography to obtain 76-2 (4.5 g, yield: 91.8%).

### Step 2: Preparation of compound 76-3

76-2 (4.5 g, 18.36 mmol) was added to dichloromethane (180 mL), and placed in an ice bath, and aluminium chloride (120 mg, 0.9 mmol) was added. The mixture was reacted in the ice bath for 2 h. The reaction was quenched by adding the reaction solution to an aqueous sodium bicarbonate solution. The organic phase was separated, dried over anhydrous sodium sulphate and then filtered. The filtrate was concentrated, and the residue was separated and purified by silica gel column chromatography to obtain 76-3 (3.07 g, yield: 68.2%).

### Step 3: Preparation of compounds 76-4a and 24-4b

76-3 (290 mg, 1.2 mmol), (Bpin)₂ (370 mg, 1.44 mmol), potassium acetate (240 mg, 2.4 mmol) and Pd(dppf)Cl₂•DCM (49 mg, 0.06 mmol) were added to 1,4-dioxane (8 mL), and the mixture was reacted at 100°C under nitrogen atmosphere for 6 h. The reaction solution was cooled to room temperature and filtered through celite to obtain a filtrate. Water (3 mL), the mixture of 1e-2a and 1e-2b (480 mg, 1.2 mmol), potassium phosphate (760 mg, 3.58 mmol) and Pd(PPh₃)₄(57.8 mg, 0.05 mmol) were added to the filtrate, and the mixture was reacted at 100°C under nitrogen atmosphere for 4 h. The reaction solution was cooled to room temperature and filtered through celite. The filtrate was concentrated under reduced pressure, and the residue was separated and purified by silica gel column chromatography to obtain a mixture of 76-4a and 76-4b (280 mg, yield: 55.7%).

### Step 4: Preparation of 76-5a and 76-5b

The mixture of 76-4a and 76-4b (240 mg, 0.57 mmol) was dissolved in methanol (15 mL), and platinum dichloride (50 mg, 0.19 mmol) was added. The mixture was reacted under hydrogen atmosphere at room temperature for 20 h. The reaction solution was filtered through celite, and the filtrate was concentrated. The residue was separated and purified by silica gel column chromatography to obtain a mixture of 76-5a and 76-5b (133 mg, yield: 55.5%).

### Step 5: Preparation of 76-6a and 76-6b

The mixture of 76-5a and 76-5b (143 mg, 0.34 mmol) was dissolved in anhydrous ethanol (5 mL), and caesium carbonate (170 mg, 0.51 mmol) was added. The mixture was reacted at 50°C for 5 h. The reaction solution was cooled to room temperature, concentrated under reduced pressure, and then added to 1 M hydrochloric acid (5 mL) to quench the reaction. The reaction mixture was extracted with ethyl acetate (50 mL×3). The organic phases were combined, dried over anhydrous sodium sulphate and then filtered. The filtrate was concentrated, and the residue was separated and purified by silica gel column chromatography to obtain a mixture of 76-6a and 76-6b (124 mg, yield: 92.9%).
LCMS m/z =391-3 [M-H]⁻

### Step 6: Preparation of 76-7a and 76-7b

The mixture of 76-6a and 76-6b (124 mg, 0.32 mmol) was dissolved in ethyl acetate (6 mL), and triethylamine (200 mg, 1.95 mmol), T3P (50% wt in EtOAc 788 mg; containing 1-propylphosphoric anhydride 394 mg, 1.29 mmol) and 1h-2 (73 mg, 0.48 mmol) were sequentially added. The mixture was reacted at room temperature under nitrogen atmosphere for 20 h. The reaction was quenched by adding an aqueous sodium bicarbonate solution (20 mL) to the reaction system, and the mixture was extracted with ethyl acetate (20 mL×3). The organic phases were combined, dried over anhydrous sodium sulphate, filtered and concentrated to obtain a crude, which was then separated and purified by silica gel column chromatography to obtain a mixture of 76-7a and 76-7b (50 mg, yield: 29.7%).
LCMS m/z =527.2 [M+H]⁺

### Step 7: Preparation of a mixture of compound 76-8a and compound 76-8b

The mixture of 76-7a and 76-7b (50 mg, 0.095 mmol) was dissolved in a 7 M ammonia methanol solution (3 mL), and the resulting mixture was reacted at room temperature for 4 h. The reaction system was concentrated to obtain a crude, which was then subjected to silica gel column chromatography to obtain a mixture of compound 76-8a and compound 76-8b (18 mg, yield: 36.8%).
LCMS m/z =512.2 [M+H]⁺
¹H NMR (400 MHz, CDCl₃) δ 9.91(s, 1H), 8.45 (d, 1H), 8.40 - 8.30 (m, 1H), 8.18 (d, 1H), 8.00 (d, 1H), 7.07 - 6.92 (m, 1H), 6.51 (t, 1H), 5.91 (s, 1H), 5.11 (d, 1H), 4.44 - 4.29 (m, 1H), 3.00 (s, 2H), 2.79 - 2.68 (m, 1H), 1.90 (s, 3H), 1.47 (s, 3H), 1.42 (s, 3H), 1.00 - 0.92 (m, 3H).

### Example 77: Preparation of compound 77

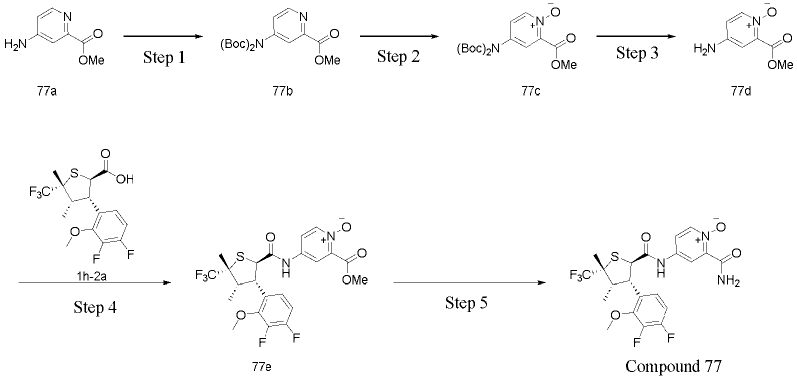

### Step 1: Preparation of compound 77b

77a (3.04 g, 20.0 mmol), (Boc)2O (4.8 g, 22 mmol) and 4-dimethylaminopyridine (244 mg, 2.0 mmol) were added to dichloromethane (70 mL), and the mixture was reacted at room temperature for 16 hours. The reaction solution was concentrated under reduced pressure, and the residue was purified by column chromatography to obtain 77b (2.38 g, yield: 33.8 %).
LCMS m/z =353.4 [M+H]⁺

### Step 2: Preparation of compound 77c

77b (352 mg, 1.0 mmol) was added to dichloromethane (10 mL), and placed in an ice bath, and m-chloroperbenzoic acid (516 mg, 3.0 mmol) was added. The mixture was further reacted at room temperature for 12 hours. The reaction solution was concentrated under reduced pressure, and the residue was separated and purified by silica gel column chromatography to obtain 77c (211 mg, yield: 57.3%)
LCMS m/z =369.2 [M+H]⁺

### Step 3: Preparation of compound 77d

77c (134 mg, 0.364 mmol) was added to dichloromethane (2 mL), and then hydrogen chloride in 1,4-dioxane (4 M, 4 mL) was added to the system. The mixture was reacted at room temperature for 2 hours. The reaction solution was concentrated under reduced pressure, and then dichloromethane (2 mL) was added. Hydrogen chloride in 1,4-dioxane (4 M, 4 mL) was added to the system, and the mixture was reacted for 2 hours. The reaction solution was cooled and concentrated under reduced pressure to obtain 77d (71 mg).
LCMS m/z =169.1 [M+H]⁺

### Step 4: Preparation of compound 77e

77d (71 mg) was dissolved in tetrahydrofuran (8 mL), and then triethylamine (121.4 mg, 1.2 mmol), T3P (50% wt in EtOAc 509 mg, containing 1-propylphosphoric anhydride 254.5 mg, 0.8 mmol) and substrate 1h-2a (74 mg, 0.2 mmol) were sequentially added to the system. After the addition, the mixture was reacted at room temperature under nitrogen atmosphere for 20 h. The reaction was quenched by adding an aqueous sodium bicarbonate solution (20 mL) to the reaction system, and the mixture was extracted with ethyl acetate (20 mL×3). The organic phases were combined, dried over anhydrous sodium sulphate, filtered and concentrated to obtain a crude, which was then separated and purified by silica gel column chromatography to obtain 77e (81 mg, yield: 77.8%).
LCMS m/z =521.1 [M+H]⁺

### Step 5: Preparation of compound 77

Substrate 77e (81 mg, 0.155 mmol) was dissolved in a 7 M ammonia methanol solution (10 mL), and the mixture was reacted at room temperature for 2 hours. The reaction system was concentrated to obtain a crude, which was then purified by silica gel column chromatography to obtain compound 77 (42 mg, yield: 53.6%).
LCMS m/z =506.1 [M+H]⁺

### Biological test example 1:

### Nav1.8 manual patch clamp test

### (1) Cell culture

CHO cell lines stably expressing human Nav1.8 were cultured in a Ham's F-12 medium containing 10% foetal bovine serum, 10 µg/mL Blasticidin, 200 µg/mL Hygromycin B and 100 µg/mL Zeocin. The cells were cultured at 37°C with a carbon dioxide concentration of 5%. The old medium was removed, and the cells were rinsed once with PBS. Then, 1 mL of 0.25%-Trypsin-EDTA solution was added, and the cells were incubated at 37°C for approximately 1.5 min. Once the cells detached from the bottom of the dish, complete medium pre-warmed to 37°C was added. The cell suspension was gently triturated with a pipette to dissociate the clumped cells. The cell suspension was transferred to a sterile centrifuge tube and centrifuged at 1000 rpm for 5 min to collect the cells. The cells were seeded into 6 cm cell culture dishes at a density of 2.5 × 10⁵ cells per cell culture dish (final volume: 5 mL) for expansion or maintenance culture. To maintain the electrophysiological activity of the cells, the cell density should not exceed 80%. Before patch clamp testing, the cells were dissociated with 0.25%-Trypsin-EDTA. 6.5 × 10³ cells were plated onto coverslips and cultured in 24-well plates (final volume: 500 µL) for 18 hours before detection.

### (2) Compound formulation

The compound was dissolved in dimethyl sulfoxide (DMSO) and prepared into a 30 mM DMSO stock solution. The stock solution was diluted with the extracellular fluid (140 mM NaCl, 3.5 mM KCl, 1 mM MgCl₂•6H₂O, 2 mM CaCl₂•2H₂O, 10 mM D-Glucose, 10 mM HEPES and 1.25 mM NaH₂PO₄•2H₂O; adjusted to pH 7.4 with NaOH) to the test concentration, and the final DMSO concentration of all test samples was 0.1%.

### (3) Electrophysiological test

First, the recording electrodes were pulled from capillary glass tubes by using a microelectrode puller. Then, the electrodes were filled with the intracellular fluid (50 mM CsCl, 10 mM NaCl, 10 mM HEPES, 60 mM CsF and 20 mM EGTA; adjusted to pH 7.2 with CsOH) and mounted onto the microelectrode holder. Under an inverted microscope, the microelectrode manipulator was used to immerse the electrodes into the extracellular fluid, and the pipette resistance (Rpip) was recorded. The electrodes were slowly brought into contact with the cell surface, and negative pressure suction was applied to form a GΩ seal. At this point, fast capacitance compensation was performed, and negative pressure was continuously applied to break the cell membrane, forming a whole-cell recording mode. Finally, slow capacitance compensation was performed, and experimental parameters such as series resistance (Rs) were recorded. No electric leakage compensation was applied. When the Nav1.8 current in whole-cell recording was stable, drug administration was initiated, with each drug concentration for approximately 5 min (or until the current was stable). The coverslips spread with the cells were placed in a recording chamber under the inverted microscope. The blank control extracellular fluid and the test compound working solution were perfused through the recording chamber via the gravity-driven perfusion method, thereby acting on the cells, and a peristaltic pump was used to facilitate liquid exchange. The current recorded from cells in compound-free extracellular fluid was used as the control group. All electrophysiological experiments were conducted at room temperature. The inhibition rate of compounds on Nav1.8 was determined by calculating the relative percentage of peak currents generated before and after the cells were treated with the compounds.

The voltage stimulation protocol for whole-cell patch clamp recording of Nav1.8 sodium currents was described as follows. When the whole-cell sealing was formed, the cells were clamped at a voltage of -120 mV. First, the voltage was stepped from -110 mV to -30 mV in 10 mV increments and maintained for 5 s, followed by a depolarizing pulse to 0 mV to obtain a half-inactivation voltage (Vhalf). The Vhalf was used as the stimulus voltage and applied for 5 s, and then the voltage was restored to -120 mV for 20 ms, followed by a depolarizing pulse (TP2) to 0 mV for 50 ms to detect the sodium current in the half-inactivated state. Finally, the voltage was restored to the clamping voltage of -120 mV, and data were repeatedly collected every 20 ms to observe the effect of drugs on the peak sodium current. The experimental data was collected by an EPC 10 amplifier (HEKA) and stored in PatchMaster (HEKA) software.

**Table 1 Inhibitory activity (IC₅₀) of test compounds against hNav1.8**

| Test compound | IC₅₀ (nM) |
|---|---|
| Mixture of compound 1-1a and compound 1-1b | <10 |
| Mixture of compound 1-2a and compound 1-2b | <0.1 |
| Compound 1-2P1 | <0.1 |
| Compound 1-2P2 | <10 |
| Trifluoroacetate of mixture of compound 3-2a and compound 3-2b | <1 |
| Trifluoroacetate of mixture of compound 4-1a and compound 4-1b | <10 |
| Mixture of compounds 5-3a and 5-3b | <1 |
| Mixture of compounds 6-2a and 6-2b | <0.1 |
| Mixture of compound 7-1a and compound 7-1b | <10 |
| Mixture of compounds 8-1a and 8-1b | <0.1 |
| Mixture of compound 11-1a and compound 11-1b | <10 |
| Mixture of compounds 18-2a and 18-2b | <1 |
| Mixture of compounds 19-2a and 19-2b | <1 |
| Mixture of compounds 20-1a and 20-1b | <0.1 |
| Mixture of compounds 21-7a and 21-7b | <1 |
| Compound 21-7P1 | <0.1 |
| Mixture of compounds 22-1a and 22-1b | <0.1 |
| Trifluoroacetate of mixture of compounds 23-8a and 23-8b | <10 |
| Mixture of compounds 24-9a and 24-9b | <10 |
| Mixture of compounds 25-1a and 25-1b | <0.1 |
| Mixture of compounds 26-1a and 26-1b | <1 |
| Trifluoroacetate of mixture of compounds 27-1a and 27-1b | <0.1 |
| Mixture of compounds 28-2a and 28-2b | <0.1 |
| Compound 31-1P1 | <0.1 |
| Compound 32-1P1 | <1 |
| Compound 32-1P2 | <1 |
| Mixture of compounds 33-1a and 33-1b | <1 |
| Mixture of compounds 34-1a and 34-1b | <1 |
| Mixture of compounds 35-8a and 35-8b | <0.1 |
| Mixture of compounds 36-2a and 36-2b | <0.1 |
| Mixture of compounds 37-1a and 37-1b | <1 |
| Compound 1-2a | <0.1 |
| Compound 5-3a | <0.1 |
| Compound 6-2a | <0.1 |
| Compound 7-1a | <0.1 |
| Compound 8-1a | <0.1 |
| Compound 18-2a | <1 |
| Compound 19-2a | <1 |
| Compound 20-1a | <1 |
| Compound 22-1a | <0.1 |
| Compound 25-1a | <0.1 |
| Compound 26-1a | <0.1 |
| Compound 27-1a | <0.1 |
| Compound 28-2a | <0.1 |
| Compound 36-2a | <0.1 |
| Compound 48-1a | <1 |
| Compound 50 | <1 |
| Compound 54 | <0.1 |
| Compound 55 | <1 |
| Compound 57 | <1 |
| Trifluoroacetate of compound 58 | <1 |
| Compound 59-b | <0.1 |
| Compound 61-b | <0.1 |
| Compound 63-b | <0.1 |
| Compound 65-b | <0.1 |
| Compound 68-b | <1 |
| Compound 70-a | <0.1 |
| Compound 72-a | <0.1 |
| Compound 74-a | <1 |
| Mixture of compounds 76-8a and 76-8b | <1 |

| | |
|---|---|
| Conclusion: the compounds of the present invention, such as the example compounds, have good Nav1.8 inhibitory activity. Specifically, compound 31-1P1, for example, exhibits IC₅₀ = 0.0412 nM against hNav1.8. | |

### Biological test example 2: Pharmacokinetic test in rats

### Experimental animals: male SD rats, about 220 g, 6-8 weeks old, 6 rats/compound

Experimental design: on the day of the experiment, 24 SD rats were randomly grouped according to their body weights. The animals were fasted with water available for 12-14 h one day before the administration, and were fed 4 h after the administration.

**Table 2. Administration information**

| Number | Administration information | | | | | |
|---|---|---|---|---|---|---|
| Male | Test compound | Administration dosage (mg/kg) | Administration concentration (mg/mL) | Administration volume (mL/kg) | Collected sample | Administration mode |
| 3 | | 2.5 | 0.5 | 5 | Plasma | Intravenous administration |
| 3 | | 10 | 1 | 10 | Plasma | Intragastric administration |

| | | | | | | |
|---|---|---|---|---|---|---|
| Note: vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: methylcellulose) | | | | | | |

Before and after the administration, 0.10 mL of blood was taken from the orbit under isoflurane anaesthesia, and placed in an EDTAK2 centrifuge tube. Centrifugation was performed at 5000 rpm at 4°C for 10 min, and the plasma was collected. The blood collection time points for both the intravenous administration group and the intragastric administration group were: 0, 5 min, 15 min, 30 min, 1 h, 2 h, 4 h, 6 h, 8 h and 24 h. Before analysis and detection, all samples were stored at -80°C. The samples were quantitatively analysed by LC-MS/MS.

Conclusion: the compounds of the present invention, such as the example compounds, exhibit good oral performance in rats.

### Biological test example 3: Pharmacokinetic test in mice

Experimental animals: C57 mice, 22-25 g, 6 mice/compound.

Experimental design: on the day of the experiment, C57 mice were randomly grouped according to their body weights. The animals were fasted with water available for 12-14 h one day before the administration, and were fed 4 h after the administration.

**Table 3. Administration information**

| Number | Administration information | | | | | |
|---|---|---|---|---|---|---|
| Male | Test compound | Administration dosage (mg/kg) | Administration concentration (mg/mL) | Administration volume (mL/kg) | Collected sample | Administration mode |
| 9 | | 2.5 | 0.5 | 5 | Plasma | Intravenous administration |
| 9 | | 30 | 3 | 10 | Plasma | Intragastric administration |

| | | | | | | |
|---|---|---|---|---|---|---|
| Note: vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: methylcellulose) | | | | | | |

Before and after the administration, 0.06 mL of blood was taken from the orbit under isoflurane anaesthesia, and placed in an EDTAK2 centrifuge tube. Centrifugation was performed at 5000 rpm at 4°C for 10 min, and the plasma was collected. The blood collection time points for both the intravenous administration group and the intragastric administration group were: 0, 5 min, 15 min, 30 min, 1 h, 2 h, 4 h, 7 h, 24 h and 48 h. Before analysis and detection, all samples were stored at -80°C. The samples were quantitatively analysed by LC-MS/MS.

**Table 3-1 Pharmacokinetic results of test compounds in mice**

| Test compound | Administration mode | AUC₀₋ₜ (hr×ng/mL) |
|---|---|---|
| Compound 31-1P1 | PO 30 mg/kg | 61590±9483 |
| Compound 58 | PO 30 mg/kg | 65149±9204 |

| | | |
|---|---|---|
| Conclusion: the compounds of the present invention, such as the example compounds, exhibit good oral performance in mice. | | |

### Biological test example 4: Pharmacokinetic test in beagle dogs

Experimental animals: male beagle dogs, about 8-11 kg, 6 dogs/compound, purchased from Beijing Marshall Biotechnology Co., Ltd.

Experimental method: on the day of the experiment, 12 beagle dogs were randomly grouped according to their body weights. The animals were fasted with water available for 12-14 h one day before the administration, and were fed 4 h after the administration. Administration was performed as per Table 4.

**Table 4. Administration information**

| Number | Administration information | | | | | |
|---|---|---|---|---|---|---|
| Male | Test compound | Administration dosage (mg/kg) | Administration concentration (mg/mL) | Administration volume (mL/kg) | Collected sample | Administration mode |
| 3 | | 1 | 1 | 1 | Plasma | Intravenous administration |
| 3 | | 5 | 1 | 5 | Plasma | Intragastric administration |

| | | | | | | |
|---|---|---|---|---|---|---|
| Note: vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: methylcellulose solution ) | | | | | | |

Before and after the administration, 1 ml of blood was taken from the jugular veins or limb veins, and placed in an EDTAK2 centrifuge tube. Centrifugation was performed at 5000 rpm at 4°C for 10 min, and the plasma was collected. The blood collection time points for both the intravenous administration group and the intragastric administration group were: 0, 5 min, 15 min, 30 min, 1 h, 2 h, 4 h, 6 h, 8 h, 10 h, 12 h, 24 h, 48 h, and 72 h. Before analysis and detection, all samples were stored at -80°C. The samples were quantitatively analysed by LC-MS/MS.

Conclusion: the compounds of the present invention, such as the example compounds, exhibit good oral performance in dogs.

### Biological test example 5: Pharmacokinetic test in monkeys

Experimental animals: male cynomolgus monkeys, 3-5 kg, 3-6 years old, 6 monkeys/compound, purchased from Suzhou Xishan Biotechnology Co., Ltd.

Experimental method: on the day of the experiment, 6 monkeys were randomly grouped according to their body weights. The animals were fasted with water available for 14-18 h one day before the administration, and were fed 4 h after the administration.

**Table 5. Administration information**

| Number | Administration information | | | | | |
|---|---|---|---|---|---|---|
| Male | Test compound | Administration dosage (mg/kg) | Administration concentration (mg/mL) | Administration volume (mL/kg) | Collected sample | Administration mode |
| 3 | | 1 | 0.5 | 2 | Plasma | Intravenous administration |
| 3 | | 5 | 1 | 5 | Plasma | Intragastric administration |

| | | | | | | |
|---|---|---|---|---|---|---|
| Note: vehicle for intravenous administration: 5% DMA+5% Solutol+90% Saline; vehicle for intragastric administration: 0.5%MC (containing 0.5% Tween 80) *Dosage is calculated based on free bases. | | | | | | |

Before and after the administration, 1.0 mL of blood was taken from the limb veins, and placed in an EDTAK2 centrifuge tube. Centrifugation was performed at 5000 rpm at 4°C for 10 min, and the plasma was collected. The blood collection time points for both the intravenous administration group and the intragastric administration group were: 0, 5 min, 15 min, 30 min, 1 h, 2 h, 4 h, 6 h, 8 h, 10 h, 12 h, and 24 h. Before analysis and detection, all samples were stored at -80°C. The samples were quantitatively analysed by LC-MS/MS.

Conclusion: the compounds of the present invention, such as the example compounds, exhibit good oral performance in monkeys.

### Biological test example 6: CYP450 enzyme inhibition test

The purpose of this study was to evaluate the effect of test compounds on the activity of five isoenzymes (CYP1A2, CYP2C9, CYP2C19, CYP2D6, and CYP3A4) of human liver microsomal cytochrome P450 (CYP) by using an *in vitro* testing system. The specific probe substrates of CYP450 isoenzymes were co-incubated with human liver microsomes and test compounds at different concentrations, and the reaction was initiated by adding reduced nicotinamide adenine dinucleotide phosphate (NADPH). After the completion of the reaction, the samples were treated and subjected to the liquid chromatography-tandem mass spectrometry (LC-MS/MS) to quantitatively detect metabolites produced by the specific substrates. The changes in CYP enzyme activity were determined, and IC₅₀ values were calculated to evaluate the inhibitory potential of the test compounds on each CYP enzyme subtype.

Conclusion: the compounds of the present invention, such as the example compounds, have weak CYP inhibition. For example, compound 58 exhibits IC₅₀ greater than 30 µM against five CYP isoenzymes (CYP1A2, CYP2C9, CYP2C19, CYP2D6, and CYP3A4).

### Biological test example 7: Test of Caco2 permeability

In the experiment, a monolayer of Caco-2 cells was used, and incubated in triplicate in a 96-well Transwell plate. A transport buffer solution (HBSS, 10 mM HEPES, pH 7.4 ± 0.05) containing the compounds of the present invention (2 µM) or the control compound digoxin (10 µM), nadolol (2 µM) or metoprolol (2 µM) was added to the administration end hole on the apical side or basal side. DMSO-containing transport buffer solution was added to the corresponding receiving end hole. After incubation at 37°C ± 1°C for 2 hours, the cell plate was removed, and an appropriate amount of samples were taken from the apical side and basal side and transferred to a new 96-well plate. Subsequently, acetonitrile containing an internal standard was added to precipitate the protein. The samples were analysed using LC MS/MS, and the concentrations of the compounds of the present invention and the control compounds were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

Conclusion: the compounds of the present invention, such as the example compounds, exhibit good permeability.

### Biological test example 8: Spinal nerve ligation (SNL)-induced mouse model of neuropathic pain

Male C57BL/6J mice purchased from Zhejiang Vital River Laboratory Animal Technology Co., Ltd. were adaptively raised for one week and then the models were established. The specific establishment method comprises the following steps:
(1) disinfecting the surgical instrument and ligature;
(2) anaesthetizing the mice with isoflurane and then placing the mice on the operating table in the prone position;
(3) shaving the fur near the hip bones of the mice for skin preparation, and making an incision of about 2 cm along the spine;
(4) isolating the fascia along the spine, performing blunt dissection of the muscles, and exposing the transverse process of L5;
(5) carefully cutting the transverse process of L5 with forceps and exposing the L5 spinal nerve;
(6) carefully isolating the L5 nerve with a glass dissecting needle, and ligating the L5 nerve with a 5-0 ligature; and
(7) suturing the muscles and skin and disinfecting same with iodophor.

The mice that were unsuccessful in modelling were eliminated the next day after modelling (marker for successful modelling: mice with their hind paws curled up). After modelling, the mice were stroked for 3 to 5 minutes every day to ensure that the animals were familiar with the experimenter, and then the mice were placed on a metal pain measuring frame for 40 to 60 minutes of adaptation. After 3 days of acclimatization, Von Frey filaments (Aesthesio^{®}; 0.16 g, 0.4 g, 0.6 g, 1.0 g, 1.4 g and 2.0 g) were used to test the pre-administration baseline values of the animals (Ascending testing approach). Each animal was tested twice and average values were taken, with intervals of at least 5 minutes. The animals were grouped according to the baseline values (10 animals per group). After grouping, the test compounds (3 and 30 mg/kg) or vehicles (0.5% methylcellulose) were administered intragastrically, and the mechanical pain threshold (MPT) of the mice was measured at different time points after the administration. Time-MPT curves were plotted using GraphPad 8.3.0, and statistical analysis was performed.

Conclusion: according to the analysis of the area under the time-MPT curve, the compounds of the present invention, such as the example compounds, have significant analgesic efficacy. For example, compound 6-2a exhibits significant analgesic efficacy at both 3 mg/kg and 30 mg/kg.

## Claims

1. A compound, or a stereoisomer, deuterated compound, solvate, prodrug, metabolite, pharmaceutically acceptable salt or cocrystal thereof, **characterized in that** the compound is selected from a compound as represented by general formula (I), 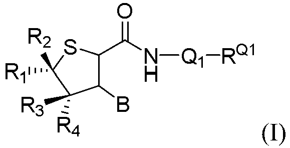
Q₁ is selected from C₆₋₁₀ aryl, 5- to 10-membered heteroaryl, C₅₋₁₀ carbocyclyl, 5- to 10-membered heterocyclyl or 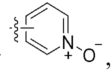 and the aryl, heteroaryl, carbocyclyl or heterocyclyl is optionally substituted with 1 to 5 R^{q};
R^{Q1} is selected from H, COOH, NR^{q1}R^{q2}, -C(=O)NR^{q1}R^{q2}, -S(=O)₂NR^{q1}R^{q2}, OH, =O, -OR^{q1}, -C(=O)R^{q1}, -S(=O)₂R^{q1}, -S(=O)(=NR^{q1})R^{q2} or -P(=O)R^{q1}R^{q2};
R^{q1} and R^{q2} are each independently selected from H, C₁₋₆ alkyl, C₃₋₆ carbocyclyl, or 4- to 7-membered heterocyclyl, and the alkyl, carbocyclyl or heterocyclyl is optionally substituted with 1 to 4 R^{k};
alternatively, R^{q1} and R^{q2} are directly linked to form 4- to 7-membered heterocyclyl, and the heterocyclyl is optionally substituted with 1 to 4 R^{k};
B is selected from C₆₋₁₀ aryl, 5- to 10-membered heteroaryl, C₅₋₁₀ carbocyclyl, or 5- to 10-membered heterocyclyl, and the aryl, heteroaryl, carbocyclyl or heterocyclyl is optionally substituted with 1 to 4 R^{B};
R¹ and R² are each independently selected from H, halogen, CN, OH, C₁₋₆ alkyl, C₂₋₆ alkenyl, C₂₋₆ alkynyl, -OC₁₋₆ alkyl, -SC₁₋₆ alkyl, C₃₋₆ carbocyclyl, or 3-to 7-membered heterocyclyl, and the alkyl, alkenyl, alkynyl, carbocyclyl or heterocyclyl is optionally substituted with 1 to 4 R^{k}, provided that R¹ and R² are not both H;
R³, R⁴, R^{q}, and R^{B} are each independently selected from H, deuterium, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, C₃₋₇ carbocyclyl, -OC₃₋₇ carbocyclyl, 3- to 7-membered heterocyclyl or -P(=O)R^{q1}R^{q2}, and the alkyl, alkenyl, alkynyl, carbocyclyl or heterocyclyl is optionally substituted with 1 to 4 R^{k};
R^{k} is selected from deuterium, =O, halogen, CN, OH, NH₂, NHC₁₋₆ alkyl, N(C₁₋₆ alkyl)₂, C₁₋₆ alkyl, C₂₋₆ alkenyl, C₂₋₆ alkynyl, -OC₁₋₆ alkyl, -SC₁₋₆ alkyl, -O-C₃₋₆ carbocyclyl, -O-3- to 7-membered heterocyclyl, -NH-C₃₋₆ carbocyclyl, -NH-3-to 7-membered heterocyclyl, -C₁₋₄ alkylene-C₃₋₆ carbocyclyl, -C₁₋₄ alkylene-3- to 7-membered heterocyclyl, C₃₋₆ carbocyclyl, or 3- to 7-membered heterocyclyl, and the alkyl, alkylene, alkenyl, alkynyl, carbocyclyl or heterocyclyl is optionally substituted with 1 to 4 substituents selected from deuterium, halogen, =O, CN, OH, NH₂, C₁₋₆ alkyl, or C₁₋₆ alkoxy.

2. The compound or the stereoisomer, deuterated compound, solvate, prodrug, metabolite, pharmaceutically acceptable salt or cocrystal thereof according to claim 1, **characterized in that**
R^{q1} and R^{q2} are each independently selected from H, C₁₋₄ alkyl, C₃₋₆ carbocyclyl, or 4- to 7-membered heterocyclyl, and the alkyl, carbocyclyl or heterocyclyl is optionally substituted with 1 to 4 R^{k};
alternatively, R^{q1} and R^{q2} are directly linked to form 4- to 7-membered heterocyclyl, and the heterocyclyl is optionally substituted with 1 to 4 R^{k};
Q₁ is selected from phenyl, benzo C₄₋₆ carbocyclyl, benzo 4- to 6-membered heterocyclyl, 5- to 6-membered heteroaryl, 8- to 10-membered fused heteroaryl or 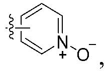 and the Q₁ is optionally substituted with 1 to 4 R^{q};
B is selected from phenyl, benzo C₄₋₆ carbocyclyl, benzo 4- to 6-membered heterocyclyl, 5- to 6-membered heteroaryl, or 8- to 10-membered fused heteroaryl, and the B is optionally substituted with 1 to 4 R^{B};
R¹ and R² are each independently selected from H, halogen, CN, OH, C₁₋₄ alkyl, C₂₋₄ alkenyl, C₂₋₄ alkynyl, -OC₁₋₄ alkyl, -SC₁₋₄ alkyl, C₃₋₆ carbocyclyl, or 3-to 7-membered heterocyclyl, and the alkyl, alkenyl, alkynyl, carbocyclyl or heterocyclyl is optionally substituted with 1 to 4 R^{k}, provided that R¹ and R² are not both H;
R³, R⁴, R^{q}, and R^{B} are each independently selected from H, deuterium, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, C₃₋₇ carbocyclyl, -OC₃₋₇ carbocyclyl, 3- to 7-membered heterocyclyl or -P(=O)R^{q1}R^{q2}, and the alkyl, alkenyl, alkynyl, carbocyclyl or heterocyclyl is optionally substituted with 1 to 4 R^{k};
R^{k} is selected from deuterium, =O, halogen, CN, OH, NH₂, NHC₁₋₄ alkyl, N(C₁₋₄ alkyl)₂, C₁₋₄ alkyl, C₂₋₄ alkenyl, C₂₋₄ alkynyl, -OC₁₋₄ alkyl, -SC₁₋₄ alkyl, -O-C₃₋₆ carbocyclyl, or -O-3- to 7-membered heterocyclyl, and the alkyl, alkylene, alkenyl, alkynyl, carbocyclyl or heterocyclyl is optionally substituted with 1 to 4 substituents selected from deuterium, halogen, CN, OH, NH₂, C₁₋₄ alkyl, or C₁₋₄ alkoxy.

3. The compound or the stereoisomer, deuterated compound, solvate, prodrug, metabolite, pharmaceutically acceptable salt or cocrystal thereof according to claim 2, **characterized in that**
R^{q1} and R^{q2} are each independently selected from H, methyl, ethyl, propyl, isopropyl, cyclopropyl, cyclobutyl or cyclopentyl, and the methyl, ethyl, propyl, isopropyl, cyclopropyl, cyclobutyl or cyclopentyl is optionally substituted with 1 to 4 R^{k};
R¹ and R² are each independently selected from H, F, Cl, Br, cyano, methyl, ethyl, methoxy, ethoxy, methylthio, cyclopropyl, cyclobutyl, ethenyl, or ethynyl, and the methyl, ethyl, methoxy, ethoxy, methylthio, cyclopropyl, cyclobutyl, ethenyl, or ethynyl is optionally substituted with 1 to 4 R^{k}, provided that R¹ and R² are not both H;
B is selected from 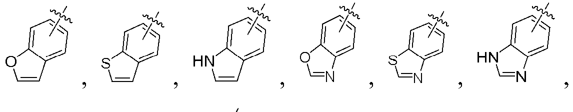 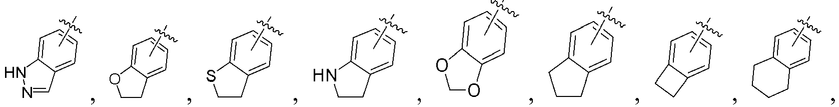 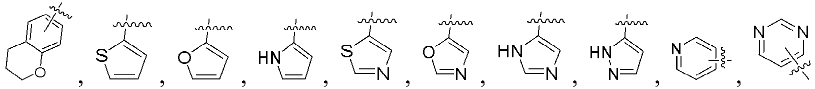 or phenyl, and the B is optionally substituted with 1 to 5 R^{B};
R³, R⁴, R^{q}, and R^{B} are each independently selected from H, deuterium, F, Cl, Br, cyano, methyl, ethyl, isopropyl, methoxy, ethoxy, isopropyloxy, methylthio, cyclopropyl, cyclopropyloxy, cyclobutyl, ethenyl, ethynyl, -P(=O)(CH₃)₂, - P(=O)(CH₂CH₃)₂, or -P(=O)(CH₃)(cyclopropyl), and the methyl, ethyl, isopropyl, methoxy, ethoxy, isopropyloxy, methylthio, cyclopropyl, cyclobutyl, ethenyl, or ethynyl is optionally substituted with 1 to 4 R^{k};
R^{k} is selected from deuterium, =O, F, Cl, Br, I, CN, OH, NH₂, NH(CH₃), NH(CH₂CH₃), N(CH₃)₂, N(CH₂CH₃)₂, methyl, ethyl, ethenyl, ethynyl, methoxy, ethoxy, methylthio, -O-cyclopropyl, -NH-cyclopropyl, -CH₂-cyclopropyl, -CH₂-cyclobutyl, -CH₂-cyclopentyl, -CH₂-cyclohexyl, cyclopropyl, cyclobutyl, cyclopentyl, or cyclohexyl, and the methyl, ethyl, ethenyl, ethynyl, methoxy, ethoxy, methylthio, cyclopropyl, cyclobutyl, cyclopentyl, or cyclohexyl is optionally substituted with 1 to 4 substituents selected from deuterium, halogen, CN, OH, NH₂, C₁₋₄ alkyl, or C₁₋₄ alkoxy.

4. The compound or the stereoisomer, deuterated compound, solvate, prodrug, metabolite, pharmaceutically acceptable salt or cocrystal thereof according to claim 3, **characterized in that**
Q₁ is selected from 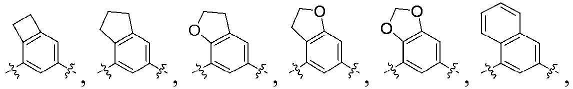 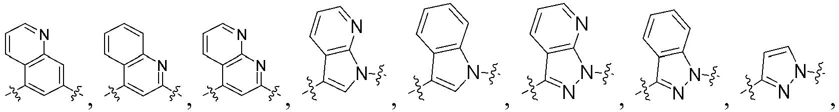 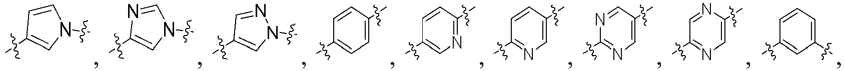 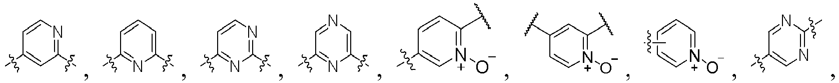 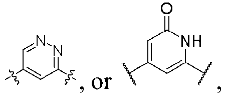 and the Q₁ is optionally substituted with 1 to 5 R^{q};
R³ and R⁴ are each independently selected from H, methyl, ethyl, CH₂F, CHF₂, or CF₃;
R¹ and R² are each independently selected from H, methyl, ethyl, CH₂F, CHF₂, or CF₃, provided that R¹ and R² are not both H;
R^{q} and R^{B} are each independently selected from H, F, Cl, Br, cyano, CH₂F, CHF₂, CF₃, -OCH₂F, -OCHF₂, -OCF₃, -OCD₃, methyl, -S-methyl, -S-CF₃, ethyl, isopropyl, ethynyl, methoxy, ethoxy, isopropyloxy, propyloxy, cyclopropyl, -O-cyclopropyl, -P(=O)(CH₃)₂, -P(=O)(CH₂CH₃)₂, or -P(=O)(CH₃)(cyclopropyl), and the methyl, ethyl, isopropyl, ethynyl, methoxy, ethoxy, isopropyloxy, propyloxy, or cyclopropyl is optionally substituted with 1 to 4 R^{k};
R^{k} is selected from deuterium, F, Cl, Br, I, CN, OH, -CH₂OH, methyl, ethyl, ethenyl, ethynyl, methoxy, ethoxy, methylthio, -O-cyclopropyl, -NH-cyclopropyl, - CH₂-cyclopropyl, -CH₂-cyclobutyl, -CH₂-cyclopentyl, -CH₂-cyclohexyl, cyclopropyl, cyclobutyl, cyclopentyl, or cyclohexyl.

5. The compound or the stereoisomer, deuterated compound, solvate, prodrug, metabolite, pharmaceutically acceptable salt or cocrystal thereof according to claim 4, **characterized in that** 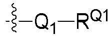
is selected from 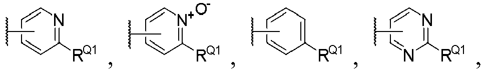 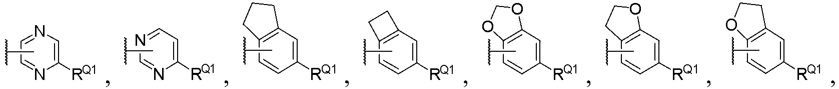 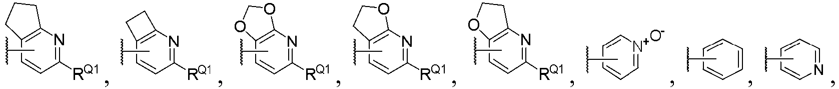 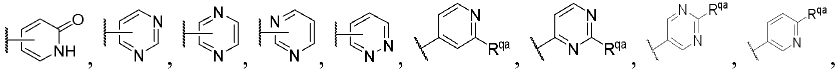 or 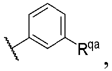 and the Q₁ is optionally substituted with 1 to 3 R^{q};
R^{qa} is selected from 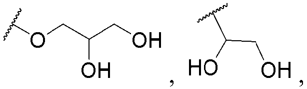 -CH₂OH, -CF₂CH₂OH, NH₂, -P(=O)(CH₃)₂, -P(=O)(CH₂CH₃)₂, or -P(=O)(CH₃)(cyclopropyl), and preferably, R^{qa} is selected from 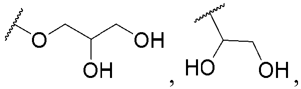 or -CH₂OH;
preferably, 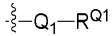 is selected from 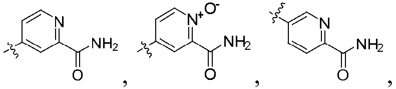 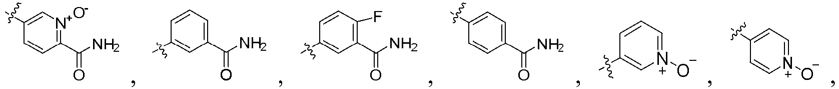 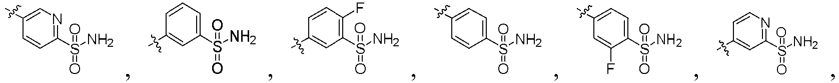 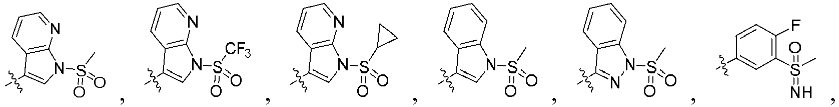 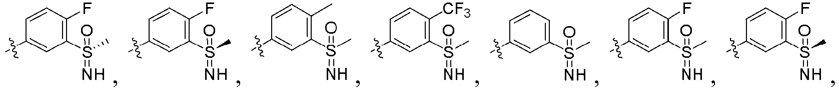 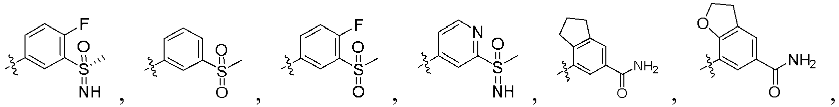 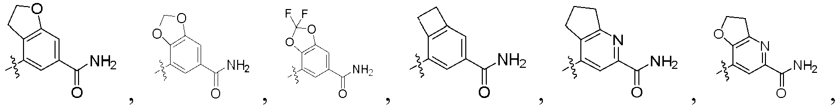 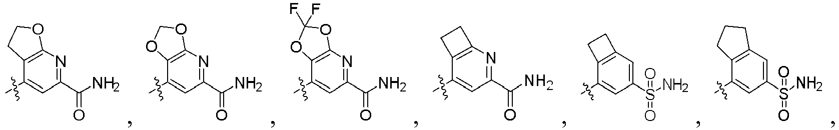 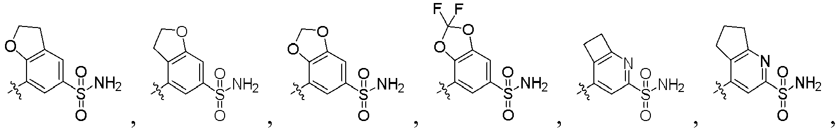 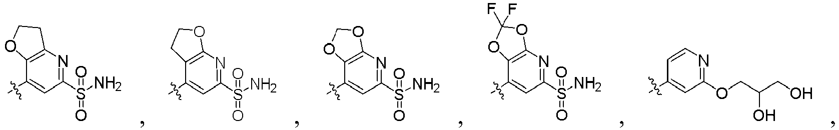 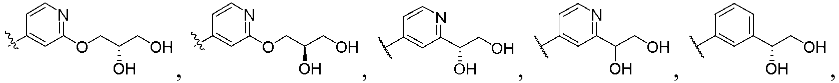 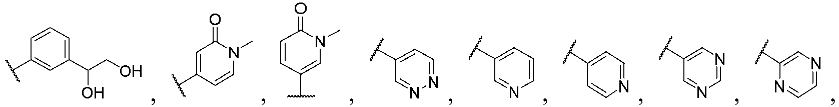 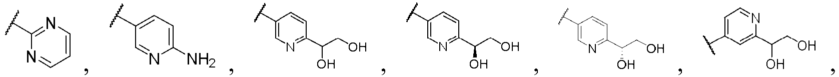 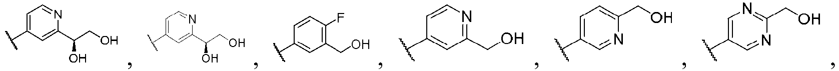 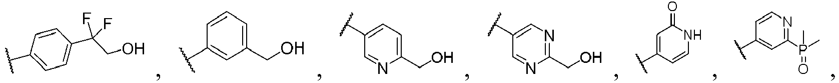 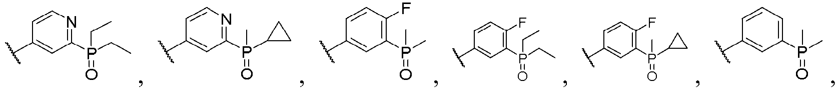 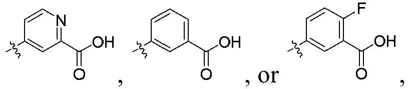 and the Q₁ is optionally substituted with 1 to 3 R^{q};
B is selected from 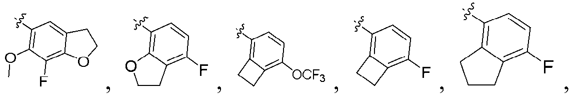 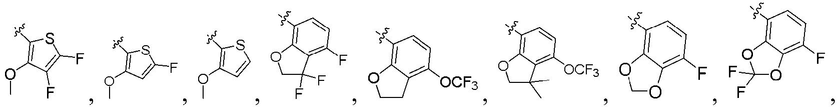 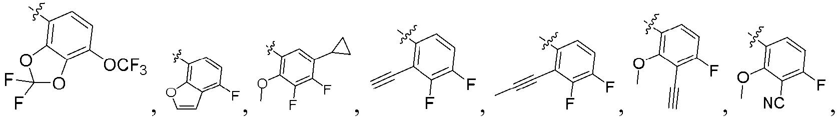 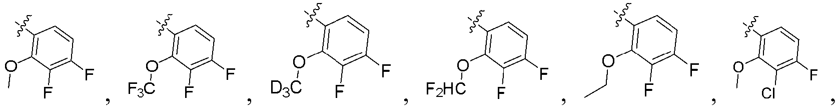 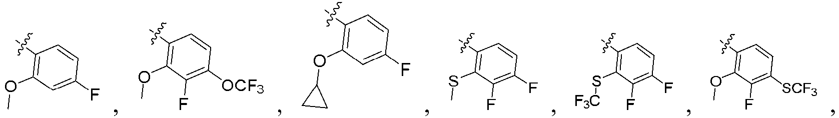 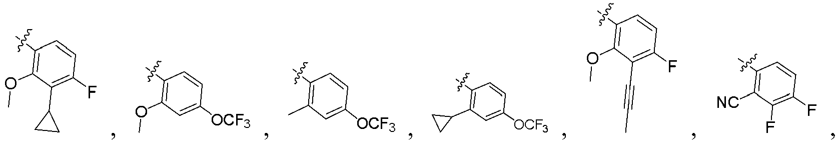 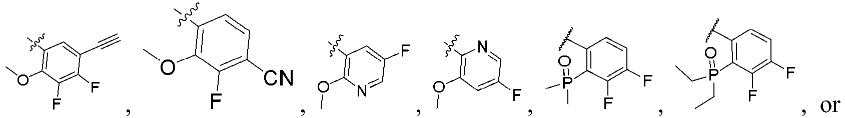 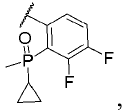 preferably

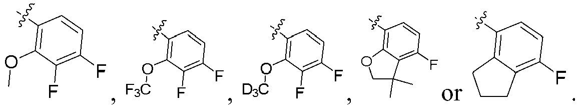

6. The compound or the stereoisomer, deuterated compound, solvate, prodrug, metabolite, pharmaceutically acceptable salt or cocrystal thereof according to claim 1, **characterized in that** the compound has a structure selected from one of the structures shown in Table E.

7. A pharmaceutical composition, **characterized by** comprising the compound or the stereoisomer, deuterated compound, solvate, prodrug, metabolite, pharmaceutically acceptable salt or cocrystal thereof according to any one of claims 1-6, and a pharmaceutically acceptable carrier, wherein preferably, the pharmaceutical composition comprises 1-1500 mg of the compound or the stereoisomer, deuterated compound, solvate, prodrug, metabolite, pharmaceutically acceptable salt or cocrystal thereof according to any one of claims 1-6.

8. Use of the compound or the stereoisomer, deuterated compound, solvate, prodrug, metabolite, pharmaceutically acceptable salt or cocrystal thereof according to any one of claims 1-6 in the preparation of a drug for treating or/and relieving pain.

9. A method for treating or alleviating a disease in a mammal, **characterized in that** the method comprises administering to a subject a therapeutically effective amount of the compound or the stereoisomer, deuterated compound, solvate, prodrug, metabolite, pharmaceutically acceptable salt or cocrystal thereof according to any one of claims 1-6, the therapeutically effective amount is preferably 1-1500 mg, and the disease is preferably pain.
